# FASCICULE DE BREVET EUROPEEN

(11) **EP 2 430 006 B1**
(45) Date de publication et mention de la délivrance du brevet: **26.11.2014**
(21) Numéro de dépôt: 10728741.9
(22) Date de dépôt: 11.05.2010
(51) Int. Cl.: C07D 277/64, C07D 285/06, C07D 403/12, C07D 413/14, C07D 417/12, C07D 417/14, A61K 31/403, A61P 29/00

(54) **DÉRIVÉS DE CYCLOPENTA[C]PYRROLE-2-CARBOXYLATES, LEUR PRÉPARATION ET LEUR APPLICATION EN THÉRAPEUTIQUE**
CYCLOPENTA[C]PYRROL-2-CARBOXYLAT-DERIVATE, IHRE ZUBEREITUNG UND THERAPEUTISCHE VERWENDUNG
CYCLOPENTA[C]PYRROLE-2-CARBOXYLATE DERIVATIVES, PREPARATION THEREOF, AND THERAPEUTIC USE THEREOF

(30) Priorité: 12.05.2009 FR 0902268
(43) Date de publication de la demande: 21.03.2012
(73) Titulaire: SANOFI, 75008 Paris (FR)
(72) Inventeur: ABOUABDELLAH, Ahmed, F-75013 Paris (FR); EVEN, Luc, F-75013 Paris (FR); FAYOL, Aude, F-75013 Paris (FR); VACHE, Julien, F-75013 Paris (FR); YAICHE, Philippe, F-75013 Paris (FR)
(74) Mandataire: Le Coupanec, Pascale A.M.P.
(86) Numéro de dépôt international: PCT/FR2010/050913
(87) Numéro de publication internationale: WO 2010/130944

(56) Documents cités:
- WO-A2-2005/070910
- JP-A- 2009 040 709
- US-A1- 2004 152 724
- US-A1- 2006 047 114

## Description

L'invention a pour objet des dérivés de cyclopenta[c]pyrrole-2-carboxylates, leur préparation et leur application en thérapeutique.

Il existe toujours une nécessité de trouver et de développer des produits inhibiteurs de l'enzyme FAAH (Fatty Acid Amide Hydrolase). Les composés de l'invention répondent à ce but.

Les composés de l'invention répondent à la formule générale (I) : 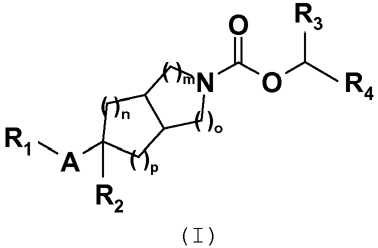 dans laquelle
R₂ représente un atome d'hydrogène, de fluor ou un groupe hydroxyle, cyano, trifluorométhyle, C₁₋₆-alkyle, C₁₋₆-alcoxy, NR₈R₉ ;
m, n, o et p ont pour valeur 1, ou bien, m et o ont pour valeur 1, n a pour valeur 0 et p a pour valeur 2;
A représente une liaison covalente ou un atome d'oxygène;
R₁ représente un groupe R₅ non substitué ou substitué par un ou plusieurs groupes R₆ et/ou R₇ ;
R₅ représente un groupe choisi parmi un phényle, pyridinyle, pyridazinyle, pyrimidinyle, pyrazinyle, triazinyle, naphtalènyle, quinolinyle, isoquinolinyle, phtalazinyle, quinazolinyle, quinoxalinyle, cinnolinyle, naphthyridinyle, benzothiazolyle, benzoxazolyle, benzimidazolyle, benzisothiazolyle, bensisoxazolyle, indazolyle, benzotriazolyle ;
R₆ représente un atome d'halogène, un groupe cyano, -CH₂CN, nitro, hydroxyle, C₁₋₆-alkyle, C₁₋₆-alcoxy, C₁₋₆-thioalkyle, C₁₋₆-haloalkyle, C₁₋₆-haloalcoxy, C₁₋₆-halothioalkyle, C₃₋₇-cycloalkyle, C₃₋₇-cycloalkyle-C₁₋₃-alkylène, C₃₋₇-cycloalkyle-C₁-alkylène-O-, NR₈R₉, NR₈COR₉, NR₈CO₂R₉, NR₈SO₂R₉, NR₈SO₂NR₈R₉, COR₈, CO₂R₈, CONR₈R₉, SO₂R₈, SO₂NR₈R₉ ou -O-(C₁₋₃-alkylène)-O- ;
R₇ représente un groupe choisi parmi un phényle, pyridinyle, pyridazinyle, pyrimidinyle, pyrazinyle, triazinyle; le ou les groupes R₇ pouvant être substitués par un ou plusieurs groupes R₆ identiques ou différents l'un de l'autre ;
R₃ représente un atome d'hydrogène, de fluor, un groupe C₁₋₆-alkyle ou un groupe trifluorométhyle ;
R₄ représente un groupe choisi parmi un thiazolyle, oxazolyle, isoxazolyle, thiadiazolyle, triazolyle;
ce groupe étant non substitué ou substitué par un ou plusieurs groupes C₁₋₆-alkyle, CONR₈R₉ ou CON(R₈) (C₁₋₃-alkylène-NR₁₀R₁₁) ;
R₈, R₉, R₁₀ et R₁₁ représentent indépendamment l'un de l'autre un atome d'hydrogène, ou un groupe C₁₋₆-alkyle,
A l'état de base ou de sel d'addition à un acide.

Parmi les composés de formule générale (I), un premier sous-groupe de composés est constitué des composés pour lesquels R₂ représente un atome d'hydrogène ou un groupe hydroxyle. Parmi les composés de formule générale (I), un second sous-groupe de composés est constitué des composés pour lesquels R₂ représente un atome d'hydrogène.

Parmi les composés de formule générale (I), un deuxieme sous-groupe de composés est constitué des composés pour lesquels m, n, o et p ont pour valeur 1.

Parmi les composés de formule générale (I), un troisième sous-groupe de composés est constitué des composés pour lesquels A représente un atome d'oxygène.

Parmi les composés de formule générale (I), un quatrième sous-groupe de composés est constitué des composés pour lesquels R₁ représente un groupe R₅ non substitué ou substitué par un ou plusieurs groupes R₆ et/ou R₇ ;
R₅ représente un groupe phényle, naphtalènyle, benzothiazolyle ou isoquinolinyle ;
R₆ représente un atome d'halogène, plus particulièrement un atome de fluor ou de chlore, ou un groupe C₁₋₆-haloalkyle, plus particulièrement trifluorométhyle, ou un groupe C₁₋₆-alcoxy, plus particulièrement un groupe méthoxy ou éthoxy;
R₇ représente un phényle pouvant être substitué par un ou plusieurs groupes R₆ identiques ou différents l'un de l'autre.

Parmi les composés de formule générale (I), un cinsuième sous-groupe de composés est constitué des composés pour lesquels R₁ représente un groupe R₅ substitué par un ou plusieurs groupes R₆ et/ou R₇ ;
R₅ représente un groupe phényle;
R₆ représente un atome d'halogène, plus particulièrement un atome de fluor ou de chlore, ou un groupe C₁₋₆-haloalkyle, plus particulièrement trifluorométhyle;
R₇ représente un phényle pouvant être substitué par un ou plusieurs groupes R₆ identiques ou différents l'un de l'autre choisi parmi un atome d'halogène, plus particulièrement un atome de fluor ou de chlore, ou un groupe C₁₋₆-alcoxy, plus particulièrement un groupe méthoxy ou éthoxy.

Parmi les composés de formule générale (I), un sixième sous-groupe de composés est constitué des composés pour lesquels R₁ représente un groupe R₅ substitué par un ou plusieurs groupes R₆ ;
R₅ représente un groupe naphtalèn-2-yle;
R₆ représente un groupe C₁₋₆-alcoxy, plus particulièrement un groupe méthoxy ou éthoxy.

Parmi les composés de formule générale (I), un septième sous-groupe de composés est constitué des composés pour lesquels R₁ représente un groupe R₅ substitué par un ou plusieurs groupes R₆ ;
R₅ représente un groupe naphtalèn-1-yle;
R₆ représente un atome d'halogène, plus particulièrement un atome de fluor ou de chlore.

Parmi les composés de formule générale (I), un huitième sous-groupe de composés est constitué des composés pour lesquels R₁ représente un groupe R₅ substitué par un ou plusieurs groupes R₆ ;
R₅ représente un groupe benzothiazol-2-yle;
R₆ représente un atome d'halogène, plus particulièrement un atome de fluor ou de chlore.

Parmi les composés de formule générale (I), un neuvième sous-groupe de composés est constitué des composés pour lesquels R₁ représente un groupe R₅ non substitué ;
R₅ représente un groupe isoquinolin-7-yle.

Parmi les composés de formule générale (I), un dixième sous-groupe de composés est constitué des composés pour lesquels R₁ représente un groupe R₅ non substitué ;
R₅ représente un groupe isoquinolin-6-yle.

Parmi les composés de formule générale (I), un onzième sous-groupe de composés est constitué des composés pour lesquels R₃ représente un atome d'hydrogène.

Parmi les composés de formule générale (I), un douzième sous-groupe de composés est constitué des composés pour lesquels R₄ représente un groupe thiazol-4-yle; ce groupe étant non substitué.

Parmi les composés de formule générale (I), un treizième sous-groupe de composés est constitué des composés pour lesquels R₄ représente un groupe thiazol-2-yle;
ce groupe étant non substitué ou substitué par un ou plusieurs groupes CONR₈R₉;
R₈ et R₉ représentent indépendamment l'un de l'autre un atome d'hydrogène ou un groupe C₁₋₆-alkyle. Plus particulièrement, le groupe C₁₋₆-alkyle est un méthyle.

Parmi les composés de formule générale (I), un quatorzième sous-groupe de composés est constitué des composés pour lesquels R₄ représente un groupe 1,2,3-thiadiazol-4-yle; ce groupe étant non substitué.

Parmi les composés de formule générale (I), un quinzième sous-groupe de composés est constitué des composés pour lesquels R₄ représente un groupe 1,3,4-thiadiazol-2-yle; ce groupe étant substitué par un ou plusieurs groupes C₁₋₆-alkyle.

Parmi les composés de formule générale (I), un seizième sous-groupe de composés est constitué des composés pour lesquels R₄ représente un groupe isoxazol-5-yle;
ce groupe étant substitué par un ou plusieurs groupes CONR₈R₉ ou CON (R₈) (C₁₋₃-alkylène-NR₁₀R₁₁) ;
R₈, R₉, R₁₀ et R₁₁ représentent indépendamment l'un de l'autre un atome d'hydrogène ou un groupe C₁₋₆-alkyle. Plus particulièrement, le groupe C₁₋₆-alkyle est un méthyle ou éthyle.

Parmi les composés de formule générale (I), un dix-septème sous-groupe de composés est constitué des composés pour lesquels R₄ représente un groupe 1H-1,2,4-triazol-5-yle; ce groupe étant substitué par un ou plusieurs groupes C₁₋₆-alkyle. Parmi les composés de formule générale (I), un dix-huitième sous-groupe de composés est constitué des composés pour lesquels R₄ représente un groupe oxazol-2-yle;
ce groupe étant non substitué ou substitué par un ou plusieurs groupes CONR₈R₉;
R₈ et R₉ représentent indépendamment l'un de l'autre un atome d'hydrogène ou un groupe C₁₋₆-alkyle. Plus particulièrement, le groupe C₁₋₆-alkyle est un méthyle.

Parmi les composés de formule générale (I), un dix-neuvième sous-groupe de composés est constitué par les composés de formule générale (I) dans laquelle à la fois R₁ et/ou R₂ et/ou R₃ et/ou R₄ et/ou n et/ou m et/ou o et/ou p et/ou A sont tels que définis dans les groupes ci-dessus.

Parmi les composés de formule générale (I), les composés suivants peuvent être cités (nomenclature IUPAC générée par le logiciel AutoNom):
1. (3a*R*,5*s,*6a*S*)-5-(4-chlorophénoxy)hexahydrocyclopenta[c]pyrrole-2(1*H*)-carboxylate de thiazol-2-ylméthyle (exo)
2. (3a*R*,5*s*,6a*S*)-5-[4-(trifluorométhyl)phénoxylhexahydrocyclopenta[*c*]pyrrole-2(1*H*)-carboxylate de thiazol-2-ylméthyle (exo)
3. (3aR,5s,6aS)-5-[(7-éthoxynaphthalèn-2-yl)oxy]hexahydrocyclopenta[*c*]pyrrole-2(1*H*)-carboxylate de thiazol-2-ylméthyle (exo)
4. (3a*R*,5*r*,6a*S*)-5-[3-(trifluorométhyl)phénoxy]hexahydrocyclopenta[*c*]pyrrole-2(1*H*)-carboxylate de thiazol-2-ylméthyle (endo)
5. (3a*R*,5*s*,6a*S*)-5-[3-(trifluoromethyl)phenoxy]hexahydrocyclopenta[*c*]pyrrole-2(1*H*)-carboxylate de thiazol-2-ylméthyle (exo)
6. (3a*R*,5r,6a*S*)-5-[(4'-éthoxybiphényl-3-yl)oxy]hexahydrocyclopenta[*c*]pyrrole-2(1*H*)-carboxylate de thiazol-2-ylméthyle (endo)
7. (3a*R*,5*s*,6a*S*)-5-(4-chlorophénoxy)hexahydrocyclopenta[*c*]pyrrole-2(1*H*)-carboxylate de 1,2,3-thiadiazol-4-ylméthyle (exo)
8. (3a*R*,5*s*,6a*S*)-5-(4-chlorophénoxy)hexahydrocyclopenta[*c*]pyrrole-2(1*H*)-carboxylate de (5-*tert*-butyl-1,3,4-thiadiazol-2-yl)méthyle (exo)
9. (3a*R*,5*s*,6a*S*)-5-(4-fluorophénoxy)hexahydrocyclopenta[*c*]pyrrole-2(1*H*)-carboxylate de 1,2,3-thiadiazol-4-ylméthyle (exo)
10. (3a*R*,5*s*,6a*S*)-5-(4-fluorophénoxy)hexahydrocyclopenta[*c*]pyrrole-2(1*H*)-carboxylate de (3-carbamoylisoxazol-5-yl)méthyle (exo)
11. (3a*R*,5*s*,6a*S*)-5-(4-chlorophénoxy)hexahydrocyclopenta[*c*]pyrrole-2(1*H*)-carboxylate de [3-(méthylcarbamoyl)isoxazol-5-yl]méthyle (exo)
12. (3a*R*,5*s*,6a*S*)-5-(4-chlorophénoxy)hexahydrocyclopenta[*c*]pyrrole-2(1*H*)-carboxylate de (3-carbamoylisoxazol-5-yl)méthyle (exo)
13. (3a*R*,5*s*,6a*S*)-5-(4-chlorophénoxy)hexahydrocyclopenta[*c*]pyrrole-2(1*H*)-carboxylate de (1-méthyl-1*H*-1,2,4-triazol-5-yl)méthyle (exo)
14. (3a*R*,5*s*,6a*S*)-5-(4-chlorophénoxy)hexahydrocyclopenta[*c*]pyrrole-2(1*H*)-carboxylate de (4-carbamoyl-thiazol-2-yl)méthyle (exo)
15. (3a*R*,5*s*,6a*S*)-5-(4-chlorophénoxy)hexahydrocyclopenta[*c*]pyrrole-2(1*H*)-carboxylate de [4-(méthylcarbamoyl)- thiazol-2-yl]méthyle (exo)
16. (3a*R*,5*s*,6a*S*)-5-[3-(trifluoroméhyl)phénoxylhexahydrocyclopenta[*c*]pyrrole-2(1*H*)-carboxylate de (3-carbamoylisoxazol-5-yl)méthyle (exo)
17. (3a*R*,5*s*,6a*S*)-5-[3-(trifluorométhyl)phénoxy]hexahydrocyclopenta[*c*]pyrrole-2(1*H*)-carboxylate de [3-(méthylcarbamoyl)isoxazol-5-yl]méthyle (exo)
18. (3a*R*,5*s*,6a*S*)-5-[(4'-éthoxybiphényl-3-yl)oxy]hexahydrocyclopenta[*c*]pyrrole-2(1*H*)-carboxylate de thiazol-2-ylméthyle (exo)
19. (3a*R*,5*s*,6a*S*)-5-(4-chlorophénoxy)hexahydrocyclopenta[*c*]pyrrole-2(1*H*)-carboxylate de (4-carbamoyl-oxazol-2-yl)méthyle (exo)
20. (3a*R*,4*S*,6a*S*)-4-[(6-méthoxynaphthalèn-2-yl)oxy]hexahydrocyclopenta[*c*]pyrrole-2(1*H*)-carboxylate de thiazol-4-ylméthyle (exo)
21. (3a*R*,4*R*,6a*S*)-4-[3-(trifluorométhyl)phenoxy]hexahydrocyclopenta[*c*]pyrrole-2(1*H*)-carboxylate de thiazol-4-ylméthyle (endo)
22. (3a*R*,5*s*,6a*S*)-5-[(7-éthoxynaphthalèn-2-yl)oxy]hexahydrocyclopenta[*c*]pyrrole-2(1*H*)-carboxylate de (4-carbamoyl-thiazol-2-yl)méthyle (exo)
23. (3a*R*,5*s*,6a*S*)-5-[(7-éthoxynaphthalèn-2-yl)oxy]hexahydrocyclopenta[*c*]pyrrole-2(1*H*)-carboxylate de [4-(méthylcarbamoyl)- thiazol-2-yl]méthyle (exo)
24. (3a*R*,5*s*,6a*S*)-5-[(7-éthoxynaphthalèn-2-yl)oxy]hexahydrocyclopenta[*c*]pyrrole-2(1*H*)*-*carboxylate de (3-carbamoylisoxazol-5-yl)méthyle (exo)
25. (3a*R*,5*s*,6a*S*)-5-[(7-éthoxynaphthalèn-2-yl)oxy]hexahydrocyclopenta[*c*]pyrrole-2(1*H*)-carboxylate de [3-(méthylcarbamoyl)isoxazol-5-yl]méthyle (exo)
26. (3a*R*,5*s*,6a*S*)-5-(4-chlorophénoxy)hexahydrocyclopenta[*c*]pyrrole-2(1*H*)-carboxylate de [4-(méthylcarbamoyl)-oxazol-2-yl]méthyle (exo)
27. (3a*R*,5*s*,6a*S*)-5-[(4-chloronaphthalèn-1-yl)oxy]hexahydrocyclopenta[*c*]pyrrole-2(1*H*)-carboxylate de (3-carbamoylisoxazol-5-yl)méthyle (exo)
28. (3a*R*,5*s*,6a*S*)-5-[(4-chloronaphthalèn-1-yl)oxy]hexahydrocyclopenta[*c*]pyrrole-2(1*H*)-carboxylate de [3-(méthylcarbamoyl)isoxazol-5-yl]méthyle (exo)
29. (3a*R*,4*S*,6a*S*)-4-[(4-chloronaphthalèn-1-yl)oxy]hexahydrocyclopenta[*c*]pyrrole-2(1*H*)-carboxylate de thiazol-4-ylméthyle (exo)
30. (3a*R*,4*R*,6a*S*)-4-[(4'-éthoxybiphényl-3-yl)oxy]hexahydrocyclopenta[*c*]pyrrole-2(1*H*)-carboxylate de thiazol-4-ylméthyle (endo)
31. (3a*R*,5*s*,6a*S*)-5-[(4'-fluorobiphényl-4-yl)oxy]hexahydrocyclopenta[*c*]pyrrole-2(1*H*)-carboxylate de (3-carbamoylisoxazol-5-yl)méthyle (exo)
32. (3a*R*,5s,6a*S*)-5-(4-chloro-2-fluorophénoxy)hexahydrocyclopenta[*c*]pyrrole-2(1*H*)-carboxylate de [3-(méthylcarbamoyl)isoxazol-5-yl]méthyle (exo)
33. (3a*R*,5*s*,6a*S*)-5-(4-chloro-2-fluorophénoxy)hexahydrocyclopenta[*c*]pyrrole-2(1*H*)-carboxylate de (3-carbamoylisoxazol-5-yl)méthyle (exo)
34. (3a*R*,5*s*,6a*S*)-5-(4-chloro-3-fluorophénoxy)hexahydrocyclopenta[*c*]pyrrole-2(1*H*)-carboxylate de (3-carbamoylisoxazol-5-yl)méthyle (exo)
35. (3a*R*,5*s*,6a*S*)-5-(4-chloro-3-fluorophénoxy)hexahydrocyclopenta[*c*]pyrrole-2(1*H*)-carboxylate de [3-(méthylcarbamoyl)isoxazol-5-yl]méthyle (exo)
36. (3a*R*,5*s*,6a*S*)-5-(2,4-dichlorophénoxy)hexahydrocyclopenta[*c*]pyrrole-2(1*H*)-carboxylate de (3-carbamoylisoxazol-5-yl)méthyle (exo)
37. (3a*R*,5*s*,6a*S*)-5-(2,4-dichlorophénoxy)hexahydrocyclopenta[*c*]pyrrole-2(1*H*)-carboxylate de [3-(méthylcarbamoyl)isoxazol-5-yl]méthyle (exo)
38. (3a*R*,5*s*,6a*S*)-5-(isoquinolin-7-yloxy)hexahydrocyclopenta[*c*]pyrrole-2(1*H*)-carboxylate de (3-carbamoylisoxazol-5-yl)méthyle (exo)
39. (3a*R*,5*s*,6a*S*)-5-(isoquinolin-6-yloxy)hexahydrocyclopenta[*c*]pyrrole-2(1*H*)-carboxylate de (3-carbamoylisoxazol-5-yl)méthyle (exo)
40. (3a*R*,5*r*,6a*S*)-5-(4-fluoro-1,3-benzothiazol-2-yl)-5-hydroxyhexahydrocyclopenta[*c*]pyrrole-2(1*H*)-carboxylate de (3-carbamoylisoxazol-5-yl)méthyle (exo)
41. (3a*R*,5*s*,6a*S*)-5-[(7-éthoxynaphthalèn-2-yl)oxy]hexahydrocyclopenta[*c*]pyrrole-2(1*H*)-carboxylate de (4-carbamoyl-oxazol-2-yl)méthyle (exo)
42. (3a*R*,5*s*,6a*S*)-5-(4-fluorophénoxy)hexahydrocyclopenta[*c*]pyrrole-2(1*H*)-carboxylate de (3-{[2-(diméthylamino)éthyl]carbamoyl}isoxazol-5-yl)méthyle (exo), et son chlorhydrate
43. (3a*R*,5*s*,6a*S*)-5-[(7-éthoxynaphthalèn-2-yl)oxy]hexahydrocyclopenta[*c*]pyrrole-2(1*H*)-carboxylate de [4-(méthylcarbamoyl)-oxazol-2-yl]méthyle (exo)
44. (3a*R*,5*s*,6a*S*)-5-(4-fluorophénoxy)hexahydrocyclopenta[*c*]pyrrole-2(1*H*)-carboxylate de (3-diméthylcarbamoylisoxazol-5-yl)méthyle (exo)
45. (3a*R*,5*s*,6a*S*)-5-[(4'-fluorobiphényl-4-yl)oxy]hexahydrocyclopenta[*c*]pyrrole-2(1*H*)-carboxylate de (3-méthylcarbamoylisoxazol-5-yl)méthyle (exo)

Les composés de formule générale (I) peuvent comporter un ou plusieurs carbones asymétriques. Ils peuvent exister sous forme d'énantiomères ou de diastéréoisomères. Les composés de formule générale (I) peuvent également exister sous forme de stéréoisomères *cis* ou *trans.* Ces stéréoisomères, énantiomères et diastéréoisomères, ainsi que leurs mélanges, y compris les mélanges racémiques, font partie de l'invention. Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Dans le cadre de l'invention, on entend par :
- C_{t-z} où t et z peuvent prendre les valeurs de 1 à 8, une chaîne carbonée pouvant avoir de t à z atomes de carbone, par exemple C₁₋₃ une chaîne carbonée qui peut avoir de 1 à 3 atomes de carbone ;
- alkyle, un groupe aliphatique saturé, linéaire ou ramifié; par exemple un groupe C₁₋₆-alkyle représente une chaîne carbonée de 1 à 6 atomes de carbone, linéaire ou ramifiée, plus particulièrement un méthyle, éthyle, propyle, isopropyle, butyle, isobutyle, *tert*-butyle, pentyle, hexyle ;
- alkylène, un groupe alkyle divalent saturé, linéaire ou ramifié, par exemple un groupe C₁₋₃-alkylène représente une chaîne carbonée divalente de 1 à 3 atomes de carbone, linéaire ou ramifiée, plus particulièrement un méthylène, éthylène, 1-méthyléthylène, propylène ;
- cycloalkyle, un groupe alkyle cyclique, par exemple un groupe C₃₋₇-cycloalkyle représente un groupe carboné cyclique de 3 à 7 atomes de carbone, plus particulièrement un cyclopropyle, cyclobutyle, cyclopentyle, cyclohexyle, cycloheptyle ;
- alcoxy, un groupe -O-alkyle à chaîne aliphatique saturée, linéaire ou ramifiée ;
- thioalkyle, un groupe -S-alkyle à chaîne aliphatique saturée, linéaire ou ramifiée ;
- haloalkyle, un groupe alkyle dont un ou plusieurs atomes d'hydrogène ont été substitués par un atome d'halogène ;
- haloalcoxy, un groupe alcoxy dont un ou plusieurs atomes d'hydrogène ont été substitués par un atome d'halogène ;
- halothioalkyle, un groupe thioalkyle dont un ou plusieurs atomes d'hydrogène ont été substitués par un atome d'halogène ;
- atome d'halogène, un fluor, un chlore, un brome ou un iode.
- Le terme '*exo*' correspond au groupement -A-R1 en cis par rapport aux hydrogènes de jonction de cycle. Le terme '*endo*' correspond au groupement -A-R1 en trans par rapport aux hydrogènes de jonction de cycle.
- r et s indique la stéréochimie des atomes de carbones pseudo-asymétriques, selon les règles IUPAC.

Les composés de l'invention peuvent être préparés selon différentes méthodes, illustrées par les schémas qui suivent. Ces méthodes, ainsi que les composés intermédiaires utilisés, sont un objet de la présente invention.

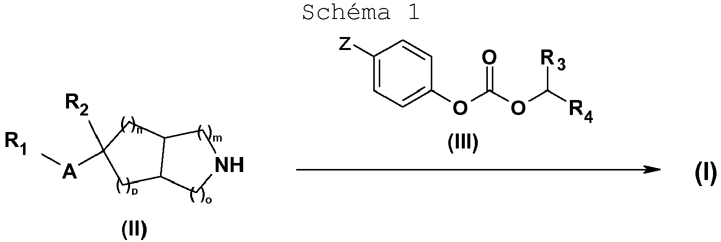

Ainsi une première méthode de préparation (schéma 1) consiste à faire réagir une amine de formule générale (II), dans laquelle A, R₁, R₂, m, n, o et p sont tels que définis dans la formule générale (I) définie ci-dessus, avec un carbonate de formule générale (III) dans laquelle Z représente un atome d'hydrogène ou un groupe nitro, R₃ et R₄ sont tels que définis dans la formule générale (I) définie ci-dessus, en présence d'une base telle que la triéthylamine, la pyridine, la *N,N-*diméthylaminopyridine ou la *N,N*-diisopropyléthylamine, dans un solvant tel que le toluène, l'acétonitrile ou le dichloroéthane, à une température comprise entre la température ambiante et la température de reflux du solvant.

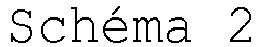

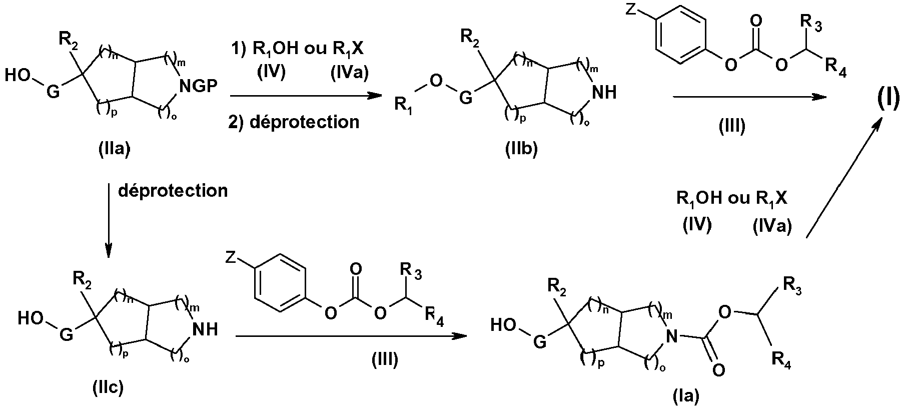

Une seconde méthode de préparation (schéma 2) pour obtenir les composes de formule générale (I) dans laquelle A représente plus particulièrement un atome d'oxygène, consiste à faire réagir, dans un premier temps, un alcool de formule générale (IIa), dans laquelle R₂, m, n, o et p sont tels que définis dans la formule générale (I) définie ci-dessus, G représente une partie du groupe A tel que défini dans la formule générale (I), à savoir une liaison covalente, et GP représente un groupe protecteur tel qu'un Boc (tert-butyloxycarbonyl), un Cbz (benzyloxycarbonyl), un benzyle ou un benzhydrile ;
- soit avec un dérivé alcool de formule générale R₁OH (IV), dans laquelle R₁ est tel que défini ci-dessus en utilisant les conditions de réaction de Mitsunobu *(*Synthesis, 1981, 1-28),
- soit avec un dérivé halogéné de formule générale R₁X (IVa), dans laquelle R₁ est tel que défini ci-dessus et X représente un atome de fluor, de chlore, de brome ou d'iode en utilisant les réactions de substitution nucléophiles aromatiques, hétéroaromatiques ou de *O-*arylation, *O*-hétéroarylation de Buchwald, par exemple au moyen d'un catalyseur au Palladium ou au Cuivre ;
suivie d'une réaction de déprotection par exemple en présence d'acide trifluoroacétique ou d'une solution d'acide chlorhydrique dans l'isopropanol ou le dioxane, pour conduire à l'amine de formule générale (IIb) dans laquelle G, R₂, m, n, o et p sont tels que définis dans la formule générale (IIa) définie ci-dessus et R₁ est tel que défini ci-dessus. Le dérivé de formule générale (IIb) ainsi obtenu est ensuite transformé en composé de formule générale (I) selon une réaction de condensation avec un carbonate de formule générale (III) telle que définie ci-dessus, dans les conditions décrites ci-dessus (schéma 1).

Une variante d'obtention des composés de formule générale (I) (schéma 2) dans laquelle A représente plus particulièrement un atome d'oxygène, consiste à déprotéger un alcool de formule générale (IIa) tel que défini ci-dessus, selon une réaction de déprotection telle que définie ci-dessus afin d'obtenir un aminoalcool de formule générale (IIc) puis à faire réagir cet aminoalcool de formule générale (IIc) dans laquelle G, R₂, m, n, o et p sont tels que définis dans la formule générale (IIa) définie ci-dessus, avec un carbonate de formule générale (III) telle que définie ci-dessus, dans les conditions décrites ci-dessus (schéma 1), pour conduire au dérivé carbamate de formule générale (Ia), dans laquelle G, R₂, R₃, R₄, m, n, o et p sont tels que définis dans la formule générale (IIa) définie ci-dessus. Le dérivé carbamate (Ia) ainsi obtenu est ensuite transformé en composé de formule générale (I), par action d'un alcool de formule générale R₁OH (IV) telle que définie ci-dessus, en utilisant les conditions de réaction de Mitsunobu ou par action d'un dérivé halogéné de formule générale R₁X (IVa tel que défini ci-dessus en utilisant les réactions de substitution nucléophiles aromatiques, hétéroaromatiques ou de *O*-arylation, *O*-hétéroarylation de Buchwald, par exemple au moyen d'un catalyseur au Palladium ou au Cuivre.

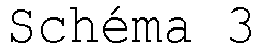

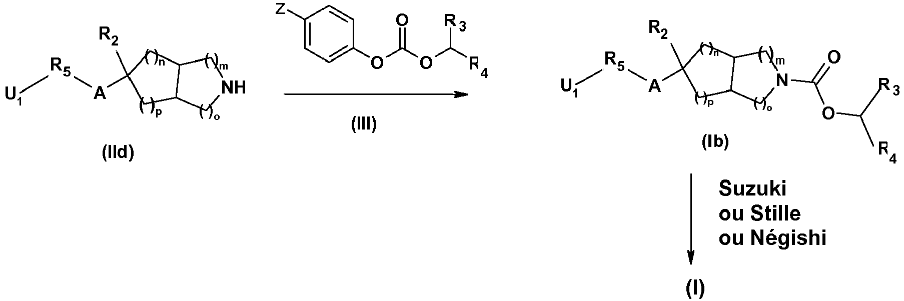

Une troisième méthode (schéma 3) a été développée pour la synthèse des composés de formule générale (I), dans laquelle R₁ représente un groupe R₅ substitué notamment par un groupe R₆ de type C₁₋₆-alkyle, C₃₋₇-cycloalkyle ou C₃₋₇-cycloalkyle-C₁₋₃-alkylène, ou par un groupe R₇ tel que défini dans la formule générale (I) définie ci-dessus. Ainsi, la première étape consiste à faire réagir une amine de formule générale (IId), dans laquelle A, R₂, R₅, m, n, o et p sont tels que définis dans la formule générale (I) définie ci-dessus et U₁ représente un atome de chlore, de brome, d'iode ou un groupement triflate, avec un carbonate de formule générale (III) telle que définie ci-dessus, dans les conditions décrites ci-dessus (schéma 1), pour conduire au dérivé carbamate de formule générale (Ib), dans laquelle A, R₂, R₃, R₄, R₅, m, n, o et p sont tels que définis dans la formule générale (I) définie ci-dessus et U₁ est tel que défini ci-dessus. La réaction de couplage catalysée au moyen d'un métal de transition comme le Palladium (0) est ensuite réalisée sur l'intermédiaire clé de formule générale (Ib) telle que définie ci-dessus, U₁ étant dans la position où l'on souhaite introduire le groupe R₆ ou R₇ (schéma 3):
- soit par une réaction de type Suzuki, par exemple au moyen d'un acide boronique d'alkyle, de cycloalkyle, d'aryle ou d'hétéroaryle,
- soit selon une réaction de type Stille, par exemple en utilisant un dérivé *tri*-alkylstanneux d'aryle ou d'hétéroaryle
- soit par une réaction de type Negishi, par exemple en utilisant un dérivé zincate d'halogénure d'alkyle, de cycloalkyle, d'aryle ou d'hétéroaryle.

Un autre objet de la présente invention se rapporte aux composés de formule générale (Ia) 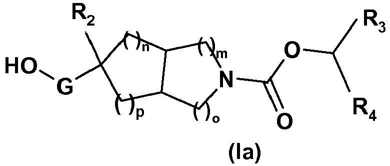 dans laquelle R₂, R₃, R₄, m, n, o et p sont tels que définis dans la formule générale (I), et G représente une partie du groupe A tel que défini dans la formule générale (I), à savoir une liaison covalente.

Parmi ces composés, on peut citer :
(3aR,5r,6aS)-5-hydroxyhexahydrocyclopenta [*c*]pyrrole-2(1*H*)-carboxylate de [3-(éthoxycarbonyl)isoxazol-5-yl]méthyle ;
(3a*R*,5*r*,6a*S*)-5-hydroxyhexahydro cyclopenta[*c*]pyrrole-2(1*H*)-carboxylate de thiazol-2-ylméthyle.

Un autre objet de la présente invention se rapporte aux composés de formule générale (II) : 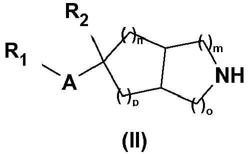 dans laquelle R₁, R₂, m, n, o et p sont tels que définis dans la formule générale (I), A représente un atome d'oxygène ou une liaison covalente, étant donné que lorsque A représente une liaison covalente alors R1 représente un groupe benzothiazolyle.

Parmi ces composés, on peut citer :
(3aR,5s,6aS)-5-[3-(trifluorométhyl)phénoxy]octahydrocyclo penta[*c*]pyrrole ;
(3aR,5s,6aS)-5-(4-fluorophénoxy)octahydrocyclopenta [c]pyrrole (*RMN 1H 400Mhz DMSO,* δ (ppm) : 7,10 (t, 2H) ; 6,95 (m, 2H) ; 4,85 (m, 1H) ; 2,90 (m, 2H) ; 2,75 (m, 4H) ; 2,00 (m, 2H) ; 1,70 (m, 2H));
(3aR,5r,6aS)-5-(4-fluoro-1,3-benzothiazol-2-yl)octahydro cyclopenta[c]pyrrol-5-ole ;
(3aR,5s,6aS)-5-(4-Chloro-3-fluoro-phénoxy)-octahydro-cyclopenta[c]pyrrole;
(3aR,5s,6aS)-5-(4-chlorophénoxy)octahydrocyclopenta [c]pyrrole;
(3aR,5s,6aS)-5-[(7-éthoxynaphthalèn-2-yl)oxy]octahydrocyclo penta[c]pyrrole;
(3a*R*,5*r*,6a*S*)-5-[(4'-éthoxybiphényl-3-yl)oxy]octahydrocyclo penta[*c*]pyrrole;
(3a*R*,5*s*,6a*S*)-5-[(4'-éthoxybiphényl-3-yl)oxy]octahydrocyclo penta[*c*]pyrrole;
(3a*R*,4*S*,6a*S*)-4-[(4-chloronaphthalèn-1-yl)oxy]octahydrocyclo penta[*c*]pyrrole;
(3a*R*,4*R*,6a*S*)-4-[(4'-éthoxybiphényl-3-yl)oxy]octahydrocyclo penta[*c*]pyrrole.

Un autre objet de la présente invention se rapporte aux composés de formule générale (IIe) : 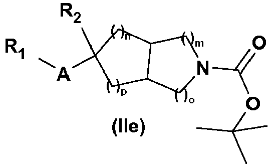 dans laquelle R₁, R₂, m, n, o et p sont tels que définis dans la formule générale (I), A représente un atome d'oxygène ou une liaison covalente, étant donné que lorsque A représente une liaison covalente alors R1 représente un groupe benzothiazolyle.

Parmi ces composés, on peut citer :
(3a*R*,5*r*,6a*S*)-5-[(4'-éthoxybiphényl-3-yl)oxy]hexahydrocyclopenta[*c*]pyrrole-2(1*H*)-carboxylate de *tert*-butyle ;
(3a*R*,5*s*,6a*S*)-5-(3-bromophénoxy) hexahydrocyclopenta[*c*]pyrrole-2(1*H*)-carboxylate de *tert-*butyle ;
(3a*R*,5*s*,6a*S*)-5-[(4'-éthoxybiphényl-3-yl)oxy]hexahydrocyclopenta[*c*]pyrrole-2(1*H*)-carboxylate de *tert*-butyle ;
(3a*R*,4*S*,6a*S*)-4-[(4-chloronaphthalèn-1-yl)oxy]hexahydrocyclopenta[*c*]pyrrole-2(1*H*)-carboxylate de *tert*-butyle ;
(3a*R*,4*R*,6a*S*)-4-(3-bromophénoxy)hexahydro cyclopenta[*c*]pyrrole-2(1*H*)-carboxylate de *tert*-butyle ;
(3a*R*,4*R*,6a*S*)-4-[(4'-éthoxybiphényl-3-yl)oxy]hexahydrocyclopenta[*c*]pyrrole-2(1*H*)-carboxylate de *tert*-butyle ;
(3a*R*,5*r*,6a*S*)-5-(4-fluoro-1,3-benzothiazol-2-yl)-5-hydroxyhexahydrocyclopenta[*c*]pyrrole-2(1*H*)-carboxylate de *tert*-butyle.

Les autres composés de formules générales (II), (IIa), (IIb), (IIc), (IId), (III), (IV) et (IVa) ainsi que les autres réactifs sont disponibles dans le commerce ou décrits dans la littérature, ou bien peuvent être préparés selon des méthodes qui y sont décrites ou qui sont connues de l'homme du métier.

Les exemples qui suivent illustrent la préparation de quelques composés de l'invention. Ces exemples ne sont pas limitatifs et ne font qu'illustrer l'invention. Les microanalyses, les spectres I.R. et R.M.N. et/ou la LC-MS (Liquid Chromatography coupled to Mass Spectroscopy) confirment les structures et les puretés des composés obtenus.
PF(°C) représente le point de fusion en degrés Celsius.
R_{f} indique le temps de rétention obtenu par analyse CCM (Chromatographie sur Couche Mince).
Les numéros indiqués entre parenthèses dans les titres des exemples correspondent à ceux de la 1ère colonne des tableaux ci-après.

La nomenclature UICPA (Union Internationale de Chimie Pure et Appliquée - IUPAC en anglais) a été utilisée pour la dénomination des composés dans les exemples ci-dessous.

### Exemple 1 (Composé N°6)

### (3aR,5r,6aS)-5-[(4'-éthoxybiphényl-3-yl)oxy]hexahydrocyclopenta[c]pyrrole-2(1H)-carboxylate de thiazol-2-ylméthyle (endo)

### 1.1. (3aR,5s,6aS)-5-(3-bromophénoxy)hexahydro cyclopenta[c]pyrrole-2(1H)-carboxylate de tert-butyle

0,20 g (0,88 mmole) de (3a*R*,5*r*,6a*S*)-5-hydroxyhexahydrocyclopenta[*c*]pyrrole-2(1*H*)-carboxylate de *tert*-butyle (obtenue selon un procédé décrit dans WO2006108059) sont mis en solution dans 4,4 mL de diméthylformamide puis 0,19 g (1,10 mmoles) de 1-bromo-3-iodobenzène et 0,03 g (1,32 mmoles) d'hydrure de sodium sont ajoutés. Le mélange est agité à 90°C pendant 15 heures. On dilue par ajout d'eau et d'acétate d'éthyle. On extrait avec de l'acétate d'éthyle puis on sèche les phases organiques réunies sur du sulfate de sodium puis on évapore à sec après filtration. On purifie le résidu par chromatographie sur gel de silice en éluant avec un mélange de cyclohexane et d'acétate d'éthyle. On obtient 0,175 g (52%) de produit attendu sous la forme d'une huile incolore.

### 1.2. (3aR,5r,6aS)-5-[(4'-éthoxybiphényl-3-yl)oxy]hexahydrocyclopenta[c]pyrrole-2(1H)-carboxylate de tert-butyle

Sous atmosphère inerte, sont introduits 0,170 g (0,44 mmole) de (3a*R*,5*s*,6a*S*)-5-(3-bromophénoxy)hexahydrocyclopenta[*c*]pyrrole-2(1*H*)-carboxylate de *tert*-butyle, obtenu à l'étape 1.1., 0,088 g (0,53 mmole) d'acide 4-éthoxyphénylboronique, 0,434 g (1,33 mmoles) de carbonate de césium dans 5 ml d'un mélange 9/1 de tétrahydrofurane et d'eau. 0,036 g (0,04 mmole) de PdCl₂dppf.CH₂Cl₂ sont ajoutés et le milieu est chauffé à 75°C pendant 15 heures. Le milieu est laissé revenir à température ambiante puis dilué avec de l'acétate d'éthyle et de l'eau. On sépare la phase aqueuse, on l'extrait deux fois avec de l'éthyle acétate, on lave les phases organiques réunies avec une solution aqueuse saturée en chlorure de sodium et on les sèche sur sulfate de sodium. Après évaporation du solvant, on purifie le résidu par chromatographie sur gel de silice en éluant avec un mélange de cyclohexane et d'acétate d'éthyle. On obtient 0,145g (77%) de produit attendu sous la forme d'une huile. LC-MS : M+H = 424

### 1.3. (3aR,5r,6aS)-5-[(4'-éthoxybiphényl-3-yl)oxy]octahydrocyclopenta[c]pyrrole

0,14 g (0,34 mmole) de (3a*R*,5*r*,6a*S*)-5-[(4'-éthoxybiphényl-3-yl)oxy]hexahydrocyclopenta [*c*]pyrrole-2(1*H*)-carboxylate de *tert*-butyle, obtenu à l'étape 1.2., est mis en solution dans du dichlorométhane puis 1,71 mL (6.85 mmoles) d'une solution 4 N d'acide chlorhydrique dans le dioxane sont ajoutés. Le mélange est agité à température ambiante pendant 2 heures. Un traitement avec de la soude 1M après extraction au dichlorométhane puis séchage sur sulfate de sodium et évaporation à sec, conduit à 0,084g d'une huile colorée.

### 1.4. (3aR,5r,6aS)-5-[(4'-éthoxybiphényl-3-yl)oxy]hexahydrocyclopenta[c]pyrrole-2(1H)-carboxylate de thiazol-2-ylméthyle

0,07 g (0,28 mmole) de (3a*R*,5*r*,6a*S*)-5-[(4'-éthoxybiphényl-3-yl)oxy]octahydrocyclopenta [*c*]pyrrole, obtenu à l'étape 1.3., est mis en solution dans 2,5 mL de dichlorométhane puis 0,06 mL (0,34 mmole) de N,N-diisopropyléthylamine et 0,09 g (0,31 mmole) de (4-nitro-phényle)- carbonate de thiazole-4-ylméthyle (WO2008013834) sont ajoutés. Le mélange est agité à température ambiante pendant 15 heures puis dilué dans l'acétate d'éthyle. La phase organique est successivement lavée avec une solution aqueuse 1M de soude puis deux fois avec une solution aqueuse saturée en chlorure de sodium, séchée sur sulfate de sodium, filtrée et évaporée à sec. Le résidu obtenu est purifié par chromatographie sur gel de silice en éluant avec un mélange de cyclohexane et d'acétate d'éthyle. On obtient 0,088 g (67%) de produit attendu sous la forme d'une huile jaune. LC-MS : M+H = 465
RMN-¹H (DMSO) δ (ppm) : 7,80 (d, 1H) ; 7,70 (d, 1H) ; 7,60 (d, 2H) ; 7,30 (t, 1H) ; 7,15 (d, 1H) ; 7,05 (s, 1H) ; 7,00 (d, 2H) ; 6,80 (d, 1H) ; 5,35 (S, 2H) ; 5,00 (m, 1H) ; 4,10 (q, 2H) ; 3,55 (m, 2H) ; 3,35 (m, 2H) ; 2,75 (m, 2H) ; 2,30 (m, 2H) ; 1,70 (m, 2H) ; 1,35 (t, 3H).

### Exemple 2 (Composé N°7)

### (3aR,5s,6aS)-5-(4-chlorophénoxy) hexahydrocyclopenta[c]pyrrole-2(1H)-carboxylate de 1,2,3-thiadiazol-4-ylméthyle (exo)

### 2.1. (3aR,5s,6aS)-5-(4-chlorophénoxy) octahydro cyclopenta[c]pyrrole

2,00 g (8,80 mmoles) de (3a*R*,5*r*,6a*S*)-5-hydroxyhexahydrocyclopenta[*c*]pyrrole-2(1*H*)-carboxylate de *tert*-butyle (voir synthèse: WO2006108059) sont mis en solution dans 88 mL de toluène. 2,77 g (10,56 mmoles) de triphénylphosphine et 1,47 g (11,44 mmoles) de 4-chlorophénol sont ajoutés, puis le milieu est refroidi à 0°C pour addition lente d'une solution de 1,69 g (9,68 mmoles) de diéthylazodicarboxylate dans 10 mL de toluène. Le milieu est agité 14 heures à température ambiante.
On concentre sous pression réduite. Le résidu obtenu est repris dans une solution aqueuse 1N de soude et extrait deux fois au dichlorométhane. Les phases organiques groupées sont lavées une fois avec une solution aqueuse saturée en chlorure de sodium, séchées sur sulfate de sodium, filtrées et concentrées sous vide. Le résidu obtenu est repris dans 50 mL de dichlorométhane pour déprotection par addition lente de 50 mL d'une solution 4N d'acide chlorhydrique dans le dioxane. Après 1 heure d'agitation à température ambiante, le milieu est concentré sous vide et le résidu est repris dans une solution aqueuse 1N d'acide chlorhydrique. La phase aqueuse est extraite deux fois à l'acétate d'éthyle puis lentement alcalinisée jusqu'à pH 10 par addition de carbonate de potassium. La phase aqueuse est extraite trois fois au dichlorométhane. Ces trois extraits organiques sont groupés, lavés une fois avec une solution aqueuse saturée en chlorure de sodium, séchés sur sulfate de sodium, filtrés et concentrés sous vide. Le résidu est purifié par chromatographie sur gel de silice en éluant avec un mélange 98/2/0,2 à 95/5/0,5 de dichlorométhane, de méthanol et d'ammoniaque 30%. On obtient 1,22g (61%) de produit attendu sous la forme d'une cire.
LC-MS : M+H = 238
RMN-¹H (DMSO) δ (ppm) : 7,35 (d, 2H) ; 7,00 (d, 2H) ; 4,95 (m, 1H) ; 3,55 (large s, 1H) ; 2,80 (m, 2H) ; 2,75-2,60 (m, 4H) ; 2,00 (m, 2H) ; 1,70 (m, 2H).

### 2.2. (3aR,5s,6aS)-5-(4-chlorophénoxy) hexahydro cyclopenta[c]pyrrole-2(1H)-carboxylate de 1,2,3-thiadiazol-4-ylméthyle

0,070 g (0,61 mmole) de 1,2,3-thiadiazole-4-méthanol (DD232495) et 0,18 mL (1,06 mmoles) de *N,N-*diisopropyléthylamine sont mis en solution dans 1 mL de 1,2-dichloroéthane puis refroidis à 0°C. 0,11 g (0,56 mmole) de chloroformiate de *p*-nitrophényle en solution dans 2 mL de 1,2-dichloroéthane sont additionnés. Le mélange est agité à température ambiante pendant 15 minutes puis une solution de 0,12 g (0,50 mmoles) de (3a*R*,5*s*,6a*S*)-5-(4-chlorophénoxy) octahydrocyclopenta[*c*]pyrrole, obtenu à l'étape 2.1., est ajoutée. Le mélange est chauffé à 60°C pendant 15 heures. Après retour à température ambiante, une solution aqueuse 1N de soude est ajoutée, et le produit est extrait avec du dichlorométhane. Les phases organiques réunies sont ensuite successivement lavées par une solution aqueuse saturée de chlorure d'ammonium, puis par une solution aqueuse saturée de chlorure de sodium. Après avoir séché les phases organiques sur sulfate de sodium, elles sont filtrées et évaporées à sec. Après purification sur colonne de gel de silice en éluant avec un mélange 99/1/0,1 de dichlorométhane, de méthanol et d'ammoniaque 30%, on obtient 0,068 g (58%) de produit attendu sous la forme d'une poudre blanche.
Point de fusion (°C) : 122-124°C
LC-MS : M+H = 380
RMN-¹H (DMSO) δ (ppm) : 9,20 (s, 1H) ; 7,30 (d, 2H); 6,90 (d, 2H) ; 5,55 (s, 2H) ; 4,95 (m, 1H) ; 3,55 (m, 2H) ; 3,20 (m, 2H) ; 2,80 (m, 2H); 2,05-1,90 (m, 2H); 1,90-1,80 (m, 2H).

### Exemple 3 (Composé N°8)

### (3aR,5s,6aS)-5-(4-chlorophénoxy) hexahydrocyclopenta[c]pyrrole-2(1H)-carboxylate de (5-tert-butyl-1,3,4-thiadiazol-2-yl)méthyle (exo)

### 3.1 (5-tert-butyle-[1,3,4]thiadiazol-2-yl)-méthanol

1 g (4,67 mmoles) de 5-*tert*-butyl-1,3,4-thiadiazole-2-carboxylate d'éthyle est mis en solution dans 45 mL de méthanol et 0,353 g (9,33 mmoles) de borohydrure de sodium sont ajoutés par portions, sous agitation, à température ambiante. Le milieu est agité 1 heure à température ambiante puis concentré sous vide. Le résidu obtenu est repris dans une solution aqueuse saturée en chlorure de sodium. La solution aqueuse est ramenée à pH 7 par addition lente, sous agitation, d'une solution aqueuse 1N d'acide chlorhydrique. Après 1 heure d'agitation à température ambiante, la phase aqueuse est extraite trois fois au dichlorométhane puis les phases organiques groupées sont lavées une fois avec une solution aqueuse saturée en chlorure de sodium, séchées sur sulfate de sodium, filtrées et concentrées à sec. On obtient 0,802 g (100%) du produit attendu sous la forme d'une huile.
LC-MS : M+H = 173
RMN-¹H (CDCl₃) δ (ppm): 5,10 (d, 2H) ; 4,60 (t, 1H) ; 1,65 (s, 9H).

### 3.2. (3aR,5s,6aS)-5-(4-chlorophénoxy) hexahydrocyclopenta[c]pyrrole-2(1H)-carboxylate de (5-tert-butyl-1,3,4-thiadiazol-2-yl)méthyle

On procède suivant le mode opératoire décrit dans l'exemple 2, étape 2.2.. A partir de 0,10 g (0,42 mmole) de (3a*R*,5*s*,6a*S*)-5-(4-chlorophénoxy)octahydrocyclopenta[*c*]pyrrole, obtenu à l'étape 2.1., de 0,07 g (0,46 mmole) de (5-*tert*-butyle-[1,3,4]thiadiazol-2-yl)-méthanol, obtenu à l'étape 3.1., de 0,08 g (0,42 mmole) de chloroformiate de *para*-nitrophényle et de 0,15 mL (0,88 mmole) de N,N-diisopropyléthylamine, et après chromatographie sur plaques préparatives de gel de silice en éluant avec un mélange 98/2/0,2 de dichlorométhane, de méthanol et d'ammoniaque 30%, on obtient 0,08 g (54%) de produit attendu sous la forme d'une cire.
LC-MS : M+H = 436
RMN-¹H (DMSO) δ (ppm) : 7,30 (d, 2H); 6,90 (d, 2H); 5,4 (s, 2H) ; 4,95 (m, 1H) ; 3,55 (m, 2H) ; 3,25 (m, 2H) ; 2,85 (m, 2H) ; 2,00 (m, 2H); 1,90 (m, 2H); 1,45 (s, 9H).

### Exemple 4 (Composé N°16)

### (3aR,5s,6aS)-5-[3-(trifluorométhyl)phénoxy] hexahydrocyclopenta[c]pyrrole-2(1H)-carboxylate de (3-carbamoylisoxazol-5-yl)méthyle (exo)

### 4.1. (3aR,5s,6aS)-5-[3-(trifluorométhyl) phénoxy]octahydrocyclopenta[c]pyrrole

On procède suivant le mode opératoire décrit dans l'exemple 2, étape 2.1. A partir de 1,4 g (6,16 mmoles) de (3a*R*,5*r*,6a*S*)-5-hydroxyhexahydrocyclopenta[*c*]pyrrole-2(1*H*)-carboxylate de *tert*-butyle (voir synthèse: WO2006108059), de 1,39 g (8,62 mmoles) de 3-trifluorométhyl-phénol, de 1,39 g (8,01 mmoles) de diéthylazodicarboxylate, de 2,26 g (8,62 mmoles) de triphénylphosphine et de 30 mL d'une solution 4N d'acide chlorhydrique dans le dioxane, on obtient 0,48 g (29%) de produit attendu sous la forme d'une cire.
LC-MS : M+H = 272
RMN-¹H (CDCl₃) δ (ppm) : 7,45 (t, 1H) ; 7,30-7,05 (m, 3H) ; 4,95 (qt, 1H) ; 3,05-2,70 (m, 6H) ; 2,25 (m, 2H) ; 1,65 (m, 2H).

### 4.2. 4-Nitro-phényl-carbonate de 3-carbamoyl-isoxazol-5-ylméthyle

A une solution de 2,0 g (14,07 mmoles) de 3-carbamoyl-isoxazol-5-ylméthanol, de 1,71 ml (21,11 mmoles) de pyridine et de 0,17 g (1,41 mmoles) de *N,N*-diméthylaminopyridine dans 15 ml de dichlorométhane, refroidie à environ 0°C, sont ajoutés par petites portions 2,84 g (14,07 mmoles) de chloroformiate de 4-nitrophényle. Le milieu est maintenu sous agitation à 0°C pendant 1 heure puis à température ambiante pendant 1 heure.
Le précipité formé est filtré puis rincé abondamment avec le diisopropyléther. Après séchage sous vide à environ 60°C, on obtient 3,12 g (72%) de produit attendu sous la forme d'un solide blanc utilisé tel quel dans l'étape suivante.
PF(°C) : 143-145°C
RMN ¹H (DMSO) δ (ppm) : 8,40(d, 2H) ; 8,25 (large s, 1H) ; 7,90 (large s, 1H) ; 7,65 (d, 2H) ; 7,0 (s, 1H) ; 5,50 (s, 2H).

### 4.3. (3aR,5s,6aS)-5-[3-(trifluorométhyl) phénoxy]hexahydrocyclopenta[c]pyrrole-2(1H)-carboxylate de (3-carbamoylisoxazol-5-yl)méthyle

On procède suivant le mode opératoire décrit dans l'exemple 1, étape 1.3.. A partir de 0,15 g de (3a*R*,5*s*,6a*S*)-5-[3-(trifluorométhyl) phénoxy]octahydrocyclopenta[*c*]pyrrole, obtenu à l'étape 4.1., de 0,18 g (0,61 mmole) de 4-nitro-phényl-carbonate de 3-carbamoyl-isoxazol-5-ylméthyle, obtenu à l'étape 4.2., de 0,03 g (0,28 mmole) de *N,N-*diméthylaminopyridine, de 0,21 mL (1,22 mmoles) de *N,N-*diisopropyléthylamine, et après chromatographie sur gel de silice en éluant avec un mélange de 99/1/0,1 à 97/3/0,3 de dichlorométhane, de méthanol et d'ammoniaque 30%, on obtient 0,21 g (87%) de produit attendu sous la forme d'une poudre blanche.
Point de fusion (°C) : 130-132°C
LC-MS : M+H = 440
RMN-¹H (DMSO) δ (ppm) : 8,15 (s, 1H) ; 7,85 (s, 1H); 7,55 (t, 1H) ; 7,25 (d, 1H) ; 7,20 (d, 1H) ; 7,15 (s, 1H) ; 6,80 (s, 1H) ; 5,25 (s, 2H) ; 5,10 (m, 1H) ; 3,55 (m, 2H) ; 3,20 (m, 2H) ; 2,85 (m, 2H) ; 2,05-1,95 (m, 2H); 1,95-1,80 (m, 2H).

### Exemple 5 (Composé N°22)

### (3aR,5s,6aS)-5-[(7-éthoxynaphthalèn-2-yl)oxy]hexahydrocyclopenta[c]pyrrole-2(1H)-carboxylate de (4-carbamoyl-thiazol-2-yl)méthyle (exo)

### 5.1. (3aR,5s,6aS)-5-[(7-éthoxynaphthalèn-2-yl)oxy]octahydrocyclopenta[c]pyrrole

On procède suivant le mode opératoire décrit dans l'exemple 2, étape 2.1. A partir de 1,4 g (6,16 mmoles) de (3a*R*,5*r*,6a*S*)-5-hydroxyhexahydrocyclopenta[*c*]pyrrole-2(1*H*)-carboxylate de *tert*-butyle (voir synthèse: WO2006108059), de 1,51 g (8,00 mmoles) de 7-éthoxy-naphthalèn-2-ol, de 1,28 g (7,39 mmoles) de diéthylazodicarboxylate et de 1,93 g (7,39 mmoles) de triphénylphosphine, et de 30 mL d'une solution 4N d'acide chlorhydrique dans le dioxane, on obtient 1,36 g (74%) de produit attendu sous la forme d'une huile.
LC-MS : M+H = 298
RMN ¹H (DMSO) δ (ppm) : 7,70 (d, 2H) ; 7,20 (m, 2H), 7,00 (m, 2H), 5,05 (m, 1H) ; 4,15 (qd, 2H) ; 2,95 (m, 2H) ; 2,85-2,50 (m, 4H) ; 2,10 (m, 2H) ; 1,85 (m, 2H) ; 1,40 (t, 3H).

### 5.2. 2-Hydroxyméthyl-thiazole-4-carboxylate de méthyle

### 5.2.1. 2-[(acétyloxy)méthyl]-thiazole-4-carboxylate d'éthyle

2,7 g (10,80 mmoles) de 2-(bromométhyl) thiazole-4-carboxylate d'éthyle sont mis en solution dans 108 mL d'acétonitrile. 2,225 g (22,67 mmoles) d'acétate de potassium sont ajoutés et le milieu est agité à température ambiante pendant 14 heures.
On concentre sous pression réduite. Le résidu obtenu est repris dans une solution aqueuse de chlorure de sodium et extrait deux fois au dichlorométhane. Les phases organiques groupées sont séchées sur sulfate de sodium, filtrées et concentrées à sec. On obtient 2,347 g (95%) de produit attendu sous la forme d'une cire.
RMN-¹H (CDCl₃) δ (ppm) : 8,15 (s, 1H) ; 5,35 (s, 2H) ; 4,35 (qd, 2 H) ; 2,10 (s, 3H) ; 1,35 (t, 3H).

### 5.2.2. 2-Hydroxyméthyl-thiazole-4-carboxylate de méthyle

2,347 g (10,24 mmoles) de 2-acétoxyméthyl-thiazole-4-carboxylate d'éthyle, obtenu à l'étape 5.2.1., sont mis en solution dans 100 mL d'un mélange 5/1 de dichlorométhane et de méthanol. 2,58 mL (11,26 mmoles) d'une solution 4,37N de méthanolate de sodium dans le méthanol sont additionnés et le milieu est agité à température ambiante pendant deux heures avant d'être concentré sous pression réduite. Le résidu obtenu est repris par une solution aqueuse saturée de chlorure de sodium et extrait trois fois au dichlorométhane. Les phases organiques groupées sont lavées une fois avec une solution aqueuse saturée en chlorure de sodium, séchées sur sulfate de sodium, filtrées et concentrées à sec. Le résidu obtenu est purifié par chromatographie sur gel de silice en éluant avec un mélange 98/2/0,2 de dichlorométhane, de méthanol et d'ammoniaque 30%. On obtient 0,92 g de produit attendu sous la forme d'une poudre blanche.
Point de fusion (°C) : 158-160°C
RMN-¹H (CDCl₃) δ (ppm) : 8,10 (s, 1H) ; 4,95 (s, 2H); 3,90 (s, 3H); 2,50 (large s, 1H).

### 5.3. (3aR,5s,6aS)-5-[(7-éthoxynaphthalèn-2-yl)oxy]hexahydrocyclopenta[c]pyrrole-2(1H)-carboxylate de [4-(méthoxycarbonyl)-thiazol-2-yl]méthyle

On procède suivant le mode opératoire décrit dans l'exemple 2, étape 2.2.. A partir de 0,25 g (0,84 mmole) de (3a*R*,5*s*,6a*S*)-5-[(7-éthoxynaphthalèn-2-yl)oxy]octahydrocyclopenta [*c*]pyrrole, obtenu à l'étape 5.1., de 0,18 g (1,09 mmoles) de 2-hydroxyméthyl-thiazole-4-carboxylate de méthyle, obtenu à l'étape 5.2., de 0,20 g (1,01 mmoles) de *para*-nitrophényle chloroformiate et de 0,37 mL (2,10 mmoles) de *N,N*-diisopropyléthylamine, et après chromatographie sur gel de silice en éluant avec un mélange 99/1/0,1 de dichlorométhane, de méthanol et d'ammoniaque, on obtient 0,25 g de produit attendu sous la forme d'une cire.

### 5.4. (3aR,5s,6aS)-5-[(7-éthoxynaphthalèn-2-yl)oxy]hexahydrocyclopenta[c]pyrrole-2(1H)-carboxylate de (4-carbamoyl-thiazol-2-yl)méthyle

Dans un tube scellé, 0,125 g (0,25 mmole) de (3a*R*,5*s*,6a*S*)-5-[(7-éthoxynaphthalèn-2-yl)oxy]hexahydrocyclopenta[*c*]pyrrole-2(1*H*)-carboxylate de [4-(méthoxycarbonyl)-thiazol-2-yl]méthyle, obtenu à l'étape 5.3., sont mis en solution dans 5 mL de méthanol. 10 mL (70 mmoles) d'une solution 7N d'ammoniaque dans le méthanol sont ajoutés et le milieu, tube scellé, est chauffé à 60°C pour 14 heures d'agitation.
Le milieu revenu à température ambiante est concentré sous vide et le résidu obtenu est chromatographié sur plaques préparatives de gel de silice en éluant avec un mélange 95/5/0,5 de dichlorométhane, de méthanol et d'ammoniaque 30%. On obtient ainsi 0,072 g (59%) du produit attendu sous la forme d'une poudre blanche.
Point de fusion (°C) : 143-145°C
LC-MS : M+H = 482
RMN-¹H (DMSO) δ (ppm) : 8,30 (s, 1H); 7,75 (d, 2H); 7,75 (s, 1H) ; 7,60 (s, 1H) ; 7,20 (m, 2H) ; 6,95 (m, 2H) ; 5,40 (s, 2H) ; 5,10 (m, 1H); 4,15 (qd, 2H); 3,60 (m, 2H); 3,25 (m, 2H); 2,90 (m, 2H); 2,15-2,05 (m, 2H); 2,05-1,90 (m, 2H); 1,40 (t, 3H).

### Exemple 6 (Composé N°25)

### (3aR,5s,6aS)-5-[(7-éthoxynaphthalèn-2-yl)oxy]hexahydrocyclopenta[c]pyrrole-2(1H)-carboxylate de [3-(méthylcarbamoyl)isoxazol-5-yl]méthyle (exo)

### 6.1. 4-Nitro-phényl-carbonate de 3-méthylcarbamoyl-isoxazol-5-ylméthyle

On procède suivant le mode opératoire décrit dans l'exemple 4 (étape 4.2.). A partir de 2,00 g (12,81 mmoles) de 3-méthylcarbamoyl-isoxazol-5-ylméthanol, de 2,58 g (12,81 mmoles) de chloroformiate de 4-nitrophényle, de 1,52 g (19,21 mmoles) de pyridine et de 0,157 g (1,28 mmoles) de *N,N-*diméthylaminopyridine, on obtient 2,6 g (63%) de produit pur sous forme de poudre blanche.
PF(°C) : 166-168°C
RMN ¹H (CDCl₃) δ (ppm) : 8,40(d, 2H) ; 7,50 (d, 2H) ; 7,0 (s, 1H) ; 6,90 (large s, 1H) ; 5,50 (s, 2H) ; 3,10 (d, 3H).

### 6.2. (3aR,5s,6aS)-5-[(7-éthoxynaphthalèn-2-yl)oxy]hexahydrocyclopenta[c]pyrrole-2(1H)-carboxylate de [3-(méthylcarbamoyl)isoxazol-5-yl]méthyle

On procède suivant le mode opératoire décrit dans l'exemple 1, étape 1.3. A partir de 0,15 g (0,50 mmole) de (3a*R*,5*s*,6a*S*)-5-[(7-éthoxynaphthalèn-2-yl)oxy]octahydrocyclopenta [*c*]pyrrole, obtenu à l'étape 5.1., de 0,17 g (0,55 mmole) de 4-nitro-phényl-carbonate de 3-méthylcarbamoyl-isoxazol-5-ylméthyle, obtenu à l'étape 6.1., de 0,03 g (0,28 mmole) de *N,N*-diméthylaminopyridine et de 0,19 mL (1,11 mmoles) de *N,N*-diisopropyléthylamine, et après chromatographie sur gel de silice en éluant avec un mélange 98/2/0,2 de dichlorométhane, de méthanol et d'ammoniaque, on obtient 0,13 g (54%) de produit attendu sous la forme d'une poudre blanche.
Point de fusion (°C) : 108-110°C
LC-MS : M+H = 480
RMN-¹H (DMSO) δ (ppm) : 8,69 (s, 1H) ; 7,69 (d, 2H) ; 7,18 (s, 1H); 7,16 (s, 1H) ; 6,95 (m, 2H); 6,81 (s, 1H), 5,25 (s, 2H); 5,06 (m, 1H) ; 4,12 (qd, 2H); 3,56 (m, 2H); 3,23 (m, 2H); 2,85 (m, 2H); 2,77 (d, 3H); 2,06 (m, 2H); 1,94 (m, 2H); 1,38 (t, 3H).

### Exemple 7 (Composé N°26)

### (3aR,5s,6aS)-5-(4-chlorophénoxy) hexahydrocyclopenta[c]pyrrole-2(1H)-carboxylate de [4-(méthylcarbamoyl)-oxazol-2-yl]méthyle (exo)

### 7.1 2-Hydroxyméthyl-oxazole-4-carboxylate de méthyle

### 7.1.1. 2-[(acétyloxy)méthyl]-oxazole-4-carboxylate d'éthyle

9,5 g (10,80 mmoles) de 2 -(bromométhyl)oxazole-4-carboxylate d'éthyle sont mis en solution dans 135 mL d'acétonitrile. 9,96 g (101,47 mmoles) d'acétate de potassium sont ajoutés et le milieu est agité à température ambiante pendant 14 heures.
On concentre sous vide. Le résidu obtenu est repris dans une solution aqueuse de chlorure de sodium et extrait deux fois au dichlorométhane. Les phases organiques groupées sont séchées sur sulfate de sodium, filtrées et concentrées sous pression réduite. On obtient 8,50 g d'un résidu huileux qui est utilisé tel quel dans l'étape suivante.

### 7.1.2. 2-(hydroxyméthyl)-oxazole-4-carboxylate de méthyle

8,50 g (11,16 mmoles) de 2-acétoxyméthyl-oxazole-4-carboxylate d'éthyle, obtenu à l'étape 7.1.1., sont mis en solution dans 280 mL d'un mélange 5/1 de dichlorométhane et de méthanol. 2,55 mL (11,16 mmoles) d'une solution 4,37N de méthanolate de sodium dans le méthanol sont additionnés et le milieu est agité à température ambiante pendant trois heures.
On refroidit à 0°C pour addition de 10 mL d'une solution aqueuse saturée en chlorure d'ammonium avant d'être concentré sous pression réduite. Le résidu obtenu est repris par une solution aqueuse saturée en chlorure de sodium et extrait trois fois au dichlorométhane. Les phases organiques groupées sont lavées une fois avec une solution aqueuse saturée en chlorure de sodium, séchées sur sulfate de sodium, filtrées et concentrées à sec. Le résidu obtenu est purifié par chromatographie sur gel de silice en éluant avec un mélange de 99/1/0,1 à 97/3/0,3 de dichlorométhane, de méthanol et d'ammoniaque 30%. On obtient 1,3 g de produit attendu sous la forme d'un solide brun.
Point de fusion (°C) : 81-82°C
RMN-¹H (CDCl₃) δ (ppm) : 8,25 (s, 1H) ; 4,85 (s, 2H); 4,00 (s, 3H); 3,50 (s, 1H).

### 7.2. (3aR,5s,6aS)-5-(4-chlorophénoxy) hexahydro cyclopenta[c]pyrrole-2(1H)-carboxylate de [4-(méthoxycarbonyl)-oxazol-2-yl]méthyle

On procède suivant le mode opératoire décrit dans l'exemple 2, étape 2.2. A partir de 0,15 g (0,63 mmole) de (3a*R*,5*s*,6a*S*)-5-(4-chlorophénoxy)octahydrocyclopenta[*c*]pyrrole, obtenu à l'étape 2.1., de 0,12 g (0,82 mmole) de 2-hydroxyméthyl-oxazole-4-carboxylate de méthyle, obtenu à l'étape 7.1.2., de 0,15 g (0,76 mmole) de chloroformiate de *para*-nitrophényle et de 0,27 mL (1,58 mmoles) de *N,N*-diisopropyléthylamine, et après chromatographie sur gel de silice en éluant avec un mélange 99/1/0,1 de dichlorométhane, de méthanol et d'ammoniaque, on obtient 0,24 g (90%) de produit attendu sous la forme d'une huile.

### 7.3. (3aR,5s,6aS)-5-(4-chlorophénoxy) hexahydro cyclopenta[c]pyrrole-2(1H)-carboxylate de [4-(méthylcarbamoyl)-oxazol-2-yl]méthyle

On procède suivant le mode opératoire décrit dans l'exemple 5, étape 5.4. A partir de 0,24 g (0,58 mmole) de (3a*R*,5*s*,6a*S*)-5-(4-chlorophénoxy)hexahydrocyclopenta[*c*]pyrrole-2(1*H*)-carboxylate de [4-(méthoxycarbonyl)-oxazol-2-yl]méthyle, obtenu à l'étape 7.2., et de 8,00 mL (64 mmoles) d'une solution 8N de méthylamine dans l'éthanol, et après chromatographie sur plaques préparatives de gel de silice en éluant avec un mélange 95/5/0,5 de dichlorométhane, de méthanol et d'ammoniaque, on obtient 0,11 g (45%) de produit attendu sous la forme d'une poudre blanche.
Point de fusion (°C) : 104-106°C
LC-MS : M+H = 420
RMN-¹H (DMSO) δ (ppm) : 8,60 (s, 1H); 8,25 (s, 1H); 7,30 (d, 2H) ; 6,95 (d, 2H) ; 5,20 (s, 2H) ; 4,95 (m, 1H) ; 3,55 (m, 2H) ; 3,20 (m, 2H); 2,85 (m, 2H); 2,80 (d, 3H); 2,05-1,95 (m, 2H); 1,95-1,80 (m, 2H).

### Exemple 8 (Composé N°31)

### (3aR,5s,6aS)-5-[(4'-fluorobiphényl-4-yl)oxy]hexahydrocyclopenta[c]pyrrole-2(1H)-carboxylate de (3-carbamoylisoxazol-5-yl)méthyle (exo)

### 8.1. chlorhydrate (1 :1) de (3aR,5r,6aS)-octahydro cyclopenta[c]pyrrol-5-ol

3,00 g (13,20 mmoles) de (3a*R*,5*r*,6a*S*)-5-hydroxyhexahydrocyclopenta[*c*]pyrrole-2(1*H*)-carboxylate de *tert*-butyle (voir synthèse: WO2006108059), sont mis en solution dans 150 mL d'un mélange 2/1 de dioxane et de 1,2-dichloroéthane. 30 mL (120 mmoles) d'une solution d'acide chlorhydrique à 4N dans le dioxane sont versés dans le milieu sous agitation. Après 3 heures d'agitation à température ambiante, le milieu est concentré à sec et le produit obtenu sous forme de chlorhydrate est repris dans l'éther diéthylique pour organisation du sel. Après filtration, et séchage sous pression réduite, on obtient 1,745 g (81%) du produit attendu sous la forme d'un solide hygroscopique.
RMN-¹H (DMSO) δ (ppm) : 9,40 (s, 1H) ; 8,75 (s, 1H) ; 5,10 (s, 1H) ; 4,15 (qt, 1H) ; 3,25 (m, 2H) ; 3,10 (m, 2H) ; 2,80 (m, 2H) ; 1,85 (m, 2H); 1,55 (m, 2H).

### 8.2. (3aR,5r,6aS)-5-hydroxyhexahydrocyclopenta [c]pyrrole-2(1H)-carboxylate de [3-(éthoxycarbonyl)isoxazol-5-yl]méthyle

0,627 g (3,67 mmoles) de 5-(hydroxyméthyl)isoxazole-3-carboxylate d'éthyle sont mis en solution dans 10 mL de dichlorométhane, 1,17 mL (6,72 mmoles) de *N,N-*diisopropyléthylamine sont ajoutés et le milieu est refroidi à 0°C pour addition d'une solution de 0,647 g (3,21 mmoles) de chloroformiate de 4-nitro-phényle dans 5 mL de dichlorométhane. Le milieu est agité une heure à température ambiante puis additionné lentement sur une solution, préalablement refroidie à -10°C, de 0,50 g (3,06 mmoles) de chlorhydrate de (3aR,5r,6aS)-octahydrocyclopenta[*c*]pyrrol-5-ol, obtenu à l'étape 8.1., et de 0,59 mL (3,36 mmoles) de *N,N-*diisopropyléthylamine dans 15 mL d'un mélange 2/1 de dichlorométhane et de méthanol. Après trois heures d'agitation à température ambiante, une solution aqueuse de soude à 1N est additionnée et le milieu est extrait trois fois au dichlorométhane. Les phases organiques groupées sont lavées une fois avec une solution aqueuse saturée en chlorure de sodium, séchées sur sulfate de sodium, filtrées et concentrées sous pression réduite. Le résidu obtenu est purifié par chromatographie sur gel de silice en éluant avec un mélange 99/1 puis 98/2 de dichlorométhane et de méthanol. On obtient 0,78 g (79%) du produit attendu sous la forme d'une huile.
LC-MS : M+H = 325
RMN-¹H (DMSO) δ (ppm) : 6,90 (s, 1H) ; 5,25 (s, 2H) ; 4,60 (d, 1H) ; 4,40 (qd, 2H) ; 4,10 (qt, 1H) ; 3,50 (m, 2H); 3,30 (m, 2H); 2,55 (m, 2H); 2,00 (m, 2H); 1,35 (m, 5H).

### 8.3. (3aR,5s,6aS)-5-(4-bromophénoxy) hexahydro cyclopenta[c]pyrrole-2(1H)-carboxylate de [3-(éthoxycarbonyl)isoxazol-5-yl]méthyle

0,76 g (2,34 mmoles) de (3a*R*,5*r*,6a*S*)-5-hydroxyhexahydrocyclopenta[c]pyrrole-2(1*H*)-carboxylate de [3-(éthoxycarbonyl)isoxazol-5-yl]méthyle, obtenu à l'étape 8.2., sont mis en solution dans 24 mL de toluène. 0,737 g (2,81 mmoles) de triphénylphosphine et 0,527 g (3,05 mmoles) de 4-bromo-phénol sont ajoutés, puis le milieu est refroidi à 0°C pour addition lente d'une solution de 0,49 g (2,81 mmoles) de diéthylazodicarboxylate dans 3 mL de toluène. Le milieu est agité 14 heures à température ambiante puis concentré sous vide. Le résidu obtenu est repris dans une solution aqueuse de soude à 1N et extrait deux fois au dichlorométhane. Les phases organiques groupées sont lavées une fois avec une solution aqueuse saturée en chlorure de sodium, séchées sur sulfate de sodium, filtrées et concentrées sous vide. Le résidu obtenu est purifié par chromatographie sur gel de silice en éluant avec du dichlorométhane puis avec un mélange 99/1 de dichlorométhane et de méthanol. On obtient 0,87 g (61%) de produit attendu sous la forme d'une cire.
LC-MS : M+H = 479
RMN-¹H (DMSO) δ (ppm) : 7,45 (d, 2H) ; 6,95 (s, 1H) ; 6,90 (d, 2H); 5,25 (s, 2H); 4,95 (qt, 1H); 4,35 (qd, 2H); 3,55 (m, 2H); 3,25 (m, 2H); 2,85 (m, 2H); 1,95 (m, 2H); 1,85 (m, 2H) ; 1,35 (t, 3H).

### 8.4. (3aR,5s,6aS)-5-(4-bromophénoxy) hexahydro cyclopenta[c]pyrrole-2(1H)-carboxylate de (3-carbamoylisoxazol-5-yl)méthyle

On procède suivant le mode opératoire décrit dans l'exemple 5, étape 5.4.. A partir de 0,350 g (0,73 mmole) de (3aR,5s,6aS)-5-(4-bromophénoxy)hexahydrocyclopenta[*c*]pyrrole-2(1*H*)-carboxylate de [3-(éthoxycarbonyl)isoxazol-5-yl]méthyle, obtenu à l'étape 8.3., dans 5 mL de méthanol et de 5 mL (35 mmoles) d'une solution d'ammoniaque à 7N dans le méthanol, et après chromatographie sur gel de silice en éluant avec un mélange de 99/1/0,1 à 98/2/0,2 de dichlorométhane, de méthanol et d'ammoniaque, on obtient 0,072 g (59%) du produit attendu sous la forme d'une poudre blanche.
Point de fusion (°C) : 132-134°C
LC-MS : M+H = 450
RMN-¹H (DMSO) δ (ppm) : 8,15 (s, 1H) ; 7,85 (s, 1H) ; 7,45 (d, 2H) ; 6,90 (d, 2H) ; 6,80 (s, 1H) ; 5,25 (s, 2H) ; 4,95 (qt, 1H) ; 3,55 (m, 2H) ; 3,20 (m, 2H) ; 2,85 (m, 2H) ; 2,22 (m, 2H) ; 1,85 (m, 2H).

### 8.5. (3aR,5s,6aS)-5-[(4'-fluorobiphényl-4-yl) oxy]hexahydrocyclopenta[c]pyrrole-2(1H)-carboxylate de (3-carbamoylisoxazol-5-yl)méthyle

Sous atmosphère inerte, 0,20 g (0,44 mmole) de (3aR,5s,6aS)-5-(4-bromophénoxy)hexahydro cyclopenta[*c*]pyrrole-2(1*H*)-carboxylate de (3-carbamoylisoxazol-5-yl)méthyle, obtenu à l'étape 8.4., 0,074 g (0,53 mmole) d'acide 4-fluoro-phénylboronique, 0,434 g (1,33 mmole) de carbonate de césium sont introduits dans 11 ml d'un mélange 10/1 de tétrahydrofurane et d'eau. 0,036 g (0,04 mmole) de PdCl₂dppf.CH₂Cl₂ sont ajoutés puis le milieu est chauffé à environ 75°C pendant 15 heures.
Après retour à température ambiante, le milieu est repris avec du dichlorométhane et une solution aqueuse saturée de carbonate de sodium. La phase aqueuse est extraite deux fois avec du dichlorométhane puis les phases organiques réunies sont lavées avec une solution aqueuse saturée en chlorure de sodium, séchées sur sulfate de sodium, filtrées et concentrées sous pression réduite. Le résidu obtenu est purifié par chromatographie sur gel de silice en éluant avec du dichlorométhane puis avec un mélange 98/2/0,2 de dichlorométhane, de méthanol et d'ammoniaque 30%. On obtient 0,14 g (72%) du produit attendu sous la forme d'une poudre blanche. Point de Fusion(°C) : 200-201°C
LC-MS : MH- = 464
RMN-¹H (DMSO) δ (ppm) : δ (ppm) : 8,15 (s, 1H); 7,85 (s, 1H); 7,65 (t, 2H); 7,55 (d, 2H); 7,25 (t, 2H); 7,00 (d, 2H); 6,80 (s, 1H); 5,25 (s, 2H); 5,00 (m, 1H) ; 3,55 (m, 2H) ; 3,25 (m, 2H); 2,85 (m, 2H); 2,05 (m, 2H); 1,90 (m, 2H).

### Exemple 9 (Composé N°27)

### (3aR,5s,6aS)-5-[(4-chloronaphthalèn-1-yl)oxy]hexahydrocyclopenta[c]pyrrole-2(1H)-carboxylate de (3-carbamoylisoxazol-5-yl)méthyle (exo)

### 9.1. (3aR,5s,6aS)-5-[(4-chloronaphthalèn-1-yl) oxy]hexahydrocyclopenta[c]pyrrole-2(1H)-carboxylate de [3-(éthoxycarbonyl)isoxazol-5-yl]méthyle

On procède suivant le mode opératoire décrit dans l'exemple 8, étape 8.3.. A partir de 0,60 g (1,85 mmoles) de (3a*R*,5*r*,6a*S*)-5-hydroxyhexahydrocyclopenta[*c*]pyrrole-2(1*H*)-carboxylate de [3-(éthoxycarbonyl)isoxazol-5-yl]méthyle, obtenu à l'étape 8.2., de 0,39 g (2,22 mmoles) de 4-chloronaphthalèn-1-ol, de 0,35 g (2,03 mmoles) de diéthylazodicarboxylate et de 0,58 g (2,22 mmoles) de triphénylphosphine, et après chromatographie sur gel de silice en éluant avec du dichlorométhane puis avec un mélange 99/1 de dichlorométhane et de méthanol, on obtient 0.94 g (54%) de produit attendu sous la forme d'une huile.
LC-MS : M+ (NH4+) = 502
RMN-¹H (DMSO) δ (ppm) : 8,25 (d, 1H) ; 8,10 (d, 1H) ; 7,70 (t, 1H) ; 7,65 (t, 1H) ; 7,60 (d, 1H) ; 6,95 (d, 1H) ; 6,95 (s, 1H) ; 5,30 (s, 2H) ; 5,20 (qt, 1H) ; 4,40 (qd, 2H) ; 3,55 (m, 2H) ; 3,25 (m, 2H) ; 2,90 (m, 2H) ; 2,15 (m, 2H) ; 1,95 (m, 2H) ; 1,35 (t, 3H).

### 9.2. (3aR,5s,6aS)-5-[(4-chloronaphthalèn-1-yl) oxy]hexahydrocyclopenta[c]pyrrole-2(1H)-carboxylate de (3-carbamoylisoxazol-5-yl)méthyle

On procède suivant le mode opératoire décrit dans l'exemple 8, étape 8.4. A partir de 0,40 g (0,82 mmole) de (3a*R*,5*s*,6a*S*)-5-[(4-chloronaphthalèn-1-yl)oxy]hexahydrocyclopenta [*c*]pyrrole-2(1*H*)-carboxylate de [3-(éthoxycarbonyl)isoxazol-5-yl]méthyle, obtenu à l'étape 9.1., de et 10 mL (70 mmoles) d'une solution d'ammoniaque à 7M dans le méthanol, et après chromatographie sur gel de silice en éluant avec un mélange de 99/1/0,1 à 98/2/0,2 de dichlorométhane, de méthanol et d'ammoniaque, on obtient 0,23 g (61%) du produit attendu sous la forme d'un solide.
PF (°C) : 185-187
LC-MS : M+H = 456
RMN-¹H (DMSO) δ (ppm) : 8,25 (d, 1H) ; 8,15 (d, 1H); 8,15 (s, 1H); 7,85 (s, 1H); 7,70 (t, 1H); 7,65 (t, 1H); 7,60 (d, 1H); 6,95 (d, 1H) ; 6,80 (s, 1H), 5,25 (s, 2H) ; 5,20 (m, 1H) ; 3,60 (m, 2H); 3,25 (m, 2H); 2,95 (m, 2H); 2,15 (m, 2H); 1,95 (m, 2H).

### Exemple 10 (Composé N°28)

### (3aR,5s,6aS)-5-[(4-chloronaphthalèn-1-yl)oxy]hexahydrocyclopenta[c]pyrrole-2(1H)-carboxylate de [3-(méthylcarbamoyl)isoxazol-5-yl]méthyle (exo)

On procède suivant le mode opératoire décrit dans l'exemple 8, étape 8.4.. A partir de 0,40 g (0,82 mmole) de (3a*R*,5*s*,6a*S*)-5-[(4-chloronaphthalèn-1-yl)oxy]hexahydrocyclopenta [*c*]pyrrole-2(1*H*)-carboxylate de [3-(éthoxycarbonyl)isoxazol-5-yl]méthyle, obtenu à l'étape 9.1., et de 10 mL (80 mmoles) d'une solution de méthylamine à 8M dans l'éthanol, et après chromatographie sur gel de silice en éluant avec un mélange 99/1/0,1 de dichlorométhane de méthanol et d'ammoniaque 30%, on obtient 0,16 g (55%) du produit attendu sous la forme d'une poudre blanche.
PF(°C) : 131-133°C
LC-MS : M+H = 470
RMN-¹H (DMSO) δ (ppm) : 8,70 (s, 1H); 8,25 (d, 1H); 8,15 (d, 1H) ; 7,75 (t, 1H) ; 7,65 (t, 1H) ; 7,60 (d, 1H) ; 6,95 (d, 1H) ; 6,82 (s, 1H), 5,25 (s, 2H) ; 5,20 (m, 1H) ; 3,55 (m, 2H) ; 3,25 (m, 2H); 2,95 (m, 2H); 2,80 (d, 3H); 2,15 (m, 2H); 1,95 (m, 2H).

### Exemple 11 (Composé N°18)

### (3aR,5s,6aS)-5-[(4'-éthoxybiphényl-3-yl)oxy]hexahydrocyclopenta[c]pyrrole-2(1H)-carboxylate de thiazol-2-ylméthyle (exo)

### 11.1. (3aR,5s,6aS)-5-(3-bromophénoxy) hexahydrocyclopenta[c]pyrrole-2(1H)-carboxylate de tert-butyle

0,20 g (0,88 mmole) de (3a*R*,5*r*,6a*S*)-5-hydroxyhexahydrocyclopenta[*c*]pyrrole-2(1*H*)-carboxylate de *tert*-butyle (WO2006108059), 0,19 g (1,10 mmoles) de 3-bromo-phénol, 0,21 g (1,06 mmole) de diisopropylazodicarboxylate et 0,34 g (1,09 mmole) de triphénylphosphine supporté sur résine (triphenylphosphine, polymer-supported, 3,2 mmol/g on polystyrene), sont dissous dans 3,5 mL de toluène. Le mélange est agité à température ambiante pendant 15 heures. Après filtration de la résine, de l'acétate d'éthyle est ajoutée puis les phases organiques sont lavées avec une solution aqueuse 1N de soude. Les phases organiques sont séchées sur sulfate de sodium, filtrées et concentrées sous pression réduite. Le résidu obtenu est purifié par chromatographie sur gel de silice en éluant avec un mélange 95/5 puis 90/10 de cyclohexane et d'acétate d'éthyle. On obtient ainsi 0,14 g (42%) de produit attendu sous la forme d'une huile.

### 11.2. (3aR,5s,6aS)-5-[(4'-éthoxybiphényl-3-yl)oxy]hexahydrocyclopenta[c]pyrrole-2(1H)-carboxylate de tert-butyle

Sous atmosphère inerte, sont introduits 0,14 g (0,37 mmole) de (3a*R*,5*s*,6a*S*)-5-(3-bromophénoxy)hexahydrocyclopenta[*c*]pyrrole-2(1*H*)-carboxylate de *tert*-butyle, obtenu à l'étape 11.1., 0,08 g (0,51 mmole) d'acide 4-éthoxy-phénylboronique et 0,04 g (1,04 mmole) de chlorure de lithium dans 3,6 ml d'un mélange 1/1/0,4 d'éthanol, de toluène et d'eau. 0,46 mL (0,92 mmole) d'une solution aqueuse 2M de carbonate de sodium et 0,02 g (0,02 mmole) de Pd(PPh₃)₄ sont ajoutés au milieu. Après 15 heures de chauffage à 75°C, le milieu est laissé revenir à température ambiante, puis repris avec de l'acétate d'éthyle et de l'eau. La phase aqueuse est extraite deux fois à l'acétate d'éthyle puis les phases organiques réunies sont lavées avec une solution aqueuse saturée en chlorure de sodium, séchées sur sulfate de sodium, filtrées et concentrées sous pression réduite. Le résidu obtenu est purifié par chromatographie sur gel de silice en éluant avec un mélange 95/5 puis 90/10 de cyclohexane et d'acétate d'éthyle. On obtient ainsi 0,088 g (57%) de produit attendu sous la forme d'une cire.

### 11.3. (3aR,5s,6aS)-5-[(4'-éthoxybiphényl-3-yl)oxy]octahydrocyclopenta[c]pyrrole

On procède suivant le mode opératoire décrit dans l'exemple 1, étape 1.2.. A partir de 0,08 g (0,21 mmole) de (3a*R*,5*s*,6a*S*)-5-[(4'-ethoxybiphenyl-3-yl)oxy]hexahydrocyclopenta [*c*]pyrrole-2(1*H*)-carboxylate de *tert*-butyle, obtenu à l'étape 11.2., et de 1,04 mL d'une solution d'acide chlorhydrique à 4N dans le dioxane, on obtient 0,06 g (96%) de produit attendu sous la forme d'une huile.

### 11.4. (3aR,5s,6aS)-5-[(4'-éthoxybiphényl-3-yl) oxy]hexahydrocyclopenta[c]pyrrole-2(1H)-carboxylate de thiazol-2-ylméthyle

On procède suivant le mode opératoire décrit dans l'exemple 1, étape 1.3.. A partir de 0,06 g (0,20 mmole) de (3a*R*,5*s*,6a*S*)-5-[(4'-éthoxybiphényl-3-yl)oxy]octahydrocyclopenta [*c*]pyrrole, obtenu à l'étape 11.3., de 0,04 mL (0,22 mmole) de *N,N-*diisopropyléthylamine et de 0,05 g (0,18 mmole) de (4-nitro-phényle)- carbonate de thiazole-4-ylméthyle (WO2008013834), et après chromatographie sur gel de silice en éluant avec un mélange 95/5 puis 90/10 de cyclohexane et d'acétate d'éthyle, on obtient 0,05 g (69%) de produit attendu sous la forme d'une huile.
LC-MS : M+H = 465
RMN-¹H (DMSO) δ (ppm) : 7,80 (d,1H) ; 7,75 (d, 1H) ; 7,60 (d, 2H) ; 7,35 (t, 1H) ; 7,15 (d, 1H) ; 7,05 (s, 1H) ; 7,00 (d, 2H) ; 6,85 (d, 1H) ; 5,35 (s, 2H) ; 5,10 (m, 1H) ; 4,10 (q, 2H) ; 3,55 (m, 2H) ; 3,30 (m, 2H) ; 2,90 (m, 2H) ; 2,10 (m, 2H) ; 1,90 (m, 2H) ; 1,40 (t, 3H)

### Exemple 12 (Composé N°1)

### (3aR,5s,6aS)-5-(4-chlorophénoxy) hexahydro cyclopenta[clpyrrole-2(1H)-carboxylate de thiazol-2-ylméthyle (exo)

### 12.1. (3aR,5r,6aS)-5-hydroxyhexahydro cyclopenta[c]pyrrole-2(1H)-carboxylate de thiazol-2-ylméthyle

On procède suivant le mode opératoire décrit dans l'exemple 1, étape 1.3.. A partir de 1,02 g (6,23 mmoles) de chlorhydrate de (3a*R*,5*r*,6a*S*)-octahydrocyclopenta[*c*]pyrrol-5-ol, obtenu suivant l'étape 8.1. de l'exemple 8, de 2,09 g (7,85 mmoles) de (4-nitro-phényle)- carbonate de thiazole-4-ylméthyle (WO2008013834) et de 3,25 mL (18,66 mmoles) de *N,N-*diisopropyléthylamine, et après chromatographie sur gel de silice en éluant avec un mélange 98/2/0,2 puis 97/3/0.3 de dichlorométhane, de méthanol et d'ammoniaque à 30%, on obtient 0,34 g (20%) de produit attendu sous la forme d'une cire.

### 12.2. (3aR,5s,6aS)-5-(4-chlorophénoxy)hexahydro cyclopenta[c]pyrrole-2(1H)-carboxylate de thiazol-2-ylméthyle

On procède suivant le mode opératoire décrit dans l'exemple 11, étape 11.1.. A partir de 0,04 g (0,16 mmole) de (3a*R*,5*r*,6a*S*)-5-hydroxyhexahydrocyclopenta[*c*]pyrrole-2(1*H*)-carboxylate de 1,3-thiazol-2-ylméthyle, obtenu à l'étape 12.1., de 0,02 g (0,19 mmole) de 4-chloro-phénol, de 0,04 g (0,20 mmole) de diisopropylazodicarboxylate et de 0,08 g (0,25 mmole) de triphénylphosphine supporté sur résine (triphenylphosphine, polymer-supported, 3,2 mmol/g on polystyrene), et après chromatographie sur gel de silice en éluant avec un mélange 95/5 puis 90/10 de cyclohexane et d'acétate d'éthyle, on obtient 0,025 g (41%) de produit attendu sous la forme d'un solide.
PF(°C) : 75-77 °C
LC-MS . M+H = 379
RMN-¹H (DMSO) δ (ppm) : 7,8 (d, 1H) ; 7,75 (d, 1H) ; 7,35 (d, 2H) ; 6,90 (d, 2H) ; 5,35 (s, 2H) ; 4,95 (m, 1H) ; 3,55 (m, 2H) ; 3,25 (large m, 2H) ; 2,85 (large m , 2H) ; 2,0 (m, 2H) ; 1,85 (m, 2H).

### Exemple 13 (Composé N°29)

### (3aR,4S,6aS)-4-[(4-chloronaphthalèn-1-yl)oxy]hexahydrocyclopenta[c]pyrrole-2(1H)-carboxylate de thiazol-4-ylméthyle (exo)

### 13.1. (3aR,4R,6aS)-4-hydroxyhexahydro cyclopenta[c]pyrrole-2(1H)-carboxylate de tert-butyle

Sous atmosphère inerte, 1,00 g (4.44 mmoles) de (3a*R*,6a*S*)-4-oxohexahydrocyclopenta[*c*]pyrrole-2(1*H*)-carboxylate de *tert-*butyle est mis en solution dans 15 mL de tétrahydrofurane anhydre ; le milieu est refroidi à -78°C puis 6,66 mL (6,66 mmoles) d'une solution de tri-sec-borohydrure de lithium (L-selectride) à 1N dans le tétrahydrofurane, sont ajoutés goutte à goutte. Le milieu est laissé revenir à température ambiante pour trois heures d'agitation avant d'être refroidi à 0°C pour addition goutte à goutte d'eau oxygénée à 35% jusqu'à cessation du dégagement gazeux. Le milieux est dilué dans l'eau et extrait trois fois à l'acétate d'éthyle. Les phases organiques groupées sont lavées une fois avec une solution aqueuse saturée en chlorure de sodium, séchées sur sulfate de sodium, filtrées et concentrées sous pression réduite. On obtient 0,826 g (82%) du produit attendu sous la forme d'une huile incolore.

### 13.2. (3aR,4S,6aS)-4-[(4-chloronaphthalèn-1-yl)oxy]hexahydrocyclopenta[c]pyrrole-2(1H)-carboxylate de tert-butyle

On procède suivant le mode opératoire décrit dans l'exemple 11, étape 11.1.. A partir de 0,15 g (0,66 mmole) de (3a*R*,4*R*,6a*S*)-4-hydroxyhexahydro cyclopenta[*c*]pyrrole-2(1*H*)-carboxylate de *tert*-butyle, obtenu à l'étape 13.1., de 0,130 g (0,73 mmole) de 4-chloronaphthalèn-l-ol, de 0,160 g (0,79 mmole) de diisopropylazodicarboxylate et de 0,206 g (0,659 mmole) de triphénylphosphine supporté sur résine (triphenylphosphine, polymer-supported, 3,2 mmol/g on polystyrene), et après chromatographie sur gel de silice en éluant avec un mélange 95/5 puis 90/10 de cyclohexane et d'acétate d'éthyle, on obtient 0,077 g (30%) de produit attendu.

### 13.3. (3aR,4S,6aS)-4-[(4-chloronaphthalèn-1-yl)oxy]octahydrocyclopenta[c]pyrrole

On procède suivant le mode opératoire décrit dans l'exemple 1, étape 1.2.. A partir de 0,103 g (0,27 mmole) de (3a*R*,4*S*,6a*S*)-4-[(4-chloronaphthalèn-1-yl)oxy]hexahydro cyclopenta[clpyrrole-2(1*H*)-carboxylate de *tert*-butyle, obtenu à l'étape 13.2., et de 1,33 mL d'une solution d'acide chlorhydrique à 4N dans le dioxane, et après chromatographie sur gel de silice en éluant avec un mélange 97/3/0,3 puis 96/4/0,4 de dichlorométhane, de méthanol et d'ammoniaque à 30%, on obtient 0,068 g (89%) de produit attendu sous la forme d'une huile rouge.

### 13.4. (3aR,4S,6aS)-4-[(4-chloronaphthalèn-1-yl) oxy]hexahydrocyclopenta[c]pyrrole-2(1H)-carboxylate de thiazol-4-ylméthyle

On procède suivant le mode opératoire décrit dans l'exemple 1, étape 1.3.. A partir de 0,059 g (0,21 mmole) de (3a*R*,4*S*,6a*S*)-4-[(4-chloronaphthalèn-1-yl)oxy]octahydro cyclopenta[*c*]pyrrole, obtenu à l'étape 13.3., de 0,04 mL (0,25 mmole) de *N,N*-diisopropyléthylamine et de 0,066 g (0,23 mmole) de (4-nitro-phényle)- carbonate de thiazole-4-ylméthyle (WO2008013834), et après chromatographie sur gel de silice en éluant avec un mélange 95/5 puis 90/10 de cyclohexane et d'acétate d'éthyle, on obtient 0,017 g (19%) de produit attendu sous la forme d'une huile.
LC-MS : M+H = 429
RMN-¹H (DMSO) δ (ppm) : 9,10 (s, 1H) ; 8,25 (d, 1H) ; 8,15 (d, 1H) ; 7,75-7,60 (m, 4H) ; 7,00 (d, 1H) ; 5,20 (s, 2H) ; 4,90 (s, 1H) ; 3,70 (m, 1H); 3,60 (m, 1H); 3,30 (m, 2H) ; 2,90 (m, 2H) ; 2,20 (m, 2H) ; 1,90 (m, 1H) ; 1,55 (m, 1H).

### Exemple 14 (Composé N°30)

### (3aR,4R,6aS)-4-[(4'-éthoxybiphényl-3-yl)oxylhexahydrocyclopenta[c]pyrrole-2(1H)-carboxylate de thiazol-4-ylméthyle (endo)

### 14.1. (3aR,4R,6aS)-4-(3-bromophénoxy)hexahydro cyclopenta[c]pyrrole-2(1H)-carboxylate de tert-butyle

On procède suivant le mode opératoire décrit dans l'exemple 1, étape 1.1.. A partir de 0,150 g (0.66 mmole) de (3a*R*, 4*R*, 6a*S*)-4-hydroxyhexahydrocyclopenta[*c*]pyrrole-2(1*H*)-carboxylate de *tert*-butyle, obtenu à l'étape 13.1., de 0,144 g (0,82 mmole) de 1-bromo-3-fluorobenzène, de 0,024 g (0,99 mmole) d'hydrure de sodium et 3 mL de *N*,*N*-diméthylformamide, et après chromatographie sur gel de silice, on obtient 0,083 g (33%) du produit attendu sous la forme d'une huile incolore.

### 14.2. (3aR,4R,6aS)-4-[(4'-éthoxybiphényl-3-yl)oxylhexahydrocyclopenta[c]pyrrole-2(1H)-carboxylate de tert-butyle

On procède suivant le mode opératoire décrit dans l'exemple 11, étape 11.2.. A partir de 0,123 g (0,324 mmole) de (3a*R*,4*R*,6a*S*)-4-(3-bromophénoxy)hexahydrocyclopenta[*c*]pyrrole-2(1*H*)-carboxylate de *tert*-butyle, obtenu à l'étape 14.1., de 0,075 g (0,45 mmole) d'acide 4-éthoxy-phénylboronique, de 0,039 g (0.91 mmole) de chlorure de lithium, de 0,40 mL (0,80 mmole) d'une solution aqueuse 2N de carbonate de sodium, et de 0,02 g (0,02 mmole) de Pd(PPh₃)₄, et après chromatographie sur gel de silice, on obtient 0,105 g (77%) du produit attendu sous la forme d'une huile incolore.

### 14.3. (3aR,4R,6aS)-4-[(4'-éthoxybiphényl-3-yl)oxy]octahydrocyclopenta[c]pyrrole

On procède suivant le mode opératoire décrit dans l'exemple 1, étape 1.2.. A partir de 0,105 g (0,25 mmol) de (3a*R*,4*R*,6a*S*)-4-[(4'-éthoxybiphényl-3-yl)oxy]hexahydrocyclopenta [*c*]pyrrole-2(1*H*)-carboxylate de *tert*-butyle, obtenu à l'étape 14.2., et de 1,24 mL d'une solution 4N d'acide chlorhydrique dans le dioxane, et après chromatographie sur gel de silice, on obtient 0,068 g (84%) de produit attendu sous la forme d'une huile.

### 14.4. (3aR,4R,6aS)-4-[(4'-éthoxybiphényl-3-yl)oxy]hexahydrocyclopenta[c]pyrrole-2(1H)-carboxylate de thiazol-4-ylméthyle

On procède suivant le mode opératoire décrit dans l'exemple 1, étape 1.3. A partir de 0,053 g (0,19 mmole) de (3a*R*,4*R*,6a*S*)-4-[(4'-éthoxybiphényl-3-yl)oxy]octahydrocyclopenta [c]pyrrole, obtenu à l'étape 14.3., de 0,04 mL (0,23 mmole) de *N,N-*diisopropyléthylamine et de 0,068 g (0,21 mmole) de (4-nitro-phényle)- carbonate de thiazole-4-ylméthyle (WO2008013834), et après chromatographie sur gel de silice en éluant avec un mélange 95/5 puis 90/10 de cyclohexane et d'acétate d'éthyle, on obtient 0,055 g (63%) de produit attendu sous la forme d'une huile.
LC-MS : M+H = 465
RMN-¹H (DMSO) δ (ppm) : 9,10 (s, 1H) ; 7,70 (s, 1H) ; 7,60 (d, 2H) ; 7,35 (t, 1H) ; 7,20 (d, 1H) ; 7,10 (s, 1H) ; 7,00 (d, 2H) ; 6,90 (d, 1H) ; 5,20 (s, 2H) ; 4,90 (q, 1H) ; 4,10 (q, 2H) ; 3, 60 (m, 2H) ; 3,30 (m, 2H) ; 3,05 (m, 1H) ; 2,80 (m, 1H) ; 2,10 (m, 1H) ; 1,85 (m,2H) ; 1,55 (m, 1H) ; 1,35 (t, 3H).

### Exemple 15 (Composé N°40)

### (3aR,5r,6aS)-5-(4-fluoro-1,3-benzothiazol-2-yl)-5-hydroxyhexahydrocyclopenta[c]pyrrole-2(1H)-carboxylate de (3-carbamoylisoxazol-5-yl)méthyle (exo)

### 15.1 4-Fluoro-2-benzothiazole

2,00 g (10,14 mmoles) d'acide 4-fluoro-2 benzothiazolecarboxylique sont mis en solution dans un mélange isovolume de 50 mL de toluène et d'éthanol. 2,508 g (13,19 mmoles) d'acide para-toluène sulfonique monohydraté sont ajoutés. Après 14 heures de chauffage au reflux, le milieu est concentré à sec et le résidu repris dans une solution aqueuse saturée en carbonate de sodium. La phase aqueuse est extraite deux fois puis les phases organiques réunies sont lavées une fois avec une solution aqueuse saturée en chlorure de sodium, séchées sur sulfate de sodium et concentrées sous pression réduite. On obtient 1,5 g (97%) de produit attendu sous la forme d'une huile.

### 15.2 (3aR,5r,6aS)-5-(4-fluoro-1,3-benzothiazol-2-yl)-5-hydroxyhexahydrocyclopenta[c]pyrrole-2(1H)-carboxylate de tert-butyle

Sous atmosphère inerte, 1 g (6,53 mmoles) de 4-fluoro-2-benzothiazole (obtenu à l'étape 15.1) est mis en solution dans 30 mL de tétrahydrofurane. Le milieu est refroidi à -78°C et 4,49 mL (7,18 mmoles) d'une solution à 1,6 M de n-butyle lithium sont additionnés goutte à goutte. Le milieu est laissé revenir à 0°C puis à nouveau refroidi à -78°C pour addition d'une solution de 1,618 g (7,18 mmoles) de (3a*R*,6a*S*)-5-oxohexahydrocyclopenta[*c*]pyrrole-2(1*H*)-carboxylate de *tert-*butyle dans 5 mL de tétrahydrofurane. Le milieu est laissé revenir à température ambiante pour une heure d'agitation puis hydrolysé avec une solution aqueuse saturée en chlorure d'ammonium. Le milieu est extrait trois fois au dichlorométhane puis les phases organiques réunies sont lavées une fois avec une solution aqueuse saturée en chlorure de sodium, séchées sur sulfate de sodium puis concentrées sous vide. Après chromatographie sur gel de silice en éluant avec du dichlorométhane puis avec un mélange 99/1/0,1 de dichlorométhane, de méthanol et d'ammoniaque 30%, et réorganisation du solide obtenu dans l'éther diéthylique, on obtient 1 g (41%) du produit attendu sous la forme d'une poudre blanche. LC-MS : M+H = 379
RMN-¹H (DMSO) δ (ppm) : 7,95 (d, 1H) ; 7,45 (m, 1H); 7,35 (t, 1H) ; 6,45 (s, 1H) ; 3,55 (t, 2H) ; 3,35 (m, 2H) ; 3,00 (m, 2H); 2,45 (m, 2H); 2,00 (d, 2H); 1,45 (s, 9H).

### 15.3 chlorhydrate de (3aR,5r,6aS)-5-(4-fluoro-1,3-benzothiazol-2-yl)octahydrocyclopenta[c]pyrrol-5-ole

0.50 g (1,32 mmole) de (3a*R*,5*r*,6a*S*)-5-(4-fluoro-1,3-benzothiazol-2-yle)-5-hydroxyhexahydrocyclopenta[c]pyrrole-2(1*H*)-carboxylate de *tert*-butyle sont mis en solution dans 30 mL de dichlorométhane. Dans le milieu refroidi à -5°C, 5,00 mL (20,00 mmoles) d'une solution à 4N d'acide chlorhydrique dans le dioxane sont lentement ajoutés sous agitation puis le milieu est laissé revenir à température ambiante pour 14 heures d'agitation. Le milieu est concentré à sec sous pression réduite. Après réorganisation du résidu obtenu dans l'éther diéthylique et filtration, on obtient 0.388 g (93%) du produit attendu sous la forme d'une poudre brune. LC-MS : M+H = 279
RMN-¹H (DMSO) δ (ppm) : 7,95 (d 1H); 7,45 (m, 1H) ; 7,35 (t, 1H) ; 3,45 (t, 2H) ; 3,25 (m, 2H) ; 3,15 (m, 2H) ; 2,45 (m, 2H) ; 2,15 (d, 2H).

### 15.4 (3aR,5r,6aS)-5-(4-fluoro-1,3-benzothiazol-2-yl)-5-hydroxyhexahydrocyclopenta[c]pyrrole-2(1H)-carboxylate de (3-carbamoylisoxazol-5-yl)méthyle

Dans un tube scellé, 0,380 g (1,21 mmoles) de chlorhydrate de (3a*R*,5*r*,6a*S*)-5-(4-fluoro-1,3-benzothiazol-2-yl)octahydrocyclopenta[*c*]pyrrol-5-ole sont mis en suspension dans 6 mL de 1,2-dichloroéthane. 0,408 g (1,33 mmoles) de 4-nitro-phényl-carbonate de 3-carbamoyl-isoxazol-5-ylméthyle, obtenu à l'étape 4.2., 0,074 g (0,60 mmole) de *N,N-*diméthylaminopyridine, 0,63 mL (3,62 mmoles) de *N,N-*diisopropyléthylamine sont ajoutés et le milieu est agité 10 minutes à température ambiante avant d'être porté à 70°C pour 4 heures d'agitation. Le milieu est laissé revenir à température ambiante, dilué avec du dichlorométhane et une solution aqueuse à 1N de soude. La phase aqueuse est extraite deux fois au dichlorométhane puis les phases organiques réunies sont lavées une fois avec une solution aqueuse saturée en chlorure d'ammonium et une fois avec une solution aqueuse saturée en chlorure de sodium, séchées sur sulfate de sodium, filtrées et concentrées sous vide. Après chromatographie sur gel de silice du résidu en éluant avec un mélange 99/1/0,1 puis 98/2/0,2 de dichlorométhane, de méthanol et d'ammoniaque 30%, on obtient 0,392 g (72%) du produit attendu sous la forme d'une poudre blanche.
PF(°C) : 173-174°C
LC-MS : M+H = 447
RMN-¹H (DMSO) δ (ppm) : 8,15 (s, 1H) ; 7, 90 (d, 1H) ; 7,85 (s, 1H); 7,40 (t, 1H) ; 7,35 (t, 1H) ; 6,80 (s, 1H) ; 6,45 (s, 1H) ; 5,25 (s, 2H); 3,65 (m, 2H) ; 3,45 (d, 2H) ; 3,00 (m, 2H) ; 2,50 (m, 2H); 2,00 (d, 2H).

### Exemple 16 (Composé N°42)

### 3aR,5s,6aS)-5-(4-fluorophénoxy)hexahydrocyclopenta[c]pyrrole-2(1H)-carboxylate de (3-{[2-(diméthylamino)éthyl]carbamoyl} isoxazol-5-yl)méthyle (exo)

### 16.1. (3aR,5s,6aS)-5-(4-fluorophénoxy)hexahydrocyclopenta[c]pyrrole-2(1H)-carboxylate de [3-(éthoxycarbonyl)isoxazol-5-yl]méthyle

On procède suivant le mode opératoire décrit dans l'exemple 8, étape 8.3.. A partir de 0,70 g (2,16 mmoles) de (3a*R*,5*r*,6a*S*)-5-hydroxyhexahydrocyclopenta[*c*]pyrrole-2(1*H*)-carboxylate de [3-(éthoxycarbonyl)isoxazol-5-yl]méthyle obtenu à l'étape 8.2., 0,679 g (2,59 mmoles) de triphénylphosphine, 0,290 g (2,59 mmoles) de 4-fluoro-phénol et 0,451 g (2,59 mmoles) de diéthylazodicarboxylate et après chromatographie sur gel de silice en éluant avec du dichlorométhane puis avec un mélange 99/1 de dichlorométhane et de méthanol, on obtient 0,80 g (88.6%) de produit attendu sous la forme d'une cire brune.
LC-MS : M+H = 419
RMN-¹H (CDCl₃) δ (ppm) : 6,90 (t, 2H) ; 6,70 (m, 3H); 5,15 (s, 2H); 4,75 (qt, 1H); 4,35 (qd, 2H); 3,55 (m, 2H); 3,25 (m, 2H); 2,85 (m, 2H); 2,10 (m, 2H); 1,70 (m, 2H) ; 1,35 (t, 3H).

### 16.2. (3aR,5s,6aS)-5-(4-fluorophénoxy)hexahydrocyclopenta[c]pyrrole-2(1H)-carboxylate de (3-{[2-(diméthylamino)éthyl]carbamoyl}isoxazol-5-yl)méthyle (chlorydrate 1/1))

0,40 g (0,96 mmole) de (3a*R*,5*s*,6a*S*)-5-(4-fluorophénoxy) hexahydro cyclopenta[*c*]pyrrole-2(1*H*)-carboxylate de [3-(éthoxycarbonyl)isoxazol-5-yl]méthyle et 0,084 g (0,96 mmole) de N,N-diméthyléthylènediamine sont mis en solution dans 5 mL de méthanol. Le milieu est chauffé 4 heures à 60°C puis laissé revenir à température ambiante pour être concentré à sec. Le résidu est purifié par chromatographie sur gel de silice en éluant avec un mélange 97/3/0,3 puis 95/5/0,5 de dichlorométhane, de méthanol et d'ammoniaque 30%. On obtient 0.267 g (60.7%) du produit attendu sous la forme d'une huile qui est mise en solution dans 10 mL de dichlorométhane. 1 mL d'une solution d'acide chlorhydrique à 4N dans le dioxane est ajouté et le milieu est agité une heure avant d'être concentré sous pression réduite. Après réorganisation du résidu dans l'éther diéthylique, filtration et séchage sous vide, on obtient 0.262 g (90.9%) du chlorhydrate correspondant attendu sous la forme d'une poudre blanche.
Point de fusion (°C) : 146-148°C
LC-MS : M+H = 461
RMN-¹H (DMSO) δ (ppm) : 10,10 (large s, 1H); 9,05 (t, 1H); 7,10 (t, 2H); 6,95 (m, 3H); 5,25 (s, 2H); 4,90 (m, 1H); 3,65 (m, 2H) ; 3,55 (m, 2H) ; 3,25 (m, 2H) ; 3,20 (m, 2H) ; 2,85 (s, 8H) ; 2,00 (m, 2H) ; 1,85 (m, 2H).

### Exemple 17 (Composé N°34)

### (3aR,5s,6aS)-5-(4-chloro-3-fluorophénoxy)hexahydrocyclopenta[c]pyrrole-2(1H)-carboxylate de (3-carbamoylisoxazol-5-yl)méthyle (exo)

### 17.1. (3aR,5s,6aS)-5-(4-Chloro-3-fluoro-phénoxy)-octahydro-cyclopenta[c]pyrrole

On procède suivant le mode opératoire décrit dans l'exemple 2, étape 2.1. A partir de 5,0 g (22,00 mmoles) de (3a*R*,5*r*,6a*S*)-5-hydroxyhexahydrocyclopenta[*c*]pyrrole-2(1*H*)-carboxylate de *tert*-butyle (voir synthèse: WO2006108059), de 3,87 g (26,40 mmoles) de 4-Chloro-3-fluoro-phenol, de 4,41 g (25,30 mmoles) de diéthylazodicarboxylate, de 6,64 g (25,30 mmoles) de triphénylphosphine et de 20 mL d'une solution 4N d'acide chlorhydrique dans le dioxane, on obtient 4,86 g (86,5%) de produit attendu sous la forme d'une cire. LC-MS : M+H = 256 RMN-¹H (CDCl₃) δ (ppm) : 7,25 (t, 1H) ; 6,65 (m, 2H) ; 4,85 (qt, 1H) ; 3,05-2,70 (m, 6H) ; 2,20 (m, 2H) ; 1,60 (m, 2H).

### 17.2. (3aR,5s,6aS)-5-(4-chloro-3-fluorophénoxy)hexahydrocyclopenta[c]pyrrole-2(1H)-carboxylate de (3-carbamoylisoxazol-5-yl)méthyle

On procède suivant le mode opératoire décrit dans l'exemple 1, étape 1.3.. A partir de 1,20 g (4,69 mmoles) de (3a*R*,5*s*,6a*S*)-5-(4-Chloro-3-fluoro-phénoxy)-octahydro-cyclopenta[c]pyrrole, obtenu à l'étape 17.1., de 1,73 g (5,63 mmoles) de 4-nitro-phényl-carbonate de 3-carbamoyl-isoxazol-5-ylméthyle, obtenu à l'étape 4.2., de 0,287 g (2,35 mmoles) de *N,N-*diméthylaminopyridine, de 2,04 mL (11,73 mmoles) de *N,N-*diisopropyléthylamine, et après chromatographie sur gel de silice en éluant avec un mélange de 98/2/0,2 de dichlorométhane, de méthanol et d'ammoniaque 30%, on obtient 1,45 g (73%) de produit attendu sous la forme d'une poudre blanche.
Point de fusion (°C) : 100-102°C
LC-MS : M+H = 424
RMN-¹H (DMSO) δ (ppm) : 8,15 (s, 1H) ; 7,85 (s, 1H); 7,45 (t, 1H) ; 7,05 (m, 1H) ; 6,80 (d, 2H) ; 5,25 (s, 2H) ; 5,00 (m, 1H) ; 3,55 (m, 2H) ; 3,20 (d, 2H) ; 2,85 (m, 2H) ; 2,00 (m, 2H) ; 1,90 (m, 2H).

Le tableau 1 qui suit illustre les structures chimiques et les propriétés physiques de quelques composés selon l'invention. Dans ce tableau :
- tous les composés sont sous forme de base libre à l'exception du composé de l'exemple 42 qui est sous forme de chlorhydrate en proportion base/sel de 1/1;

**Tableau 1**

| | | | | | | | | | | |
|---|---|---|---|---|---|---|---|---|---|---|
| 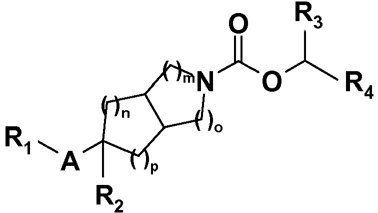 | | | | | | | | | | |

| **N°** | **R₁** | **m** | **n** | **o** | **p** | **A** | **R₂** | **R₃** | ***endo* / *exo*** | **R₄** |
|---|---|---|---|---|---|---|---|---|---|---|
| **1.** | 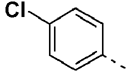 | **1** | **1** | **1** | **1** | **O** | **H** | **H** | ***exo*** | 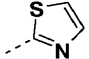 |
| **2.** | 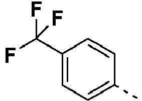 | **1** | **1** | **1** | **1** | **O** | **H** | **H** | ***exo*** | 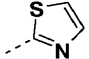 |
| **3.** | 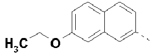 | **1** | **1** | **1** | **1** | **O** | **H** | **H** | ***exo*** | 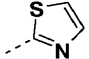 |
| **4.** | 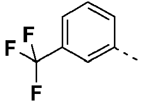 | **1** | **1** | **1** | **1** | **O** | **H** | **H** | ***endo*** | 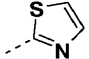 |
| **5.** | 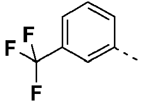 | **1** | **1** | **1** | **1** | **O** | **H** | **H** | ***exo*** | 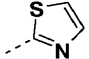 |
| **6.** | 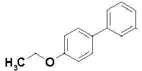 | **1** | **1** | **1** | **1** | **O** | **H** | **H** | ***endo*** | 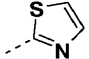 |
| **7.** | 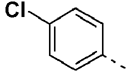 | **1** | **1** | **1** | **1** | **O** | **H** | **H** | ***exo*** | 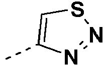 |
| **8.** | 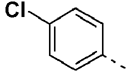 | **1** | **1** | **1** | **1** | **O** | **H** | **H** | ***exo*** | 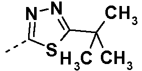 |
| **9.** | 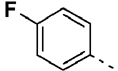 | **1** | **1** | **1** | **1** | **O** | **H** | **H** | ***exo*** | 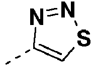 |
| **10.** | 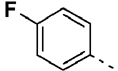 | **1** | **1** | **1** | **1** | **O** | **H** | **H** | ***exo*** | 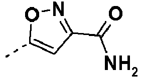 |
| **11.** | 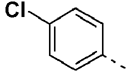 | **1** | **1** | **1** | **1** | **O** | **H** | **H** | ***exo*** | 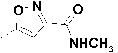 |
| **12.** | 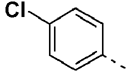 | **1** | **1** | **1** | **1** | **O** | **H** | **H** | ***exo*** | 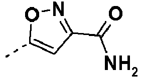 |
| **13.** | 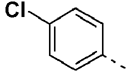 | **1** | **1** | **1** | **1** | **O** | **H** | **H** | ***exo*** | 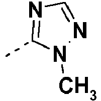 |
| **14.** | 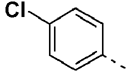 | **1** | **1** | **1** | **1** | **O** | **H** | **H** | ***exo*** | 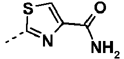 |
| **15.** | 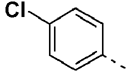 | **1** | **1** | **1** | **1** | **O** | **H** | **H** | ***exo*** | 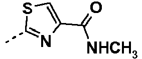 |
| **16.** | 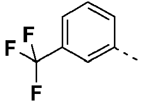 | **1** | **1** | **1** | **1** | **O** | **H** | **H** | ***exo*** | 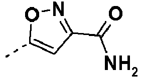 |
| **17.** | 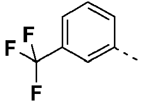 | **1** | **1** | **1** | **1** | **O** | **H** | **H** | ***exo*** | 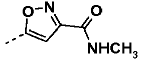 |
| **18.** | 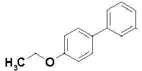 | **1** | **1** | **1** | **1** | **O** | **H** | **H** | ***exo*** | 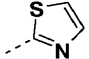 |
| **19.** | 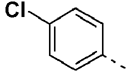 | **1** | **1** | **1** | **1** | **O** | **H** | **H** | ***exo*** | 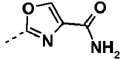 |
| **20.** | 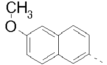 | **1** | **0** | **1** | **2** | **O** | **H** | **H** | ***exo*** | 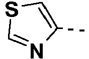 |
| **21.** | 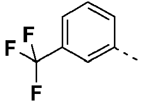 | **1** | **0** | **1** | **2** | **O** | **H** | **H** | ***endo*** | 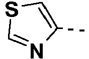 |
| **22.** | 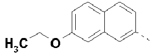 | **1** | **1** | **1** | **1** | **O** | **H** | **H** | ***exo*** | 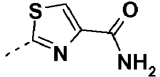 |
| **23.** | 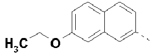 | **1** | **1** | **1** | **1** | **O** | **H** | **H** | ***exo*** | 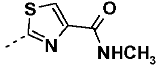 |
| **24.** | 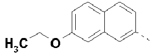 | **1** | **1** | **1** | **1** | **O** | **H** | **H** | ***exo*** | 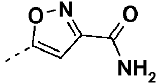 |
| **25.** | 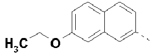 | **1** | **1** | **1** | **1** | **O** | **H** | **H** | ***exo*** | 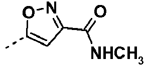 |
| **26.** | 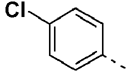 | **1** | **1** | **1** | **1** | **O** | **H** | **H** | ***exo*** | 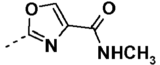 |
| **27.** | 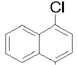 | **1** | **1** | **1** | **1** | **O** | **H** | **H** | ***exo*** | 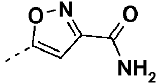 |
| **28.** | 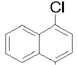 | **1** | **1** | **1** | **1** | **O** | **H** | **H** | ***exo*** | 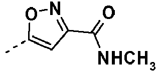 |
| **29.** | 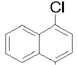 | **1** | **0** | **1** | **2** | **O** | **H** | **H** | ***exo*** | 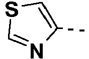 |
| **30.** | 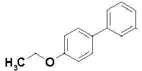 | **1** | **0** | **1** | **2** | **O** | **H** | **H** | ***endo*** | 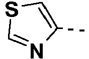 |
| **31.** | 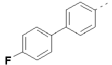 | **1** | **1** | **1** | **1** | **O** | **H** | **H** | ***exo*** | 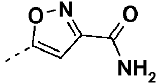 |
| **32.** | 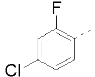 | **1** | **1** | **1** | **1** | **O** | **H** | **H** | ***exo*** | 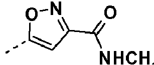 |
| **33.** | 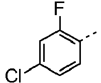 | **1** | **1** | **1** | **1** | **O** | **H** | **H** | ***exo*** | 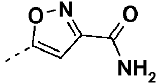 |
| **34.** | 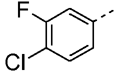 | **1** | **1** | **1** | **1** | **O** | **H** | **H** | ***exo*** | 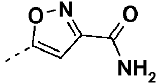 |
| **35.** | 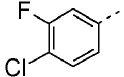 | **1** | **1** | **1** | **1** | **O** | **H** | **H** | ***exo*** | 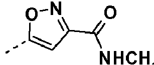 |
| **36.** | 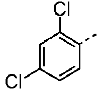 | **1** | **1** | **1** | **1** | **O** | **H** | **H** | ***exo*** | 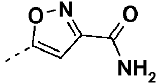 |
| **37.** | 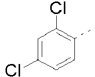 | **1** | **1** | **1** | **1** | **O** | **H** | **H** | ***exo*** | 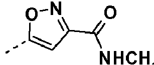 |
| **38.** | 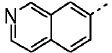 | **1** | **1** | **1** | **1** | **O** | **H** | **H** | ***exo*** | 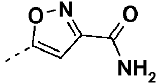 |
| **39.** | 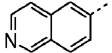 | **1** | **1** | **1** | **1** | **O** | **H** | **H** | ***exo*** | 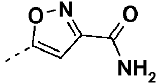 |
| **40.** | 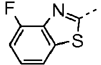 | **1** | **1** | **1** | **1** | **liaison** | **O H** | **H** | ***exo*** | 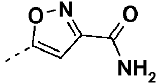 |
| **41.** | 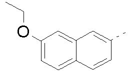 | **1** | **1** | **1** | **1** | **O** | **H** | **H** | ***exo*** | 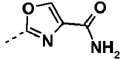 |
| **42.** | 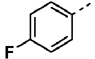 | **1** | **1** | **1** | **1** | **O** | **H** | **H** | ***exo*** | 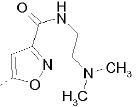 |
| **43.** | 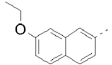 | **1** | **1** | **1** | **1** | **O** | **H** | **H** | ***exo*** | 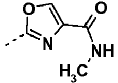 |
| **44.** | 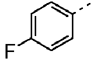 | **1** | **1** | **1** | **1** | **O** | **H** | **H** | ***exo*** | 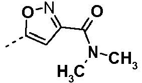 |
| **45.** | 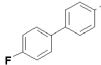 | **1** | **1** | **1** | **1** | **O** | **H** | **H** | ***exo*** | 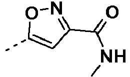 |

Le tableau 2 qui suit donne les résultats des analyses RMN ¹H, les points de fusions (PF), et les masses M+H mesurées (ou M-H lorsque la valeur est marquée d'un astérisque comme pour le composé n° 31 dont M-H = 464*) pour les composés du tableau 1.

**Tableau 2**

| ***N°*** | ***RMN 1H 400Mhz DMSO*** | ***PF*** | **M+H** |
|---|---|---|---|
| 1 | 7,8 (d, 1H) ; 7,75 (d, 1H) ; 7,35 (d, 2H) ; 6,90 (d, 2H) ; 5,35 (s, 2H) ; 4,95 (m, 2H) ; 3,55 (m, 2H) ; 3,25 (large m, 2H) ; 2,85 (large m , 2H) ; 2,0 (m, 2H) ; 1,85 (m, 2H). | 75-77°C | 379 |
| 2 | 7,85 (d, 1H) ; 7,75 (d, 1H) ; 7,60 (d, 2H) ; 7,10 (d, 2H) ; 5,35 (s, 2H) ; 5,00 (large s, 1H) ; 3,55 (large m, 2H) ; 3,30 (large m, 2H) ; 2,85 (large m , 2H) ; 2,05 (m, 2H) ; 1,90 (m, 2H). | 65-67°C | 413 |
| 3 | 7,80 (d, 1H) ; 7,70 (d, 1H) ; 7,65 (d, 2H) ; 7,15 (t, 2H) ; 6,95 (t, 2H) ; 5,35 (s, 2H) ; 5,05 (large s, 1H) ; 4,10 (q, 2H) ; 3,65 (large m, 2H) ; 3,25 (large m, 2H) ; 2,85 (large m , 2H) ; 2,10 (m, 2H) ; 1,95 (m, 2H) ; 1,40 (t, 3H). | 69-71°C | 339 |
| 4 | 7,80 (d, 1H) ; 7,75 (d, 1H) ; 7,50 (t, 1H) ; 7,30 (d, 1H) ; 7,20 (d, 1H) ; 7,15 (m, 1H) ; 5,25 (s, 2H) ; 4,95 (m, 1H) ; 3,55 (m, 2H) ; 3,40 (m, 2H) ; 2,80 (m, 2H) ; 2,30 (m, 2H) ; 1,70 (m, 2H) | huile | 413 |
| 5 | 7,80 (d, 1H) ; 7,75 (d, 1H) ; 7,50 (t, 1H) ; 7,30 (d, 1H) ; 7,20 (s,1H) ; 7,15 (d, 1H) ; 5,35 (s, 2H) ; 5,05 (m, 1H) ; 3,50 (m, 2H) ; 3,25 (m, 2H) ; 2,85 (m, 2H) ; 2,00 (m, 2H) ; 1,90 (m, 2H). | huile | 413 |
| 6 | 7,80 (d,1H) ; 7,70 (d, 1H) ; 7,60 (d, 2H) ; 7,30 (t, 1H) ; 7,15 (d, 1H) ; 7,05 (s, 1H) ; 7,00 (d, 2H) ; 6,80 (d, 1H) ; 5,35 (d, 2H) ; 5,00 (m, 1H) ; 4,10 (q, 2H) ; 3,55 (m, 2H) ; 3,35 (m, 2H) ; 2,75 (m, 2H) ; 2,30 (m, 2H) ; 1,70 (m, 2H) ; 1,35 (t, 3H) | huile | 465 |
| 7 | δ (ppm) : 9.20 (s, 1H); 7,30 (d, 2H); 6,90 (d, 2H); 5,55 (s, 2H); 4,95 (m, 1H) ; 3,55 (m, 2H) ; 3,20 (m, 2H) ; 2,80 (m, 2H) ; 2,05-1,90 (m, 2H) ; 1, 90-1, 80 (m, 2H). | 122-124°C | 380 |
| 8 | δ (ppm) : 7,30 (d, 2H) ; 6,90 (d, 2H); 5,4 (s, 2H); 4,95 (m, 1H); 3,55 (m, 2H); 3,25 (m, 2H); 2,85 (m, 2H); 2,00 (m, 2H); 1,90 (m, 2H); 1,45 (s, 9H). | cire | 436 |
| 9 | δ (ppm) : 9.20 (s, 1H); 7,10 (m, 2H); 6,90 (m, 2H); 5,55 (s, 2H); 4,90 (m, 1H) ; 3,55 (m, 2H) ; 3,20 (m, 2H) ; 2,80 (m, 2H) ; 2,05-1,90 (m, 2H) ; 1, 90-1, 80 (m, 2H). | 93-95°C | 364 |
| 10 | δ (ppm) : 8,15 (s, 1H); 7,85 (s, 1H) ; 7,10 (m, 2H) ; 6,90 (m, 2H); 6,80 (s, 1H); 5,25 (s, 2H); 4,95 (m, 1H); 3,55 (m, 2H); 3,20 (m, 2H) ; 2,80 (m, 2H) ; 2, 05-1, 90 (m, 2H); 1,90-1,80 (m, 2H). | 151-152°C | 390 |
| 11 | δ (ppm) : 8,70 (s, 1H); 7,30 (d, 2H); 6,95 (d, 2H); 6,80 (s, 1H); 5,25 (s, 2H) ; 4,95 (m, 1H); 3,55 (m, 2H); 3,25 (m, 2H); 2,85 (m, 2H); 2,80 (d, 3H); 2,05-1,90 (m, 2H); 1,90-1,80 (m, 2H). | 117-118°C | 420 |
| 12 | δ (ppm) : 8,15 (s, 1H); 7,85 (s, 1H); 7,30 (d, 2H); 6,90 (d, 2H); 6,80 (s, 1H); 5,25 (s, 2H); 4,95 (m, 1H); 3,55 (m, 2H); 3,20 (m, 2H) ; 2,85 (m, 2H) ; 2, 05-1, 90 (m, 2H); 1,90-1,80 (m, 2H). | 126-127°C | 406 |
| 13 | δ (ppm) : 7,90 (s, 1H) ; 7,30 (d, 2H); 6,90 (d, 2H); 5,25 (s, 2H); 4,95 (m, 1H); 3,90 (s, 3H); 3,55 (m, 2H); 3,20 (m, 2H); 2,80 (m, 2H); 2,05-1,90 (m, 2H); 1,90-1,80 (m, 2H). | 58-60°C | 377 |
| 14 | δ (ppm) : 8,25 (s, 1H); 7,75 (s, 1H); 7,55 (s, 1H); 7,30 (d, 2H); 6,90 (d, 2H); 5,35 (s, 2H); 4,95 (m, 1H); 3,55 (m, 2H); 3,25 (m, 2H) ; 2,85 (m, 2H) ; 2, 05-1, 90 (m, 2H); 1,90-1,80 (m, 2H). | 171-173°C | 422 |
| 15 | δ (ppm) : 8,35 (m, 1H); 8,25 (s, 1H); 7,30 (d, 2H); 6,95 (d, 2H); 5,35 (s, 2H) ; 4,95 (m, 1H) ; 3,55 (m, 2H); 3,25 (m, 2H); 2,85 (m, 2H); 2,80 (d, 3H); 2,05-1,95 (m, 2H); 1,95-1,80 (m, 2H). | 129-131 °C | 436 |
| 16 | δ (ppm) : 8,15 (s, 1H) ; 7,85 (s, 1H); 7,55 (t, 1H); 7,25 (d, 1H); 7,20 (d, 1H); 7,15 (s, 1H); 6,80 (s, 1H); 5,25 (s, 2H); 5,10 (m, 1H); 3,55 (m, 2H); 3,20 (m, 2H); 2,85 (m, 2H); 2,05-1,95 (m, 2H); 1,95-1,80 (m, 2H). | 130-132°C | 440 |
| 17 | δ (ppm) : 8,70 (s, 1H); 7,50 (t, 1H); 7,25 (d, 1H); 7,20 (d, 1H); 7,15 (s, 1H); 6,80 (s, 1H) ; 5,25 (s, 2H) ; 5,10 (m, 1H) ; 3,55 (m, 2H); 3,25 (m, 2H); 2,85 (m, 2H); 2,80 (s, 2H); 2,10-2,00 (m, 2H); 2,00-1,85 (m, 2H). | 98-100°C | 454 |
| 18 | 7,80 (d,1H) ; 7,75 (d, 1H) ; 7,60 (d, 2H) ; 7,35 (t, 1H) ; 7,15 (d, 1H) ; 7,05 (s, 1H) ; 7,00 (d, 2H) ; 6,85 (d, 1H) ; 5,35 (s, 2H) ; 5,10 (m, 1H) ; 4,10 (q, 2H) ; 3,55 (m, 2H) ; 3,30 (m, 2H) ; 2,90 (m, 2H) ; 2,10 (m, 2H) ; 1,90 (m, 2H) ; 1,40 (t, 3H) | huile | 465 |
| 19 | δ (ppm) : 8,60 (s, 1H); 7,65 (s, 1H); 7,50 (s, 1H); 7,30 (d, 2H); 6,90 (d, 2H); 5,20 (s, 2H); 4,95 (m, 1H); 3,55 (m, 2H); 3,20 (m, 2H) ; 2,80 (m, 2H) ; 2, 05-1, 95 (m, 2H); 1,95-1,80 (m, 2H). | 139-141 °C | 406 |
| 20 | 9.10 (s, 1H) ; 7,75 (m, 3H) ; 7,20 (large s, 2H) ; 7.10 (m, 2H) ; 5,20 (s, 2H) ; 4,80 (s, 1H) ; 3,85 (s, 3H); 3,70 (m, 1H) ; 3,55 (m, 1H) ; 3,35 (m, 1H) ; 3,25 (m, 1H) ; 2,80 (m , 2H) ; 2,15 (m, 1H) ; 2,05 (m, 1H) ; 1,80 (m, 1H); 1,55 (m, 1H) | huile | 425 |
| 21 | 9.15 (s, 1H) ; 7,65 (m, 1H) ; 7,55 (m, 1H) ; 7,25 (m, 3H) ; 5,20 (s, 2H) ; 4,95 (m, 1H) ; 3,60 (m, 2H) ; 3,30 (m, 2H) ; 3,05 (m, 1H) ; 2,80 (m , 1H) ; 2,05 (m, 1H) ; 1,90 (m, 2H); 1,55 (m, 1H) | huile | 413 |
| 22 | δ (ppm) : 8,30 (s, 1H); 7,75 (d, 2H); 7,75 (s, 1H); 7,60 (s, 1H); 7,20 (m, 2H); 6,95 (m, 2H); 5,40 (s, 2H); 5,10 (m, 1H); 4,15 (qd, 2H); 3,60 (m, 2H); 3,25 (m, 2H); 2,90 (m, 2H); 2,15-2,05 (m, 2H); 2,05-1,90 (m, 2H); 1,40 (t, 3H). | 143-145°C | 482 |
| 23 | δ (ppm) : 8,40 (m, 1H); 8,25 (s, 1H); 7,75 (d, 2H); 7,20 (m, 2H); 6,95 (m, 2H); 5,40 (s, 2H); 5,10 (m, 1H); 4,15 (qd, 2H); 3,60 (m, 2H); 3,25 (m, 2H); 2,90 (m, 2H); 2,80 (d, 3H); 2,15-2,05 (m, 2H); 2,05-1,90 (m, 2H); 1,40 (t, 3H). | 149-151 °C | 496 |
| 24 | δ (ppm) : 8,11 (s, 1H); 7,82 (s, 1H); 7,69 (d, 2H); 7,18 (s, 1H); 7,17 (s, 1H); 6,95 (m, 2H); 6,80 (s, 1H), 5,24 (s, 2H); 5,06 (m, 1H); 4,12 (qd, 2H); 3,56 (m, 2H); 3,23 (m, 2H); 2,85 (m, 2H); 2,07 (m, 2H); 1,94 (m, 2H); 1,38 (t, 3H). | 162-164°C | 466 |
| 25 | δ (ppm) : 8,69 (s, 1H); 7,69 (d, 2H); 7,18 (s, 1H); 7,16 (s, 1H); 6,95 (m, 2H); 6,81 (s, 1H), 5,25 (s, 2H); 5,06 (m, 1H); 4,12 (qd, 2H); 3,56 (m, 2H); 3,23 (m, 2H); 2,85 (m, 2H); 2,77 (d, 3H); 2,06 (m, 2H); 1,94 (m, 2H); 1,38 (t, 3H). | 108-110°C | 480 |
| 26 | δ (ppm) : 8,60 (s, 1H); 8,25 (s, 1H); 7,30 (d, 2H); 6,95 (d, 2H); 5,20 (s, 2H) ; 4,95 (m, 1H); 3,55 (m, 2H); 3,20 (m, 2H); 2,85 (m, 2H); 2,80 (d, 3H); 2,05-1,95 (m, 2H); 1,95-1,80 (m, 2H). | 104-106°C | 420 |
| 27 | δ (ppm) : 8,25 (d, 1H); 8,15 (d, 1H); 8,15 (s, 1H); 7,85 (s, 1H); 7,70 (t, 1H); 7,65 (t, 1H); 7,60 (d, 1H); 6,95 (d, 1H); 6,80 (s, 1H), 5,25 (s, 2H); 5,20 (m, 1H); 3,60 (m, 2H); 3,25 (m, 2H); 2,95 (m, 2H); 2,15 (m, 2H); 1,95 (m, 2H) . | 185-187°C | 456 |
| 28 | δ (ppm) : 8,70 (s, 1H); 8,25 (d, 1H); 8,15 (d, 1H); 7,75 (t, 1H); 7,65 (t, 1H); 7,60 (d, 1H); 6,95 (d, 1H); 6,82 (s, 1H), 5,25 (s, 2H); 5,20 (m, 1H); 3,55 (m, 2H); 3,25 (m, 2H) ; 2,95 (m, 2H) ; 2,80 (d, 3H); 2,15 (m, 2H); 1,95 (m, 2H). | 131-133°C | 470 |
| 29 | 9,10 (s, 1H) ; 8,25 (d, 1H) ; 8,15 (d, 1H) ; 7,75-7,60 (m, 4H) ; 7,00 (d, 1H) ; 5,20 (s, 2H) ; 4,90 (s, 1H) ; 3,70 (m, 1H); 3,60 (m, 1H); 3,30 (m, 2H) ; 2,90 (m, 2H) ; 2,20 (m, 2H) ; 1,90 (m, 1H) ; 1,55 (m, 1H). | Huile | 429 |
| 30 | 9,10 (s, 1H) ; 7,70 (s, 1H) ; 7,60 (d, 2H) ; 7,35 (t, 1H) ; 7,20 (d, 1H) ; 7,10 (s, 1H) ; 7,00 (d, 2H) ; 6,90 (d, 1H) ; 5,20 (s, 2H) ; 4,90 (q, 1H) ; 4,10 (q, 2H) ; 3,60 (m, 2H) ; 3,30 (m, 2H) ; 3,05 (m, 1H) ; 2,80 (m, 1H) ; 2,10 (m, 1H) ; 1,85 (m,2H) ; 1,55 (m, 1H) ; 1,35 (t, 3H) | huile | 465 |
| 31 | δ (ppm) : 8,15 (s, 1H); 7,85 (s, 1H); 7,65 (t, 2H); 7,55 (d, 2H); 7,25 (t, 2H); 7,00 (d, 2H); 6,80 (s, 1H); 5,25 (s, 2H); 5,00 (m, 1H); 3,55 (m, 2H); 3,25 (m, 2H); 2,85 (m, 2H) ; 2,05 (m, 2H) ; 1,90 (m, 2H). | 200-201 °C | 464* |
| 32 | δ (ppm) : 8,70 (s, 1H); 7,45 (d, 1H); 7,20 (m, 2H); 6,80 (s, 1H); 5,25 (s, 2H); 4,95 (m, 1H) ; 3,55 (m, 2H); 3,20 (d, 2H); 2,85 (m, 2H); 2,80 (s, 3H); 2,05 (m, 2H); 1,90 (m, 2H). | 104-106°C | 438 |
| 33 | δ (ppm) : 8,10 (s, 1H); 7,85 (s, 1H); 7,40 (d, 1H); 7,20 (m, 2H); 6,80 (s, 1H); 5,25 (s, 2H); 5,00 (m, 1H); 3,55 (m, 2H); 3,25 (d, 2H); 2,85 (m, 2H); 2,05 (m, 2H); 1,85 (m, 2H). | 113-115°C | 424 |
| 34 | δ (ppm) : 8,15 (s, 1H) ; 7,85 (s, 1H); 7,45 (t, 1H); 7,05 (m, 1H); 6,80 (d, 2H); 5,25 (s, 2H); 5,00 (m, 1H); 3,55 (m, 2H); 3,20 (d, 2H); 2,85 (m, 2H); 2,00 (m, 2H); 1,90 (m, 2H). | 100-102°C | 422* |
| 35 | δ (ppm) : 8,70 (s, 1H); 7,45 (t, 1H) ; 7,05 (d, 1H) ; 6,80 (m, 2H) ; 5,25 (s, 2H) ; 4,95 (m, 1H) ; 3,55 (m, 2H) ; 3,20 (d, 2H) ; 2,85 (m, 2H); 2, 80 (s, 3H); 2,00 (m, 2H); 1,90 (m, 2H). | 101-102°C | 438 |
| 36 | δ (ppm) : 8,10 (s, 1H); 7,85 (s, 1H); 7,55 (s, 1H); 7,35 (d, 1H); 7,15 (d, 1H); 6,80 (s, 1H); 5,25 (s, 2H); 5,05 (m, 1H); 3,55 (m, 2H); 3,20 (d, 2H); 2,85 (m, 2H); 2,00 (m, 2H) ; 1,85 (m, 2H). | 99-100°C | 438 |
| 37 | δ (ppm) : 8,70 (m, 1H); 7,55 (s, 1H); 7,35 (d, 1H); 7,15 (d, 1H); 6,80 (s, 1H); 5,25 (s, 2H); 5,05 (m, 1H); 3,55 (m, 2H); 3,25 (d, 2H); 2,85 (m, 2H); 2,80 (s, 3H); 2,05 (m, 2H) ; 1,85 (m, 2H). | 105-107°C | 454 |
| 38 | δ (ppm) : 9,20 (s, 1H); 8,40 (d, 1H); 8,15 (s, 1H); 7,90 (d, 1H); 7,85 (s, 1H); 7,75 (d, 1H); 7,50 (s, 1H); 7,40 (d, 1H); 6,85 (s, 1H); 5,25 (s, 2H); 5,15 (m, 1H); 3,60 (m, 2H); 3,25 (m, 2H); 2,90 (m, 2H); 2,10 (m, 2H); 2,00 (m, 2H). | 170-172°C | 423 |
| 39 | δ (ppm) : 9,15 (s, 1H); 8,40 (d, 1H); 8,15 (s, 1H); 8,00 (d, 1H); 7,85 (s, 1H); 7,70 (d, 1H); 7,30 (s, 1H); 7,25 (d, 1H); 6,80 (s, 1H); 5,25 (s, 2H); 5,15 (m, 1H); 3,60 (m, 2H); 3,25 (m, 2H); 2,85 (m, 2H); 2,10 (m, 2H); 2,00 (m, 2H). | 208-210°C | 423 |
| 40 | δ (ppm) : 8,15 (s, 1H) ; 7,90 (d, 1H); 7,85 (s, 1H); 7,40 (t, 1H); 7,35 (t, 1H); 6,80 (s, 1H); 6,45 (s, 1H); 5,25 (s, 2H); 3,65 (m, 2H); 3,45 (d, 2H); 3,00 (m, 2H); 2,50 (m, 2H); 2,00 (d, 2H). | 173-174°C | 447 |
| 41 | δ (ppm) : 8,60 (s, 1H); 7,70 (d, 2H); 7,65 (s, 1H); 7,50 (s, 1H); 7,20 (d, 2H); 6,95 (t, 2H); 5,20 (s, 2H); 5,05 (m, 1H); 4,15 (qd, 2H); 3,55 (m, 2H); 3,25 (m, 2H); 2,90 (m, 2H); 2,05 (m, 2H) ; 1,95 (m, 2H); 1,40 (t, 3H). | 166-168°C | 466 |
| 42 | δ (ppm) : 10,10 (large s, 1H); 9,05 (t, 1H); 7,10 (t, 2H); 6,95 (m, 3H); 5,25 (s, 2H); 4,90 (m, 1H); 3,65 (m, 2H); 3,55 (m, 2H); 3,25 (m, 2H); 3,20 (m, 2H); 2,85 (s, 8H); 2,00 (m, 2H); 1,85 (m, 2H). | 146-148°C (HCl) | 461 |
| 43 | δ (ppm) : 8,60 (s, 1H); 8,25 (s, 1H); 7,70 (d, 2H); 7,20 (d, 2H); 6,95 (t, 2H); 5,20 (s, 2H); 5,05 (m, 1H); 4,15 (qd, 2H); 3,55 (m, 2H); 3,25 (m, 2H); 2,90 (m, 2H); 2,75 (d, 3H); 2,05 (m, 2H); 1,95 (m, 2H); 1,40 (t, 3H). | 130-132°C | 480 |
| 44 | δ (ppm) : 7,10 (t, 2H) ; 6,90 (d, 2H); 6,75 (s, 1H); 5,25 (s, 2H); 4,90 (m, 1H); 3,55 (m, 2H); 3,20 (d, 2H); 3,10 (s, 3H); 3,05 (s, 3H); 2,80 (m, 2H); 2,00 (m, 2H); 1,85 (m, 2H). | huile | 418 |
| 45 | δ (ppm) : 8,70 (large s, 1H); 7,65 (m, 2H); 7,55 (m, 2H); 7,22 (t, 2H); 6,98 (d, 2H); 6,82 (s, 1H); 5,25 (s, 2H); 5,00 (m, 1H); 3,50 (m, 2H); 3,21 (m, 2H); 2,85 (m, 2H); 2,78 (s, 3H); 2,05 (m, 2H); 1,90 (m, 2H). | 154-156°C | 480 |

Les composés de l'invention ont fait l'objet d'essais pharmacologiques permettant de déterminer leur effet inhibiteur de l'enzyme FAAH (Fatty Acid Amide Hydrolase).

### Protocole 1

L'activité inhibitrice a été mise en évidence dans un test radioenzymatique basé sur la mesure du produit d'hydrolyse de l'anandamide [éthanolamine 1-³H] par la FAAH (Life Sciences (1995), 56, 1999-2005 et Journal of Biochemical and Biophysical Methods (2004), 60(2), 171-177). Ainsi, les cerveaux de souris (moins le cervelet) sont prélevés et conservés à -80°C. Les homogénats membranaires sont préparés extemporanément par homogénéisation des tissus à l'aide d'un appareil Precellys® dans le tampon de réaction (Tris-HCl 10 mM pH=8, NaCl 150 mM et acide éthylène-diamine-tétraacétique (EDTA) 1 mM). La réaction enzymatique est conduite dans des plaques de filtration Multiscreen 96puits dans un volume final de 70µl. Du tampon de réaction supplémenté avec de l'albumine de sérum bovin sans acides gras (BSA, 1 mg/ml) est utilisé pour la réaction enzymatique, la dilution des composés et de l'anandamide [éthanolamine 1-³H]. Sont ajoutés successivement dans les puits, du tampon de réaction contenant la BSA (43µl/puits), les composés dilués testés à différentes concentrations (7µl/puits contenant 1% de DMSO) et la préparation membranaire (10µl/puits soit 200 µg de tissu par essai). Après 20 minutes de pré-incubation des composés avec l'enzyme à 25°C, la réaction est démarrée par l'addition d'anandamide [éthanolamine 1-³H] (activité spécifique de 15-20 Ci/mmol) diluée avec de l'anandamide froide (10µl/puits, concentration finale de 10 µM, 0,01µCi par essai). Après 20 minutes d'incubation à 25°C, la réaction enzymatique est arrêtée par addition d'une solution de charbon actif 5M préparée dans un tampon NaCl 1,5M et HCl 0,5M (50µl/puits). Le mélange est agité 10 minutes puis la phase aqueuse contenant l'éthanolamine [1-³H] est récupérée par filtration sous-vide et comptée par scintillation liquide.

### Protocole 2

L'activité inhibitrice a été mise en évidence par technique fluorescente dans un test enzymatique basé sur la mesure du produit d'hydrolyse fluorescent de l'arachidonoyl 7-amino 4-méthyl coumarin amide (AAMC) par la FAAH (Analytical Biochemistry (2005), 343:143-151, J. of Biomolecular Screening (2006), 11(5): 519-527 et J. of Neurosciences Methods (2007), 161: 47-54). Ainsi, les cerveaux de souris (moins le cervelet) sont prélevés et conservés à -80°C. Les homogénats de cerveaux sont préparés extemporanément par homogénéisation des tissus à l'aide d'un appareil Precellys® dans le tampon de réaction (Tris-HCl 10 mM pH=8, NaCl 150 mM et acide éthylène-diamine-tétraacétique (EDTA) 1 mM). La réaction enzymatique est conduite dans des plaques 384 puits noires en polystyrène dans un volume final de 50µL. Du tampon de réaction supplémenté avec de l'albumine de sérum bovin sans acides gras (BSA, 1 mg/ml) est utilisé pour la réaction enzymatique, la dilution des composés et la dilution de l'AAMC. Sont ajoutés successivement dans les puits, du tampon de réaction contenant la BSA (25µl/puits), les composés dilués testés à différentes concentrations (5µl/puits contenant 1% de DMSO) et la préparation membranaire (10µl/puits soit 200 µg de tissu par essai). Après 20 minutes de pré-incubation des composés avec l'enzyme à 25°C, la réaction est démarrée par l'addition de 10µL de substrat par puits (AAMC, concentration finale de 10 µM). Après 40 minutes d'incubation à 37°C, l'aminométhyl coumarin (AMC) produit est mesuré par comptage fluorescent (lecteur de plaque Envision).

Dans les conditions du protocole 1, les composés les plus actifs de l'invention présentent des CI₅₀ (concentration inhibant de 50 % l'activité enzymatique contrôle de la FAAH) comprises entre 0,001 et 1 µM. Par exemple les composés n°3, 7, 12, 23, 28 et 34 ont des CI₅₀ respectives de 3,5 nM, 89 nM, 19 nM, 34 nM, 12 nM et 3,2 nM.
Dans les conditions du protocole 2, les composés les plus actifs de l'invention présentent des CI₅₀ (concentration inhibant de 50 % l'activité enzymatique contrôle de la FAAH) comprises entre 0,001 et 1 µM. Par exemple les composés n°44 et n°45 ont des CI₅₀ respectives de 7.4 nM et 0,47 nM.
Il apparaît donc que les composés selon l'invention ont une activité inhibitrice sur l'enzyme FAAH.

L'activité *in vivo* des composés de l'invention a été évaluée dans un test d'analgésie.
Ainsi, l'administration intrapéritonéale (i.p.) de PBQ (phénylbenzoquinone, 2 mg/kg dans une solution de chlorure de sodium à 0,9 % contenant 5 % d'éthanol) chez des souris mâles OF1 de 25 à 30 g, provoque des étirements abdominaux, en moyenne 30 torsions ou contractions pendant la période de 5 à 15 minutes après injection. Les composés testés sont administrés par voie orale (p.o.) ou par voie intrapéritonéale (i.p.) en suspension dans du Tween 80 à 0,5 %, 60 minutes ou 120 minutes avant l'administration de PBQ. Dans ces conditions, les composés les plus puissants de l'invention réduisent de 35 à 80 % le nombre d'étirements induits par le PBQ, dans une gamme de doses comprise entre 1 et 30 mg/kg.
Par exemple, les composés n°24 et n°35 du tableau 1 réduisent de 50% le nombre d'étirements induits par le PBQ, à la dose de 30 mg/kg p.o. à 120 minutes.

L'enzyme FAAH (Chemistry and Physics of Lipids, (2000), 108, 107-121) catalyse l'hydrolyse des dérivés endogènes d'amides et d'esters de différents acides gras tels que la *N*-arachidonoyléthanolamine (anandamide), la *N*-palmitoyl-éthanolamine, la *N*-oléoyléthanolamine, l'oléamide ou le 2-arachidonoylglycérol. Ces dérivés exercent différentes activités pharmacologiques en interagissant, entre autres, avec les récepteurs cannabinoïdes et vanilloïdes.
Les composés de l'invention, bloquent cette voie de dégradation et augmentent le taux tissulaire de ces substances endogènes. Ils peuvent être utilisés à ce titre dans la prévention et le traitement des pathologies dans lesquelles les cannabinoïdes endogènes et/ou tous autres substrats métabolisés par l'enzyme FAAH, sont impliqués. On peut par exemple citer les maladies et les affections suivantes :
la douleur notamment les douleurs aiguës ou chroniques de type neurogène : migraine, douleurs neuropathiques incluant les formes associées au virus de l'herpès et au diabète et à la chimiothérapie, les douleurs aiguës ou chroniques associées aux maladies inflammatoires : arthrite, arthrite rhumatoïde, ostéoarthrite, spondylite, goutte, vascularite, maladie de Crohn, syndrome du colon irritable, les douleurs aiguës ou chroniques périphériques, les vertiges, les vomissements, les nausées en particulier celles consécutives à une chimiothérapie, les troubles du comportement alimentaire en particulier les anorexies et cachexies de diverses natures, les pathologies neurologiques et psychiatrique tremblements, dyskinésies, dystonies, spasticité, comportements compulsifs et obsessionnels, syndrome de Tourette, toutes les formes de dépression et d'anxiété de toute nature et origine, troubles de l'humeur, psychoses, les maladies neuro-dégénératives aiguës et chroniques : maladie de Parkinson, maladie d'Alzheimer, démence sénile, chorée de Huntington, lésions liées à l'ischémie cérébrale et aux traumatismes crâniens et médullaires, l'épilepsie, les troubles du sommeil incluant les apnées du sommeil, les maladies cardiovasculaires en particulier hypertension, arythmies cardiaques, artériosclérose, crise cardiaque, ischémies cardiaques, l'ischémie rénale, les cancers : tumeurs bénignes de la peau, papillomes et tumeurs cérébrales, tumeurs de la prostate, tumeurs cérébrales (glioblastomes, médullo-épithéliomes, médulloblastomes, neuroblastomes, tumeurs d'origine embryonnaire, astrocytomes, astroblastomes, épendyomes, oligodendrogliomes, tumeur du plexus, neuroépithéliomes, tumeur de l'épiphyse, épendymoblastomes, méningiomes malins, sarcomatoses, mélanomes malins, schwénnomes), les désordres du système immunitaire, notamment les maladies auto-immunes : psoriasis, lupus érythémateux, maladies du tissu conjonctif ou connectivites, syndrome de Sjögrer's, spondylarthrite ankylosante, spondylarthrite indifférenciée, maladie de Behcet's, anémies auto-immunes hémolytiques, sclérose en plaques, sclérose latérale amyo-trophique, amyloses, rejet de greffes, maladies affectant la lignée plasmocytaire, les maladies allergiques : l'hypersensibilité immédiate ou retardée, rhinites ou conjonctivites allergiques, dermatites de contact, les maladies infectieuses parasitaires, virales ou bactériennes : SIDA, méningites, les maladies inflammatoires, notamment les maladies articulaires : arthrite, arthrite rhumatoïde, ostéoarthrite, spondylite, goutte, vascularite, maladie de Crohn, syndrome du colon irritable, l'ostéoporose, les affections oculaire hypertension oculaire, glaucome, les affections pulmonaires : maladies des voies respiratoires, bronchospasmes, toux, asthme, bronchite chronique, obstruction chronique des voies respiratoires, emphysème, les maladies gastro-intestinales: syndrome du colon irritable, désordres inflammatoires intestinaux, ulcères, diarrhées, l'incontinence urinaire et l'inflammation vésicale.

L'utilisation des composés selon l'invention, à l'état de base, de sel d'addition à un acide, d'hydrate ou de solvat pharmaceutiquement acceptable, pour la préparation d'un médicament destiné à traiter les pathologies ci-dessus mentionnées fait partie intégrante de l'invention.

L'invention a également pour objet des médicaments qui comprennent un composé de formule (I), ou un sel d'addition à un acide, ou encore un hydrate ou un solvat pharmaceutiquement acceptable du composé de formule (I). Ces médicaments trouvent leur emploi en thérapeutique, notamment dans le traitement des pathologies ci-dessus mentionnées.

Selon un autre de ses aspects, la présente invention concerne des compositions pharmaceutiques renfermant en tant que principe actif, au moins un composé selon l'invention. Ces compositions pharmaceutiques contiennent une dose efficace d'un composé selon l'invention, ou un sel d'addition à un acide, ou un hydrate, ou un solvat pharmaceutiquement acceptable dudit composé, et éventuellement un ou plusieurs excipients pharmaceutiquement acceptables.
Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intrathécale, intranasale, transdermique, pulmonaire, oculaire ou rectale, le principe actif de formule (I) ci-dessus, ou son sel d'addition à un acide, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intratrachéale, intraoculaire, intranasale, par inhalation, les formes d'administration sous-cutanée, intramusculaire ou intraveineuse et les formes d'administration rectale ou vaginale. Pour l'application topique, on peut utiliser les composés selon l'invention dans des crèmes, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,01 à 20 mg de principe actif par kg de poids corporel, selon la forme galénique.

Il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

L'invention selon un autre de ses aspects, concerne également l'utilisation d'un composé de formule (I) pour la préparation d'un médicament destiné à traiter des pathologies ci-dessus indiquées qui comprend l'administration d'une dose

## Revendications

1. Composé répondant à la formule (I) 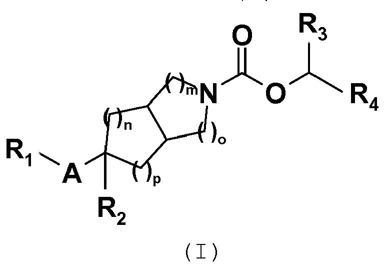 dans laquelle
R₂ représente un atome d'hydrogène, de fluor ou un groupe hydroxyle, cyano, trifluorométhyle, C₁₋₆-alkyle, C₁₋₆-alcoxy, NR₈R₉ ;
m, n, o et p ont pour valeur 1, ou bien, m et o ont pour valeur 1, n a pour valeur 0 et p a pour valeur 2;A représente une liaison covalente ou un atome d'oxygène ;
R₁ représente un groupe R₅ éventuellement substitué par un ou plusieurs groupes R₆, et/ou R₇ ;
R₅ représente un groupe choisi parmi un phényle, pyridinyle, pyridazinyle, pyrimidinyle, pyrazinyle, triazinyle, naphtalènyle, quinolinyle, isoquinolinyle, phtalazinyle, quinazolinyle, quinoxalinyle, cinnolinyle, naphthyridinyle, benzothiazolyle, benzoxazolyle, benzimidazolyle, benzisothiazolyle, bensisoxazolyle, indazolyle, benzotriazolyle ;
R₆ représente un atome d'halogène, un groupe cyano, -CH₂CN, nitro, hydroxyle, C₁₋₆-alkyle, C₁₋₆-alcoxy, C₁₋₆-thioalkyle, C₁₋₆-haloalkyle, C₁₋₆-haloalcoxy, C₁₋₆-halothioalkyle, C₃₋₇-cycloalkyle, C₃₋₇-cycloalkyle-C₁₋₃-alkylène, C₃₋₇-cycloalkyle-C₁₋₃-alkylène-O-, NR₈R₉, NR₈COR₉, NR₈CO₂R₉, NR₈SO₂R₉, NR₈SO₂NR₈R₉, COR₈, CO₂R₈, CONR₈R₉, SO₂R₈, SO₂NR₈R₉ ou -O-(C₁₋₃-alkylène)-O- ;
R₇ représente un groupe choisi parmi un phényle, pyridinyle, pyridazinyle, pyrimidinyle, pyrazinyle, triazinyle; le ou les groupes R₇ pouvant être substitués par un ou plusieurs groupes R₆ identiques ou différents l'un de l'autre ; le ou les groupes R₇ pouvant être substitués par un ou plusieurs groupes R₆ identiques ou différents l'un de l'autre ;
R₃ représente un atome d'hydrogène, de fluor, un groupe C₁₋₆-alkyle ou un groupe trifluorométhyle ;
R₄ représente un groupe choisi parmi un thiazolyle, un thiadiazolyle, un triazolyle, un oxazolyle, un isoxazolyle; ce groupe étant non substitué ou substitué par un ou plusieurs groupes C₁₋₆-alkyle, CONR₈R₉ ou CON (R₈) (C₁₋₃-alkylène-NR₁₀R₁₁) ;
R₈, R₉, R₁₀ et R₁₁ représentent indépendamment l'un de l'autre un atome d'hydrogène ou un groupe C₁₋₆-alkyle ;à l'état de base ou de sel d'addition à un acide.

2. Composé de formule (I) selon la revendication 1 **caractérisé en ce que** R₂ représente un atome d'hydrogène ou un groupe hydroxyle;
à l'état de base ou de sel d'addition à un acide.

3. Composé de formule (I) selon la revendication 1 ou 2 **caractérisé en ce que** R₁ représente un groupe R₅ non substitué ou substitué par un ou plusieurs groupes R₆ et/ou R₇ ;
R₅ représente un groupe phényle, naphtalènyle, benzothiazolyle ou isoquinolinyle ;
R₆ représente un atome d'halogène ou un groupe C₁₋₆-haloalkyle ou un groupe C₁₋₆-alcoxy ;
R₇ représente un phényle pouvant être substitué par un ou plusieurs groupes R₆ identiques ou différents l'un de l'autre; à l'état de base ou de sel d'addition à un acide.

4. Composé de formule (I) selon l'une quelconque des revendications 1 à 3 **caractérisé en ce que** R₃ représente un atome d'hydrogène; à l'état de base ou de sel d'addition à un acide.

5. Composé de formule (I) choisi parmi :
(3a*R*,5*s*,6a*S*)-5-(4-chlorophénoxy)hexahydrocyclopenta[*c*]pyrrole-2(1*H*)-carboxylate de thiazol-2-ylméthyle (exo)
(3a*R*,5*s*,6a*S*)-5-[4-(trifluorométhyl)phénoxy]hexahydrocyclopenta[*c*]pyrrole-2(1*H*)-carboxylate de thiazol-2-ylméthyle (exo)
(3a*R*,5*s*,6a*S*)-5-[(7-éthoxynaphthalèn-2-yl)oxy]hexahydrocyclopenta[*c*]pyrrole-2(1*H*)-carboxylate de thiazol-2-ylméthyle (exo)
(3a*R*,5*r*,6a*S*)-5-[3-(trifluorométhyl)phénoxy]hexahydrocyclopenta[*c*]pyrrole-2(1*H*)-carboxylate de thiazol-2-ylméthyle (endo)
(3a*R*,5*s*,6a*S*)-5-[3-(trifluoromethyl)phenoxy]hexahydrocyclopenta[*c*]pyrrole-2(1*H*)-carboxylate de thiazol-2-ylméthyle (exo)
(3a*R*,5*r*,6a*S*)-5-[(4'-éthoxybiphényl-3-yl)oxy]hexahydrocyclopenta[*c*]pyrrole-2(1*H*)-carboxylate de thiazol-2-ylméthyle (endo)
(3*aR*,5*s*,6*aS*)-5-(4-chlorophénoxy)hexahydrocyclopenta[*c*]pyrrole-2(1*H*)-carboxylate de 1,2,3-thiadiazol-4-ylméthyle (exo)
(3a*R*,5*s*,6a*S*)-5-(4-chlorophénoxy)hexahydrocyclopenta[*c*]pyrrole-2(1*H*)-carboxylate de (5-*tert*-butyl-1,3,4-thiadiazol-2-yl)méthyle (exo)
(3a*R*,5*s*,6a*S*)-5-(4-fluorophénoxy)hexahydrocyclopenta[*c*]pyrrole-2(1*H*)-carboxylate de 1,2,3-thiadiazol-4-ylméthyle (exo)
(3a*R*,5*s*,6a*S*)-5-(4-fluorophénoxy)hexahydrocyclopenta[*c*]pyrrole-2(1*H*)-carboxylate de (3-carbamoylisoxazol-5-yl)méthyle (exo)
(3a*R*,5*s*,6a*S*)-5-(4-chlorophénoxy)hexahydrocyclopenta[*c*]pyrrole-2(1*H*)-carboxylate de [3-(méthylcarbamoyl)isoxazol-5-yl]méthyle (exo)
(3a*R*,5*s*,6a*S*)-5-(4-chlorophénoxy)hexahydrocyclopenta[*c*]pyrrole-2(1*H*)-carboxylate de (3-carbamoylisoxazol-5-yl)méthyle (exo)
(3a*R*,5*s*,6a*S*)-5-(4-chlorophénoxy)hexahydrocyclopenta[*c*]pyrrole-2(1*H*)-carboxylate de (1-méthyl-1*H*-1,2,4-triazol-5-yl)méthyle (exo)
(3a*R*,5*s*,6a*S*)-5-(4-chlorophénoxy)hexahydrocyclopenta[*c*]pyrrole-2(1*H*)-carboxylate de (4-carbamoyl-thiazol-2-yl)méthyle (exo)
(3a*R*,5*s*,6a*S*)-5-(4-chlorophénoxy)hexahydrocyclopenta[*c*]pyrrole-2(1*H*)-carboxylate de [4-(méthylcarbamoyl)- thiazol-2-yl]méthyle (exo)
(3a*R*,5*s*,6a*S*)-5-[3-(trifluoroméhyl)phénoxylhexahydrocyclopenta[*c*]pyrrole-2(1*H*)-carboxylate de (3-carbamoylisoxazol-5-yl)méthyle (exo)
(3a*R*,5*s*,6a*S*)-5-[3-(trifluorométhyl)phénoxy]hexahydrocyclopenta[*c*]pyrrole-2(1*H*)-carboxylate de [3-(méthylcarbamoyl)isoxazol-5-yl]méthyle (exo)
(3a*R*,5*s*,6a*S*)-5-[(4'-éthoxybiphényl-3-yl)oxy]hexahydrocyclopenta[*c*]pyrrole-2(1*H*)-carboxylate de thiazol-2-ylméthyle (exo)
(3a*R*,5*s*,6a*S*)-5-(4-chlorophénoxy)hexahydrocyclopenta[*c*]pyrrole-2(1*H*)-carboxylate de (4-carbamoyl-oxazol-2-yl)méthyle (exo)
(3aR,4S,6aS)-4-[(6-méthoxynaphthalèn-2-yl)oxy]hexahydrocyclopenta[*c*]pyrrole-2(1*H*)-carboxylate de thiazol-4-ylméthyle (exo)
(3a*R*,4*R*,6a*S*)-4-[3-(trifluorométhyl)phenoxy]hexahydrocyclopenta[*c*]pyrrole-2(1*H*)-carboxylate de thiazol-4-ylméthyle (endo)
(3a*R*,5*s*,6a*S*)-5-[(7-éthoxynaphthalèn-2-yl)oxy]hexahydrocyclopenta[*c*]pyrrole-2(1*H*)-carboxylate de (4-carbamoyl-thiazol-2-yl)méthyle (exo)
(3a*R*,5*s*,6a*S*)-5-[(7-éthoxynaphthalèn-2-yl)oxy]hexahydrocyclopenta[*c*]pyrrole-2(1*H*)-carboxylate de [4-(méthylcarbamoyl)- thiazol-2-yl]méthyle (exo)
(3a*R*,5*s*,6a*S*)-5-[(7-éthoxynaphthalèn-2-yl)oxy]hexahydrocyclopenta[*c*]pyrrole-2(1*H*)-carboxylate de (3-carbamoylisoxazol-5-yl)méthyle (exo)
(3a*R*,5*s*,6a*S*)-5-[(7-éthoxynaphthalèn-2-yl)oxy]hexahydrocyclopenta[*c*]pyrrole-2(1*H*)-carboxylate de [3-(méthylcarbamoyl)isoxazol-5-yl]méthyle (exo)
(3a*R*,5*s*,6a*S*)-5-(4-chlorophénoxy)hexahydrocyclopenta[*c*]pyrrole-2(1*H*)-carboxylate de [4-(méthylcarbamoyl)-oxazol-2-yl]méthyle (exo)
(3a*R*,5*s*,6a*S*)-5-[(4-chloronaphthalèn-1-yl)oxy]hexahydrocyclopenta[*c*]pyrrole-2(1*H*)-carboxylate de (3-carbamoylisoxazol-5-yl)méthyle (exo)
(3a*R*,5*s*,6a*S*)-5-[(4-chloronaphthalèn-1-yl)oxy]hexahydrocyclopenta[*c*]pyrrole-2(1*H*)-carboxylate de [3-(méthylcarbamoyl)isoxazol-5-yl]méthyle (exo)
(3a*R*,4*S*,6a*S*)-4-[(4-chloronaphthalèn-1-yl)oxy]hexahydrocyclopenta[*c*]pyrrole-2(1*H*)-carboxylate de thiazol-4-ylméthyle (exo)
(3a*R*,4*R*,6a*S*)-4-[(4'-éthoxybiphényl-3-yl)oxy]hexahydrocyclopenta[*c*]pyrrole-2(1*H*)-carboxylate de thiazol-4-ylméthyle (endo)
(3a*R*,5*s*,6a*S*)-5-[(4'-fluorobiphényl-4-yl)oxy]hexahydrocyclopenta[*c*]pyrrole-2(1*H*)-carboxylate de (3-carbamoylisoxazol-5-yl)méthyle (exo)
(3a*R*,5*s*,6a*S*)-5-(4-chloro-2-fluorophénoxy)hexahydrocyclopenta[*c*]pyrrole-2(1*H*)-carboxylate de [3-(méthylcarbamoyl)isoxazol-5-yl]méthyle (exo)
(3a*R*,5*s*,6a*S*)-5-(4-chloro-2-fluorophénoxy)hexahydrocyclopenta[c]pyrrole-2(1*H*)-carboxylate de (3-carbamoylisoxazol-5-yl)méthyle (exo)
(3a*R*,5*s*,6a*S*)-5-(4-chloro-3-fluorophénoxy)hexahydrocyclopenta[*c*]pyrrole-2(1*H*)-carboxylate de (3-carbamoylisoxazol-5-yl)méthyle (exo)
(3a*R*,5*s*,6a*S*)-5-(4-chloro-3-fluorophénoxy)hexahydrocyclopenta[*c*]pyrrole-2(1*H*)-carboxylate de [3-(méthylcarbamoyl)isoxazol-5-yl]méthyle (exo)
(3a*R*,5*s*,6a*S*)-5-(2,4-dichlorophénoxy)hexahydrocyclopenta[*c*]pyrrole-2(1*H*)-carboxylate de (3-carbamoylisoxazol-5-yl)méthyle (exo)
(3a*R*,5*s*,6a*S*)-5-(2,4-dichlorophénoxy)hexahydrocyclopenta[*c*]pyrrole-2(1*H*)-carboxylate de [3-(méthylcarbamoyl)isoxazol-5-yl]méthyle (exo)
(3a*R*,5*s*,6a*S*)-5-(isoquinolin-7-yloxy)hexahydrocyclopenta[*c*]pyrrole-2(1*H*)-carboxylate de (3-carbamoylisoxazol-5-yl)méthyle (exo)
(3a*R*,5*s*,6a*S*)-5-(isoquinolin-6-yloxy)hexahydrocyclopenta[*c*]pyrrole-2(1*H*)-carboxylate de (3-carbamoylisoxazol-5-yl)méthyle (exo)
(3a*R*,5*r*,6a*S*)-5-(4-fluoro-1,3-benzothiazol-2-yl) -5-hydroxyhexahydrocyclopenta[*c*]pyrrole-2(1*H*)-carboxylate de (3-carbamoylisoxazol-5-yl)méthyle (exo)
(3a*R*,5*s*,6a*S*)-5-[(7-éthoxynaphthalèn-2-yl)oxy]hexahydrocyclopenta[*c*]pyrrole-2(1*H*)-carboxylate de (4-carbamoyl-oxazol-2-yl)méthyle (exo)
(3a*R*,5*s*,6a*S*)-5-(4-fluorophénoxy)hexahydrocyclopenta[*c*]pyrrole-2(1*H*)-carboxylate de (3-{[2-(diméthylamino)éthyl]carbamoyl}isoxazol-5-yl)méthyle (exo), et son chlorhydrate
(3a*R*,5*s*,6a*S*)-5-[(7-éthoxynaphthalèn-2-yl)oxy]hexahydrocyclopenta[*c*]pyrrole-2(1*H*)-carboxylate de [4-(méthylcarbamoyl)-oxazol-2-yl]méthyle (exo)
(3a*R*,5*s*,6a*S*)-5-(4-fluorophénoxy)hexahydrocyclopenta[*c*]pyrrole-2(1*H*)-carboxylate de (3-diméthylcarbamoylisoxazol-5-yl)méthyle (exo)
(3a*R*,5*s*,6a*S*)-5-[(4'-fluorobiphényl-4-yl)oxy]hexahydrocyclopenta[*c*]pyrrole-2(1*H*)-carboxylate de (3-méthylcarbamoylisoxazol-5-yl)méthyle (exo).

6. Procédé de préparation d'un composé de formule (I) selon l'une quelconque des revendications 1 à 5, comprenant l'étape consistant à faire réagir soit une amine de formule générale (II), 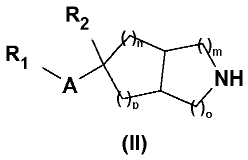 dans laquelle A, R₁, R₂, m, n, o et p sont tels que définis dans la formule générale (I) selon la revendication 1,
avec un carbonate de formule générale (III) 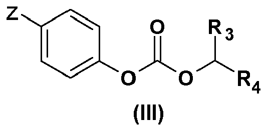 dans laquelle Z représente un atome d'hydrogène ou un groupe nitro, R₃ et R₄ sont tels que définis dans la formule générale (I) selon la revendication 1,
en présence d'une base, dans un solvant à une température comprise entre la température ambiante et la température de reflux du solvant.

7. Procédé de préparation d'un composé de formule (I) selon l'une quelconque des revendications 1 à 5, comprenant l'étape consistant à faire réagir un composé de formule générale (Ia) 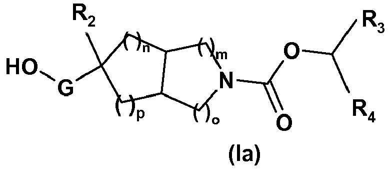 dans laquelle R₂, R₃, R₄, m, n, o et p sont tels que définis dans la formule générale (I) selon la revendication 1, et G représente une partie du groupe A tel que défini dans la formule générale (I), à savoir une liaison covalente;
avec soit un dérivé alcool de formule générale R₁OH (IV), dans laquelle R₁ est tel que défini dans la formule générale (I) selon la revendication 1, en utilisant les conditions de réaction de Mitsunobu;
soit avec un dérivé halogéné de formule générale R₁X (IVa), dans laquelle R₁ est tel que défini dans la formule générale (I) selon la revendication 1, et X représente un atome de fluor, de chlore, de brome ou d'iode en utilisant les réactions de substitution nucléophiles aromatiques, hétéroaromatiques ou de O-arylation, O-hétéroarylation de Buchwald.

8. Procédé de préparation d'un composé de formule (I) selon l'une quelconque des revendications 1 à 5, dans laquelle R₁ représente un groupe R₅ substitué notamment par un groupe R₆ de type C₁₋₆-alkyle, C₃₋₇-cycloalkyle ou C₃₋₇-cycloalkyle-C₁₋₃-alkylène, ou par un groupe R₇ tel que défini dans la formule générale (I) selon la revendication 1, comprenant l'étape consistant à réaliser une réaction de couplage, catalysée au moyen d'un métal de transition, sur le composé de formule générale (Ib), 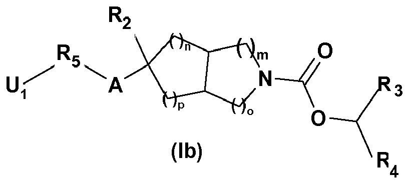 dans laquelle A, R₂, R₃, R₄, R₅, m, n, o et p sont tels que définis dans la formule générale (I) selon la revendication 1 et U₁ représente un atome de chlore, de brome, d'iode ou un groupement triflate, U₁ étant dans la position où l'on souhaite introduire le groupe R₆ ou R₇:
- soit par une réaction de type Suzuki, par exemple au moyen d'un acide boronique d'alkyle, de cycloalkyle, d'aryle ou d'hétéroaryle,
- soit selon une réaction de type Stille, par exemple en utilisant un dérivé *tri*-alkylstanneux d'aryle ou d'hétéroaryle
- soit par une réaction de type Negishi, par exemple en utilisant un dérivé zincate d'halogénure d'alkyle, de cycloalkyle, d'aryle ou d'hétéroaryle.

9. Composé de formule générale (Ia) 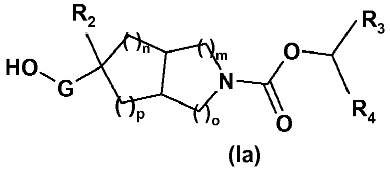 dans laquelle R₂, R₃, R₄, m, n, o et p sont tels que définis dans la revendication 1, et G représente une partie du groupe A tel que défini dans la formule générale (I), à savoir une liaison covalente.

10. Composé de formule générale (II), 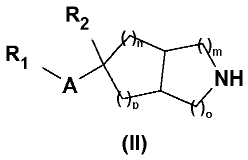 dans laquelle R₁, R₂, m, n, o et p sont tels que définis dans la revendication 1, A représente un atome d'oxygène ou une liaison covalente, étant donné que lorsque A représente une liaison covalente alors R1 représente un groupe benzothiazolyle.

11. Composés de formule générale (IIe) : 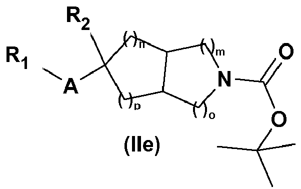 dans laquelle R₁, R₂, m, n, o et p sont tels que définis dans la revendication 1, A représente un atome d'oxygène ou une liaison covalente, étant donné que lorsque A représente une liaison covalente alors R1 représente un groupe benzothiazolyle.

12. Composé de formule (I) selon l'une quelconque des revendications 1 à 5, à l'état de base ou de sel d'addition à un acide pharmaceutiquement acceptable, pour son utilisation comme médicament.

13. Composition pharmaceutique contenant au moins un composé de formule (I) selon l'une quelconque des revendications 1 à 5, à l'état de base ou de sel d'addition à un acide pharmaceutiquement acceptable et éventuellement un ou plusieurs excipients pharmaceutiquement acceptables.

14. Utilisation d'un composé de formule (I) selon l'une quelconque des revendications 1 à 5, à l'état de base ou de sel d'addition à un acide pharmaceutiquement acceptable, pour la préparation d'un médicament destiné à prévenir ou à traiter une pathologie dans laquelle les cannabinoïdes endogènes et/ou tous autres substrats métabolisés par l'enzyme FAAH, sont impliqués.

15. Utilisation d'un composé de formule (I) selon l'une quelconque des revendications 1 à 5, à l'état de base ou de sel d'addition à un acide pharmaceutiquement acceptable, pour la préparation d'un médicament destiné à prévenir ou à traiter les douleurs aiguës ou chroniques de type neurogène, les douleurs aiguës ou chroniques associées aux maladies inflammatoires, les douleurs aiguës ou chroniques périphériques, les vertiges, les vomissements, les nausées, les troubles du comportement alimentaire, les pathologies neurologiques et psychiatriques, les maladies neuro-dégénératives aiguës ou chroniques, l'épilepsie, les troubles du sommeil, les maladies cardiovasculaires, l'ischémie rénale, les cancers, les désordres du système immunitaire, les maladies allergiques, les maladies infectieuses parasitaires , virales ou bactériennes, les maladies inflammatoires, l'ostéoporose, les affections oculaires, les affections pulmonaires, les maladies gastro-intestinales, l'incontinence urinaire ou l'inflammation vésicale.

## Patentansprüche

1. Verbindung mit der Formel (I) 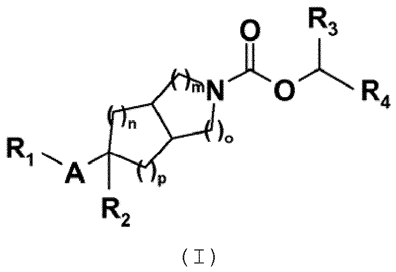 worin
R₂ für ein Wasserstoffatom, Fluoratom oder eine Hydroxyl-, Cyano-, Trifluormethyl-, C₁₋₆-Alkyl-, C₁₋₆-Alkoxy-, NR₈R₉-Gruppe steht;
m, n, o und p den Wert 1 aufweisen oder m und o den Wert 1 aufweisen, n den Wert 0 aufweist und p den Wert 2 aufweist; A für eine kovalente Bindung oder ein Sauerstoffatom steht;
R₁ für eine Gruppe R₅, gegebenenfalls substituiert mit einer oder mehreren Gruppen R₆ und/oder R₇, steht;
R₅ für eine Gruppe, ausgewählt aus einem Phenyl, Pyridinyl, Pyridazinyl, Pyrimidinyl, Pyrazinyl, Triazinyl, Naphthalinyl, Chinolinyl, Isochinolinyl, Phtalazinyl, Chinazolinyl, Chinoxalinyl, Cinnolinyl, Naphthyridinyl, Benzothiazolyl, Benzoxazolyl, Benzimidazolyl, Benzisothiazolyl, Bensisoxazolyl, Indazolyl, Benzotriazolyl, steht;
R₆ für ein Halogenatom, eine Cyanogruppe, -CH₂CN, Nitro, Hydroxyl, C₁₋₆-Alkyl, C₁₋₆-Alkoxy, C₁₋₆-Thioalkyl, C₁₋₆-Haloalkyl, C₁₋₆-Haloalkoxy, C₁₋₆-Halothioalkyl, C₃₋₇-Cycloalkyl, C₃₋₇-Cycloalkyl-C₁₋₃-alkylen, C₃₋₇-Cycloalkyl-C₁₋₃-alkylen-O-, NR₈R₉, NR₈COR₉, NR₈CO₂R₉, NR₈SO₂R₉, NR₈SO₂NR₈R₉, COR₈, CO₂R₈, CONR₈R₉, SO₂R₈, SO₂NR₈R₉ oder -O-(C₁₋₃-Alkylen)-O-steht;
R₇ für eine Gruppe, ausgewählt aus einem Phenyl, Pyridinyl, Pyridazinyl, Pyrimidinyl, Pyrazinyl, Triazinyl, steht; wobei die Gruppe(n) R₇ mit einer oder mehreren Gruppen R₆, gleich oder verschieden voneinander, substituiert sein können; wobei die Gruppe(n) R₇ mit einer oder mehreren Gruppen R₆, gleich oder verschieden voneinander, substituiert sein können;
R₃ für ein Wasserstoffatom, Fluoratom, eine C₁₋₆-Alkylgruppe oder eine Trifluormethylgruppe steht;
R₄ für eine Gruppe, ausgewählt aus einem Thiazolyl, einem Thiadiazolyl, einem Triazolyl, einem Oxazolyl, einem Isoxazolyl, steht; wobei diese Gruppe nicht substituiert oder mit einer oder mehreren C₁₋₆-Alkyl-, CONR₈R₉- oder CON(R₈) (C₁₋₃-Alkyl-NR₁₀R₁₁)-Gruppe(n) substituiert ist;
R₈ R₉, R₁₀ und R₁₁ unabhängig voneinander für ein Wasserstoffatom oder eine C₁₋₆-Alkylgruppe stehen; in Form einer Base oder eines Säureadditionssalzes.

2. Verbindung mit der Formel (I) nach Anspruch 1, **dadurch gekennzeichnet, dass** R₂ für ein Wasserstoffatom oder eine Hydroxylgruppe steht,
in Form einer Base oder eines Säureadditionssalzes.

3. Verbindung mit der Formel (I) nach Anspruch 1 oder 2, **dadurch gekennzeichnet, dass** R₁ für eine Gruppe R₅ steht, die nicht substituiert oder mit einer oder mehreren Gruppen R₆ und/oder R₇ substituiert ist;
R₅ für eine Phenyl-, Naphthalinyl-, Benzothiazolyl- oder Isochinolinylgruppe steht;
R₆ für ein Halogenatom oder eine C₁₋₆-Haloalkylgruppe oder eine C₁₋₆-Alkoxygruppe steht;
R₇ für ein Phenyl steht, das mit einer oder mehreren Gruppen R₆, gleich oder verschieden voneinander, substituiert sein kann, in Form einer Base oder eines Säureadditionssalzes.

4. Verbindung mit der Formel (I) nach einem der Ansprüche 1 bis 3, **dadurch gekennzeichnet, dass** R₃ für ein Wasserstoffatom steht, in Form einer Base oder eines Säureadditionssalzes.

5. Verbindung mit der Formel (I), ausgewählt aus:
Thiazol-2-ylmethyl(3aR,5s,6aS)-5-(4-chlorphenoxy)hexahydrocyclopenta[c]pyrrol-2(1H)-carboxylat (exo)
Thiazol-2-ylmethyl(3aR,5s,6aS)-5-[4-(trifluormethyl)phenoxy]hexahydrocyclopenta[*c*] pyrrol-2(1*H*)-carboxylat (exo)
Thiazol-2-ylmethyl(3a*R*,5*s*,6a*S*)-5-[(7-ethoxynaphthalin-2-yl)oxylhexahydrocyclopenta[*c*]pyrrol-2(1*H*)-carboxylat (exo)
Thiazol-2-ylmethyl(3a*R*,5*r*,6a*S*)-5-[3-(trifluormethyl)phenoxy]hexahydrocyclopenta[*c*] pyrrol-2(1*H*)-carboxylat (endo)
Thiazol-2-ylmethyl(3a*R*,5*s*,6a*S*)-5-[3-(trifluormethyl)phenoxy]hexahydrocyclopenta[*c*] pyrrol-2(1*H*)-carboxylat (exo)
Thiazol-2-ylmethyl(3a*R*,5*r*,6a*S*)-5-[(4'-ethoxybiphenyl-3-yl)oxy]hexahydrocyclopenta[*c*]pyrrol-2(1*H*)-carboxylat (endo)
1,2,3-Thiadiazol-4-ylmethyl(3a*R*,5*s*,6a*S*)-5-(4-chlorphenoxy)hexahydrocyclopenta[*c*]pyrrol-2(1*H*)-carboxylat (exo)
(5-*Tert*-butyl-1,3,4-thiadiazol-2-yl)methyl(3a*R*,5*s*,6a*S*)-5-(4-chlorphenoxy)hexahydrocyclopenta[*c*]pyrrol-2(1*H*)-carboxylat (exo)
1,2,3-Thiadiazol-4-ylmethyl(3a*R*,5*s*,6a*S*)-5-(4-fluorphenoxy)hexahydrocyclopenta[*c*]pyrrol-2(1*H*)-carboxylat (exo)
(3-Carbamoylisoxazol-5-yl)methyl(3a*R*,5*s*,6a*S*)-5-(4-fluorphenoxy)hexahydrocyclopenta[*c*]pyrrol-2(1*H*)-carboxylat (exo)
[3-((Methylcarbamoyl)isoxazol-5-yl]methyl(3a*R*,5*s*,6a*S*)-5-(4-chlorphenoxy)hexahydrocyclopenta[*c*]pyrrol-2(1*H*)-carboxylat (exo)
(3-Carbamoylisoxazol-5-yl)methyl(3a*R*,5*s*,6a*S*)-5-(4-chlorphenoxy)hexahydrocyclopenta[*c*]pyrrol-2(1*H*)-carboxylat (exo)
(1-Methyl-1*H*-1,2,4-triazol-5-yl)methyl(3a*R*,5*s*,6a*S*)-5-(4-chlorphenoxy)hexahydrocyclopenta[*c*]pyrrol-2(1*H*)-carboxylat (exo)
(4-Carbamoyl-thiazol-2-yl)methyl(3a*R*,5*s*,6a*S*)-5-(4-chlorphenoxy)hexahydrocyclopenta[*c*]pyrrol-2(1*H*)-carboxylat (exo)
[4-((Methylcarbamoyl)-thiazol-2-yl]methyl(3a*R*,5*s*,6a*S*)-5-(4-chlorphenoxy)hexahydrocyclopenta[*c*]pyrrol-2(1*H*)-carboxylat (exo)
(3-Carbamoylisoxazol-5-yl)methyl(3a*R*,5*s*,6a*S*)-5-[3-(trifluormethyl)phenoxy]hexahydrocyclopenta[*c*] pyrrol-2(1*H*)-carboxylat (exo)
[3-((Methylcarbamoyl)isoxazol-5-yl]methyl(3a*R*,5*s*,6a*S*)-5-[3-(trifluormethyl)phenoxy]hexahydrocyclopenta[*c*] pyrrol-2(1*H*)-carboxylat (exo)
Thiazol-2-ylmethyl(3a*R*,5*s*,6a*S*)-5-[(4'-ethoxybiphenyl-3-yl)oxy]hexahydrocyclopenta[*c*]pyrrol-2(1*H*)-carboxylat (exo)
(4-Carbamoyl-oxazol-2-yl)methyl(3a*R*,5*s*,6a*S*)-5-(4-chlorphenoxy)hexahydrocyclopenta[*c*]pyrrol-2(1*H*)-carboxylat (exo)
Thiazol-4-ylmethyl(3a*R*,4*S*,6a*S*)-4-[(6-methoxynaphthalin-2-yl)oxy]hexahydrocyclopenta[*c*]pyrrol-2(1*H*)-carboxylat (exo)
Thiazol-4-ylmethyl(3a*R*,4*R*,6a*S*)-4-[3-(trifluormethyl)phenoxy]hexahydrocyclopenta[*c*] pyrrol-2(1*H*)-carboxylat (endo)
(4-Carbamoyl-thiazol-2-yl)methyl(3a*R*,5*s*,6a*S*)-5-[(7-ethoxynaphthalin-2-yl)oxy]hexahydrocyclopenta[*c*]pyrrol-2(1*H*)-carboxylat (exo)
[4-((Methylcarbamoyl)-thiazol-2-yl]methyl(3a*R*,5*s*,6a*S*)-5-[(7-ethoxynaphthalin-2-yl)oxy]hexahydrocyclopenta[*c*]pyrrol-2(1*H*)-carboxylat (exo)
(3-Carbamoylisoxazol-5-yl)methyl(3a*R*,5*s*,6a*S*)-5-[(7-ethoxynaphthalin-2-yl)oxy]hexahydrocyclopenta[*c*]pyrrol-2(1*H*)-carboxylat (exo)
[3-((Methylcarbamoyl)isoxazol-5-yl]methyl(3a*R*,5*s*,6a*S*)-5-[(7-ethoxynaphthalin-2-yl)oxylhexahydrocyclopenta[*c*]pyrrol-2(1*H*)-carboxylat (exo)
[4-((Methylcarbamoyl)-oxazol-2-yl]methyl(3a*R*,5*s*,6a*S*)-5-(4-chlorphenoxy)hexahydrocyclopenta[*c*]pyrrol-2(1*H*)-carboxylat (exo)
(3-Carbamoylisoxazol-5-yl)methyl(3a*R*,5*s*,6a*S*)-5-[(4-chlornaphthalin-1-yl)oxy]hexahydrocyclopenta[*c*]pyrrol-2(1*H*)-carboxylat (exo)
[3-((Methylcarbamoyl)isoxazol-5-yl]methyl(3a*R*,5*s*,6a*S*)-5-[(4-chlornaphthalin-1-yl)oxy]hexahydrocyclopenta[*c*]pyrrol-2(1*H*)-carboxylat (exo)
Thiazol-4-ylmethyl(3a*R*,4*S*,6a*S*)-4-[(4-chlornaphthalin-1-yl)oxy]hexahydrocyclopenta[*c*]pyrrol-2(1*H*)-carboxylat (exo)
Thiazol-4-ylmethyl(3a*R*,4*R*,6a*S*)-4-[(4'-ethoxybiphenyl-3-yl)oxy]hexahydrocyclopenta[*c*]pyrrol-2(1*H*)-carboxylat (endo)
(3-Carbamoylisoxazol-5-yl)methyl(3a*R*,5*s*,6a*S*)-5-[(4'-fluorbiphenyl-4-yl)oxy]hexahydrocyclopenta[*c*]pyrrol-2(1*H*)-carboxylat (exo)
[3-((Methylcarbamoyl)isoxazol-5-yl]methyl(3a*R*,5*s*,6a*S*)-5-(4-chlor-2-fluorphenoxy)hexahydrocyclopenta[*c*]pyrrol-2(1*H*)-carboxylat (exo)
(3-Carbamoylisoxazol-5-yl)methyl(3a*R*,5*s*,6a*S*)-5-(4-chlor-2-fluorphenoxy)hexahydrocyclopenta[*c*]pyrrol-2(1*H*)-carboxylat (exo)
(3-Carbamoylisoxazol-5-yl)methyl(3a*R*,5*s*,6a*S*)-5-(4-chlor-3-fluorphenoxy)hexahydrocyclopenta[*c*]pyrrol-2(1*H*)-carboxylat (exo)
[3-((Methylcarbamoyl)isoxazol-5-yl]methyl(3a*R*,5*s*,6a*S*)-5-(4-chlor-3-fluorphenoxy)hexahydrocyclopenta[c]pyrrol-2(1H)-carboxylat (exo)
(3-Carbamoylisoxazol-5-yl)methyl(3aR,5s,6aS)-5-(2,4-dichlorphenoxy)hexahydrocyclopenta[*c*]pyrrol-2(1*H*)-carboxylat (exo)
[3-((Methylcarbamoyl)isoxazol-5-yl]methyl(3a*R*,5*s*,6a*S*)-5-(2,4-dichlorphenoxy)hexahydrocyclopenta[*c*]pyrrol-2(1*H*)-carboxylat (exo)
(3-Carbamoylisoxazol-5-yl)methyl(3a*R*,5*s*,6a*S*)-5-(isochinolin-7-yloxy)hexahydrocyclopenta[*c*]pyrrol-2(1*H*)-carboxylat (exo)
(3-Carbamoylisoxazol-5-yl)methyl(3a*R*,5*s*,6a*S*)-5-(isochinolin-6-yloxy)hexahydrocyclopenta[*c*]pyrrol-2(1*H*)-carboxylat (exo)
(3-Carbamoylisoxazol-5-yl)methyl(3a*R*,5*r*,6a*S*)-5-(4-fluor-1,3-benzothiazol-2-yl)-5-hydroxyhexahydrocyclopenta[*c*]pyrrol-2(1*H*)-carboxylat (exo)
(4-Carbamoyl-oxazol-2-yl)methyl(3a*R*,5*s*,6a*S*)-5-[(7-ethoxynaphthalin-2-yl)oxy]hexahydrocyclopenta[*c*]pyrrol-2(1*H*)-carboxylat (exo)
(3-{[2-(Dimethylamino)ethyl]carbamoyl}isoxazol-5-yl)methyl(3a*R*,5*s*,6a*S*)-5-(4-fluorphenoxy)hexahydrocyclopenta[*c*]pyrrol-2(1*H*)-carboxylat (exo) und sein Hydrochlorid [4-(Methylcarbamoyl)-oxazol-2-yl]methyl(3a*R*,5*s*,6a*S*)-5-[(7-ethoxynaphthalin-2-yl)oxy]hexahydrocyclopenta[*c*]pyrrol-2(1*H*)-carboxylat (exo)
(3-Dimethylcarbamoylisoxazol-5-yl)methyl(3a*R*,5*s*,6a*S*)-5-(4-fluorphenoxy)hexahydrocyclopenta[*c*]pyrrol-2(1*H*)-carboxylat (exo)
(3-Methylcarbamoylisoxazol-5-yl)methyl(3a*R*,5*s*,6a*S*)-5-[(4'-fluorbiphenyl-4-yl)oxy]hexahydrocyclopenta[*c*]pyrrol-2(1*H*)-carboxylat (exo).

6. Verfahren zur Herstellung einer Verbindung mit der Formel (I) nach einem der Ansprüche 1 bis 5, umfassend den Schritt zum Umsetzen entweder eines Amins mit der allgemeinen Formel (II), 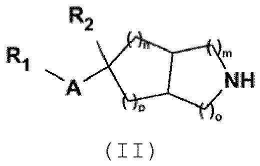 worin A, R₁, R₂, m, n, o und p sind, wie in der allgemeinen Formel (I) nach Anspruch 1 definiert, mit einem Carbonat der allgemeinen Formel (III): 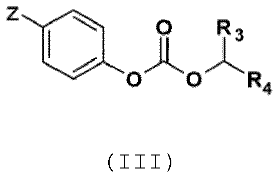 worin Z für ein Wasserstoffatom oder eine Nitrogruppe steht, R₃ und R₄ sind, wie in der allgemeinen Formel (I) nach Anspruch 1 definiert,
in Gegenwart einer Base, in einem Lösemittel bei einer Temperatur im Bereich zwischen der Umgebungstemperatur und der Rückflusstemperatur des Lösemittels.

7. Verfahren zur Herstellung einer Verbindung mit der Formel (I) nach einem der Ansprüche 1 bis 5, umfassend den Schritt zum Umsetzen einer Verbindung mit der allgemeinen Formel (Ia), 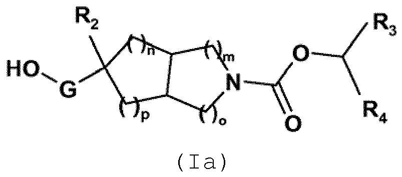 worin R₂, R₃, R₄, m, n, o und p sind, wie in der allgemeinen Formel (I) nach Anspruch 1 definiert, und G für einen Teil der Gruppe A steht, wie in der allgemeinen Formel (I) definiert, d. h. eine kovalente Bindung;
entweder mit einem Alkoholderivat mit der allgemeinen Formel R₁OH (IV), worin R₁ ist, wie in der allgemeinen Formel (I) nach Anspruch 1 definiert, unter Verwendung der Bedingungen der Mitsunobu-Reaktion;
oder mit einem Halogenderivat mit der allgemeinen Formel R₁X (IVa), worin R₁ ist, wie in der allgemeinen Formel (I) nach Anspruch 1 definiert, und X für ein Fluor-, Chlor-, Brom - oder Iodatom steht, unter Verwendung der Buchwald-Reaktionen zur nukleophilen aromatischen, heteroaromatischen Substitution oder O-Arylierung, *O-*Heteroarylierung.

8. Verfahren zur Herstellung einer Verbindung mit der Formel (I) nach einem der Ansprüche 1 bis 5, worin R₁ für eine Gruppe R₅ steht, die insbesondere mit einer Gruppe R₆ vom Typ C₁₋₆-Alkyl, C₃₋₇-Cycloalkyl oder C₃₋₇-Cycloalkyl-C₁₋₃-alkylen, oder mit einer Gruppe R₇, wie in der allgemeinen Formel (I) nach Anspruch 1 definiert, substituiert ist, umfassend den Schritt zur Durchführung einer Kopplungsreaktion, die mithilfe eines Übergangsmetalls katalysiert wird, an der Verbindung mit der allgemeinen Formel (Ib), 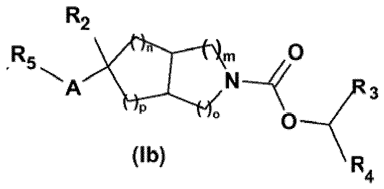 worin A, R₂, R₃, R₄, R₅, m, n, o und p sind, wie in der allgemeinen Formel (I) nach Anspruch 1 definiert, und U₁ für ein Chlor-, Brom-, Iodatom oder eine Triflat-Gruppe steht, wobei U₁ in der Position steht, an der die Gruppe R₆ oder R₇ eingeführt werden soll:
- entweder durch eine Reaktion vom Typ Suzuki, zum Beispiel mithilfe einer Alkyl-, Cycloalkyl-, Aryl- oder Heteroarylboronsäure,
- oder gemäß einer Reaktion vom Typ Stille, zum Beispiel unter Verwendung eines Aryl- oder Heteroaryl-tri-Zinntetraalkylderivats,
- oder durch eine Reaktion vom Typ Negishi, zum Beispiel unter Verwendung eines Alkyl-, Cycloalkyl-, Aryl- oder Heteroarylhalogenidzinkatderivats,

9. Verbindung mit der allgemeinen Formel (Ia): 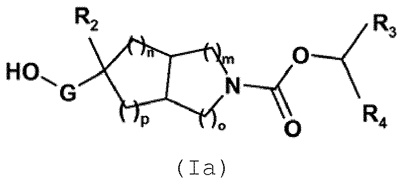 worin R₂, R₃, R₄, m, n, o und p sind, wie in Anspruch 1 definiert, und G für einen Teil der Gruppe A steht, wie in der allgemeinen Formel (I) definiert, d. h. eine kovalente Bindung.

10. Verbindung mit der allgemeinen Formel (II): 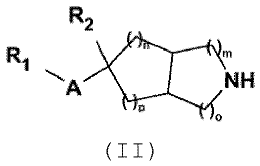 worin R₁, R₂, m, n, o und p sind, wie in Anspruch 1 definiert, A für ein Sauerstoffatom oder eine kovalente Bindung steht, angesichts dessen, dass wenn A für eine kovalente Bindung steht, R₁ für eine Benzothiazolylgruppe steht.

11. Verbindungen mit der allgemeinen Formel (IIe): 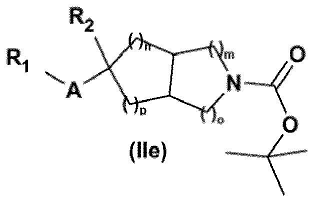 worin R₁, R₂, m, n, o und p sind, wie in Anspruch 1 definiert, A für ein Sauerstoffatom oder eine kovalente Bindung steht, angesichts dessen, dass wenn A für eine kovalente Bindung steht, R₁ für eine Benzothiazolylgruppe steht.

12. Verbindung mit der Formel (I) nach einem der Ansprüche 1 bis 5 in Form einer Base oder eines Additionssalzes einer pharmazeutisch verträglichen Säure zur Verwendung als Medikament.

13. Pharmazeutische Zusammensetzung, enthaltend mindestens eine Verbindung mit der Formel (I) nach einem der Ansprüche 1 bis 5 in Form einer Base oder eines Additionssalzes einer pharmazeutisch verträglichen Säure und gegebenenfalls einem oder mehreren pharmazeutisch verträglichen Exzipienten.

14. Verwendung einer Verbindung mit der Formel (I) nach einem der Ansprüche 1 bis 5 in Form einer Base oder eines Additionssalzes einer pharmazeutisch verträglichen Säure zur Herstellung eines Medikaments, das dazu bestimmt ist, eine Erkrankung zu verhindern oder zu behandeln, an der die endogenen Cannabinoide und/oder alle anderen Substrate, die durch das Enzym FAAH verstoffwechselt werden, beteiligt sind.

15. Verwendung einer Verbindung mit der Formel (I) nach einem der Ansprüche 1 bis 5 in Form einer Base oder eines Additionssalzes einer pharmazeutisch verträglichen Säure zur Herstellung eines Medikaments, das dazu bestimmt ist, chronische oder akute neurogene Schmerzen, akute oder chronische Schmerzen, die mit Entzündungskrankheiten verbunden sind, akute oder chronische periphere Schmerzen, Schwindelzustände, Erbrechen, Übelkeiten, Störungen des Essverhaltens, neurologische und psychiatrische Erkrankungen, akute oder chronische neurodegenerative Krankheiten, Epilepsie, Schlafstörungen, Herz-Kreislauf-Krankheiten, Nierenischämie, Krebsformen, Störungen des Immunsystems, allergische Krankheiten, parasitäre, virale oder bakterielle Infektionskrankheiten, Entzündungskrankheiten, Osteoporose, Augenleiden, Lungenleiden, Magen-Darm-Krankheiten, Harninkontinenz oder Blasenentzündung zu verhindern oder zu behandeln.

## Claims

1. Compound corresponding to formula (I) 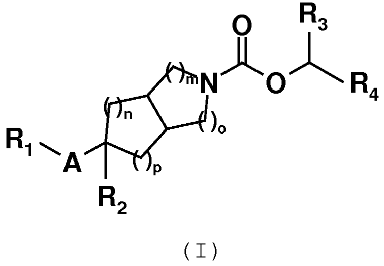 in which
R₂ represents a hydrogen or fluorine atom or a hydroxyl, cyano, trifluoromethyl, C₁₋₆-alkyl, C₁₋₆-alkoxy or NR₈R₉ group;
m, n, o and p have the value 1, or alternatively m and o have the value 1, n has the value 0 and p has the value 2;
A represents a covalent bond or an oxygen atom;
R₁ represents a group R₅ optionally substituted with one or more groups R₆ and/or R₇;
R₅ represents a group chosen from phenyl, pyridyl, pyridazinyl, pyrimidinyl, pyrazinyl, triazinyl, naphthyl, quinolyl, isoquinolyl, phthalazinyl, quinazolinyl, quinoxalinyl, cinnolinyl, naphthyridinyl, benzothiazolyl, benzoxazolyl, benzimidazolyl, benzisothiazolyl, bensisoxazolyl, indazolyl and benzotriazolyl;
R₆ represents a halogen atom or a cyano, -CH₂CN, nitro, hydroxyl, C₁₋₆-alkyl, C₁₋₆-alkoxy, C₁₋₆-thioalkyl, C₁₋₆-haloalkyl, C₁₋₆-haloalkoxy, C₁₋₆-halothioalkyl, C₃₋₇-cycloalkyl, C₃₋₇-cycloalkyl-C₁₋₃-alkylene, C₃₋₇-cycloalkyl-C₁₋₃-alkylene-O-, NR₈R₉, NR₈COR₉, NR₈CO₂R₉, NR₈SO₂R₉, NR₈SO₂NR₈R₉, COR₂, CO₂R₈, CONR₂R₉, SO₂R₈, SO₂NR₈R₉ or -O-(C₁₋₃-alkylene)-O- group;
R₇ represents a group chosen from phenyl, pyridyl, pyridazinyl, pyrimidinyl, pyrazinyl and triazinyl; the group(s) R₇ possibly being substituted with one or more groups R₆, which may be identical or different; the group(s) R₇ possibly being substituted with one or more groups R₆, which may be identical or different;
R₃ represents a hydrogen or fluorine atom, a group C₁₋₆-alkyl or a trifluoromethyl group;
R₄ represents a group chosen from a thiazolyl, a thiadiazolyl, a triazolyl, an oxazolyl and an isoxazolyl;
this group being unsubstituted or substituted with one or more groups C₁₋₆-alkyl, CONR₈R₉ or CON(R₈) (C₁₋₃-alkylene-NR₁₀R₁₁) ;
R₈, R₉, R₁₀ and R₁₁ represent, independently of each other, a hydrogen atom or a group C₁₋₆-alkyl;
in the form of the base or of an acid-addition salt.

2. Compound of formula (I) according to Claim 1, **characterized in that** R₂ represents a hydrogen atom or a hydroxyl group;
in the form of the base or of an acid-addition salt.

3. Compound of formula (I) according to Claim 1 or 2, **characterized in that** R₁ represents a group R₅ that is unsubstituted or substituted with one or more groups R₆ and/or R₇;
R₅ represents a phenyl, naphthyl, benzothiazolyl or isoquinolyl group;
R₆ represents a halogen atom or a group C₁₋₆-haloalkyl or a group C₁₋₆-alkoxy;
R₇ represents a phenyl that may be substituted with one or more groups R₆, which may be identical or different;
in the form of the base or of an acid-addition salt.

4. Compound of formula (I) according to any one of Claims 1 to 3, **characterized in that** R₃ represents a hydrogen atom; in the form of the base or of an acid-addition salt.

5. Compound of formula (I) chosen from:
thiazol-2-ylmethyl (3a*R*,5*s*,6a*S*)-5-(4-chlorophenoxy)-hexahydrocyclopenta[*c*]pyrrole-2(1*H*)-carboxylate (exo)
thiazol-2-ylmethyl (3a*R*,5*s*,6a*S*)-5-[4-(trifluoromethyl)-phenoxy]hexahydrocyclopenta[*c*]pyrrole-2(1*H*)-carboxylate (exo)
thiazol-2-ylmethyl (3a*R*,5*s*,6a*S*)-5-[(7-ethoxy-2-naphthyl)oxy]hexahydrocyclopenta[*c*]pyrrole-2(1*H*)-carboxylate (exo)
thiazol-2-ylmethyl (3a*R*,5*r*,6a*S*)-5-[3-(trifluoromethyl)-phenoxy]hexahydrocyclopenta[*c*]pyrrole-2(1*H*)-carboxylate (endo)
thiazol-2-ylmethyl (3a*R*,5*s*,6a*S*)-5-[3-(trifluoromethyl)-phenoxy]hexahydrocyclopenta[*c*]pyrrole-2(1*H*)-carboxylate (exo)
thiazol-2-ylmethyl (3a*R*,5*r*,6a*S*)-5-[(4'-ethoxybiphenyl-3-yl)oxy]hexahydrocyclopenta[*c*]pyrrole-2(1*H*)-carboxylate (endo)
1,2,3-thiadiazol-4-ylmethyl (3a*R*,5*s*,6a*S*)-5-(4-chloro-phenoxy)hexahydrocyclopenta[*c*]pyrrole-2(1*H*)-carboxylate (exo)
(5-*tert*-butyl-1,3,4-thiadiazol-2-yl)methyl (3a*R*,5*s*,6a*S*)-5-(4-chlorophenoxy)hexahydrocyclopenta[*c*]pyrrole-2(1*H*)-carboxylate (exo)
1,2,3-thiadiazol-4-ylmethyl (3a*R*,5*s*,6a*S*)-5-(4-fluoro-phenoxy)hexahydrocyclopenta[*c*]pyrrole-2(1*H*)-carboxylate (exo)
(3-carbamoylisoxazol-5-yl)methyl (3a*R*,5*s*,6a*S*)-5-(4-fluorophenoxy)hexahydrocyclopenta[*c*]pyrrole-2(1*H*)-carboxylate (exo)
[3-((methylcarbamoyl)isoxazol-5-yl]methyl (3a*R*,5*s*,6a*S*)-5-(4-chlorophenoxy)hexahydrocyclopenta[*c*]pyrrole-2(1*H*)-carboxylate (exo)
(3-carbamoylisoxazol-5-yl)methyl (3a*R*,5*s*,6a*S*)-5-(4-chlorophenoxy)hexahydrocyclopenta[*c*]pyrrole-2(1*H*)-carboxylate (exo)
(1-methyl-1*H*-1,2,4-triazol-5-yl)methyl (3a*R*,5*s*,6a*S*)-5-(4-chlorophenoxy)hexahydrocyclopenta[*c*]pyrrole-2(1*H*)-carboxylate (exo)
(4-carbamoylthiazol-2-yl)methyl (3a*R*,5*s*,6a*S*)-5-(4-chlorophenoxy)hexahydrocyclopenta[*c*]pyrrole-2(1*H*)-carboxylate (exo)
[4-((methylcarbamoyl)thiazol-2-yl]methyl (3aR,5s,6aS)-5-(4-chlorophenoxy)hexahydrocyclopenta[*c*]pyrrole-2(1*H*)-carboxylate (exo)
(3-carbamoylisoxazol-5-yl)methyl (3a*R*,5*s*,6a*S*)-5-[3-(trifluoromethyl)phenoxy]hexahydrocyclopenta[*c*]pyrrole-2(1*H*)-carboxylate (exo)
[3-((methylcarbamoyl)isoxazol-5-yl]methyl (3a*R*,5*s*,6a*S*)-5-[3-(trifluoromethyl)phenoxy]hexahydrocyclopenta[*c*]pyrrole-2(1*H*)-carboxylate (exo)
thiazol-2-ylmethyl (3a*R*,5*s*,6a*S*)-5-[(4'-ethoxybiphenyl-3-yl)oxy]hexahydrocyclopenta[*c*]pyrrole-2(1*H*)-carboxylate (exo)
(4-carbamoyloxazol-2-yl)methyl (3a*R*,5*s*,6a*S*)-5-(4-chlorophenoxy)hexahydrocyclopenta[*c*]pyrrole-2(1*H*)-carboxylate (exo)
thiazol-4-ylmethyl (3a*R*,4*S*,6a*S*)-4-[(6-methoxy-2-naphthyl)oxy]hexahydrocyclopenta[*c*]pyrrole-2(1*H*)-carboxylate (exo)
thiazol-4-ylmethyl (3a*R*,4*R*,6a*S*)-4-[3-(trifluoromethyl)-phenoxy]hexahydrocyclopenta[*c*]pyrrole-2(1*H*)-carboxylate (endo)
(4-carbamoylthiazol-2-yl)methyl (3a*R*,5*s*,6a*S*)-5-[(7-ethoxy-2-naphthyl)oxy]hexahydrocyclopenta[*c*]pyrrole-2(1*H*)-carboxylate (exo)
[4-((methylcarbamoyl)thiazol-2-yl]methyl (3a*R*,5*s*,6a*S*)-5-[(7-ethoxy-2-naphthyl)oxy]hexa-hydrocyclopenta[*c*]pyrrole-2(1*H*)-carboxylate (exo)
(3-carbamoylisoxazol-5-yl)methyl (3a*R*,5*s*,6a*S*)-5-[(7-ethoxy-2-naphthyl)oxy]hexahydrocyclopenta[*c*]pyrrole-2(1*H*)-carboxylate (exo)
[3-((methylcarbamoyl)isoxazol-5-yl]methyl (3a*R*,5*s*,6a*S*)-5-[(7-ethoxy-2-naphthyl)oxy]hexa-hydrocyclopenta[*c*]pyrrole-2(1*H*)-carboxylate (exo)
[4-((methylcarbamoyl)oxazol-2-yl]methyl (3a*R*,5*s*,6a*S*)-5-(4-chlorophenoxy)hexahydrocyclopenta[*c*]pyrrole-2(1*H*)-carboxylate (exo)
(3-carbamoylisoxazol-5-yl)methyl (3a*R*,5*s*,6a*S*)-5-[(4-chloro-1-naphthyl)oxy]hexahydrocyclopenta[*c*]pyrrole-2(1*H*)-carboxylate (exo)
[3-((methylcarbamoyl)isoxazol-5-yl]methyl (3a*R*,5*s*,6a*S*)-5-[(4-chloro-1-naphthyl)oxy]hexahydrocyclopenta[*c*]-pyrrole-2(1*H*)-carboxylate (exo)
thiazol-4-ylmethyl (3a*R*,4*S*,6a*S*)-4-[(4-chloro-1-naphthyl)oxy]hexahydrocyclopenta[*c*]pyrrole-2(1*H*)-carboxylate (exo)
thiazol-4-ylmethyl (3a*R*,4*R*,6a*S*)-4-[(4'-ethoxybiphenyl-3-yl)oxy]hexahydrocyclopenta[*c*]pyrrole-2(1*H*)-carboxylate (endo)
(3-carbamoylisoxazol-5-yl)methyl (3a*R*,5*s*,6a*S*)-5-[(4'-fluorobiphenyl-4-yl)oxy]hexahydrocyclopenta[*c*]pyrrole-2(1*H*)-carboxylate (exo)
[3-((methylcarbamoyl)isoxazol-5-yl]methyl (3a*R*,5*s*,6a*S*)-5-(4-chloro-2-fluorophenoxy)hexa-hydrocyclopenta[*c*]pyrrole-2(1*H*)-carboxylate (exo)
(3-carbamoylisoxazol-5-yl)methyl (3a*R*,5*s*,6a*S*)-5-(4-chloro-2-fluorophenoxy)hexahydrocyclopenta[*c*]pyrrole-2(1*H*)-carboxylate (exo)
(3-carbamoylisoxazol-5-yl)methyl (3a*R*,5*s*,6a*S*)-5-(4-chloro-3-fluorophenoxy)hexahydrocyclopenta[*c*]pyrrole-2(1*H*)-carboxylate (exo)
[3-((methylcarbamoyl)isoxazol-5-yl]methyl (3a*R*,5*s*,6a*S*)-5-(4-chloro-3-fluorophenoxy)hexa-hydrocyclopenta[*c*]pyrrole-2(1*H*)-carboxylate (exo)
(3-carbamoylisoxazol-5-yl)methyl (3a*R*,5*s*,6a*S*)-5-(2,4-dichlorophenoxy)hexahydrocyclopenta[*c*]pyrrole-2(1*H*)-carboxylate (exo)
[3-((methylcarbamoyl)isoxazol-5-yl]methyl (3a*R*,5*s*,6a*S*)-5-(2,4-dichlorophenoxy)hexahydrocyclopenta[*c*]pyrrole-2(1*H*)-carboxylate (exo)
(3-carbamoylisoxazol-5-yl)methyl (3a*R*,5*s*,6a*S*)-5-(isoquinolin-7-yloxy)hexahydrocyclopenta[*c*]pyrrole-2(1*H*)-carboxylate (exo)
(3-carbamoylisoxazol-5-yl)methyl (3a*R*,5*s*,6a*S*)-5-(isoquinolin-6-yloxy)hexahydrocyclopenta[*c*]pyrrole-2(1*H*)-carboxylate (exo)
(3-carbamoylisoxazol-5-yl)methyl (3a*R*,5*r*,6a*S*)-5-(4-fluoro-1,3-benzothiazol-2-yl)-5-hydroxyhexahydrocyclo-penta[*c*]pyrrole-2(1*H*)-carboxylate (exo)
(4-carbamoyloxazol-2-yl)methyl (3a*R*,5*s*,6a*S*)-5-[(7-ethoxy-2-naphthyl)oxy]hexahydrocyclopenta[*c*]pyrrole-2(1*H*)-carboxylate (exo)
(3-{[2-(dimethylamino)ethyl]carbamoyl}isoxazol-5-yl)-methyl (3a*R*,5*s*,6a*S*)-5-(4-fluorophenoxy)hexahydrocyclo-penta[*c*]pyrrole-2(1*H*)-carboxylate (exo), and its hydrochloride
[4-((methylcarbamoyl)oxazol-2-yl]methyl (3a*R*,5*s*,6a*S*)-5-[(7-ethoxy-2-naphthyl)oxy]hexa-hydrocyclopenta[*c*]pyrrole-2(1*H*)-carboxylate (exo)
(3-dimethylcarbamoylisoxazol-5-yl)methyl (3a*R*,5*s*,6a*S*)-5-(4-fluorophenoxy)hexahydrocyclopenta[*c*]pyrrole-2(1*H*)-carboxylate (exo)
(3-methylcarbamoylisoxazol-5-yl)methyl (3a*R*,5*s*,6a*S*)-5-[(4'-fluorobiphenyl-4-yl)oxy]hexahydrocyclopenta[*c*]-pyrrole-2(1*H*)-carboxylate (exo).

6. Process for preparing a compound of formula (I) according to any one of Claims 1 to 5, comprising the step consisting in reacting either an amine of general formula (II), 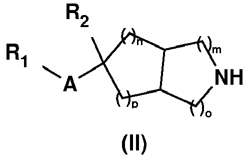 in which A, R₁, R₂, m, n, o and p are as defined in the general formula (I) according to Claim 1,
a carbonate of general formula (III) 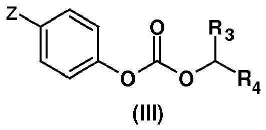 in which Z represents a hydrogen atom or a nitro group, and R₃ and R₄ are as defined in the general formula (I) according to Claim 1,
in the presence of a base, in a solvent at a temperature of between room temperature and the reflux temperature of the solvent.

7. Process for preparing a compound of formula (I) according to any one of Claims 1 to 5, comprising the step consisting in reacting a compound of general formula (Ia) 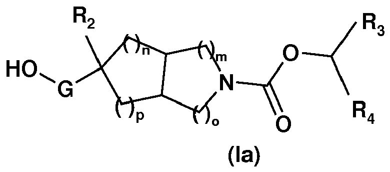 in which R₂, R₃, R₄, m, n, o and p are as defined in the general formula (I) according to Claim 1, and G represents part of the group A as defined in the general formula (I), namely a covalent bond;
with either an alcohol derivative of general formula R₁OH (IV), in which R₁ is as defined in the general formula (I) according to Claim 1, using the Mitsunobu reaction conditions;
or with a halo derivative of general formula R₁X (IVa), in which R₁ is as defined in the general formula (I) according to Claim 1, and X represents a fluorine, chlorine, bromine or iodine atom, using aromatic or heteroaromatic nucleophilic substitution, or Buchwald *O*-arylation or O-heteroarylation reactions.

8. Process for preparing a compound of formula (I) according to any one of Claims 1 to 5, in which R₁ represents a group R₅ substituted especially with a group R₆ of the type C₁₋₆-alkyl, C₃₋₇-cycloalkyl or C₃₋₇-cycloalkyl-C₁₋₃-alkylene, or with a group R₇ as defined in the general formula (I) according to Claim 1, comprising the step consisting in performing a coupling reaction, catalysed with a transition metal, on the compound of general formula (Ib), 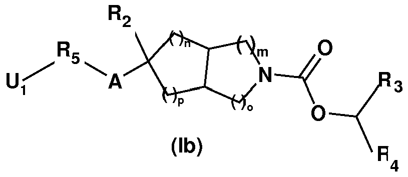 in which A, R₂, R₃, R₄, R₅, m, n, o and p are as defined in the general formula (I) according to Claim 1 and U₁ represents a chlorine, bromine or iodine atom or a triflate group, U₁ being in the position in which it is desired to introduce the group R₆ or R₇:
- either via a reaction of Suzuki type, p for example using an alkyl, cycloalkyl, aryl or heteroaryl boronic acid,
- or according to a reaction of Stille type, for example using an aryl or heteroaryl *tri*-alkylstannous derivative;
- or via a reaction of Negishi type, for example using an alkyl, cycloalkyl, aryl or heteroaryl halide zincate derivative.

9. Compound of general formula (Ia) 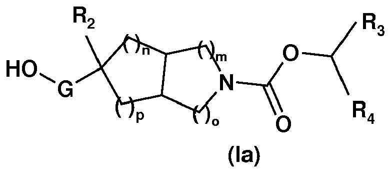 in which R₂, R₃, R₄, m, n, o and p are as defined in Claim 1, and G represents part of the group A as defined in the general formula (I), namely a covalent bond.

10. Compound of general formula (II), 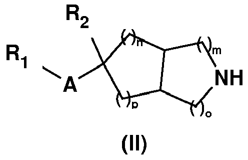 in which R₁, R₂, m, n, o and p are as defined in Claim 1, A represents an oxygen atom or a covalent bond, given that when A represents a covalent bond, then R₁, represents a benzothiazolyl group.

11. Compounds of general formula (IIe): 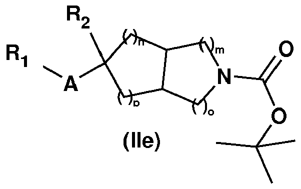 in which R₁, R₂, m, n, o and p are as defined in Claim 1, A represents an oxygen atom or a covalent bond, given that when A represents a covalent bond, then R₁ represents a benzothiazolyl group.

12. Compound of formula (I) according to any one of Claims 1 to 5, in the form of the base or of a pharmaceutically acceptable acid-addition salt, for its use as a medicament.

13. Pharmaceutical composition containing at least one compound of formula (I) according to any one of Claims 1 to 5, in the form of the base or of a pharmaceutically acceptable acid-addition salt and optionally one or more pharmaceutically acceptable excipients.

14. Use of a compound of formula (I) according to any one of Claims 1 to 5, in the form of the base or of a pharmaceutically acceptable acid-addition salt, for the preparation of a medicament for preventing or treating a pathology in which the endogenous cannabinoids and/or any substrate metabolized by the enzyme FAAH are involved.

15. Use of a compound of formula (I) according to any one of Claims 1 to 5, in the form of the base or of a pharmaceutically acceptable acid-addition salt, for the preparation of a medicament for preventing or treating acute or chronic pain of neurogenic type, acute or chronic pain associated with inflammatory diseases, acute or chronic peripheral pain, vertigo, vomiting, nausea, eating behaviour disorders, neurological and psychiatric pathologies, acute or chronic neurodegenerative diseases, epilepsy, sleeping disorders, cardiovascular diseases, renal ischaemia, cancer, immune system disorders, allergic diseases, parasitic, viral or bacterial infectious diseases, inflammatory diseases, osteoporosis, ocular complaints, pulmonary complaints, gastrointestinal diseases, urinary incontinence or bladder inflammation.
